# EUROPÄISCHE PATENTSCHRIFT

(11) **EP 3 679 023 B1**
(45) Veröffentlichungstag und Bekanntmachung des Hinweises auf die Patenterteilung: **02.11.2022**
(21) Anmeldenummer: 18759982.4
(22) Anmeldetag: 05.09.2018
(51) Int. Cl.: C07C 211/56, H01L 51/00, C07D 333/76, C07D 405/12, C07D 209/86, C07D 307/91, C07C 211/61, C07C 211/54

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 08.09.2017 EP 17190206; 22.05.2018 EP 18173679
(43) Veröffentlichungstag der Anmeldung: 15.07.2020
(73) Patentinhaber: Merck Patent GmbH, 64293 Darmstadt (DE)
(72) Erfinder: MONTENEGRO, Elvira, 69469 Weinheim (DE); MUJICA-FERNAUD, Teresa, 64283 Darmstadt (DE); MAIER-FLAIG, Florian, 69469 Weinheim (DE); VOGES, Frank, 67098 Bad Duerkheim (DE)
(86) Internationale Anmeldenummer: PCT/EP2018/073827
(87) Internationale Veröffentlichungsnummer: WO 2019/048458

(56) Entgegenhaltungen:
- WO-A1-2006/122630
- WO-A1-2017/036573
- YASUHIKO SHIROTA AND HIROSHI KAGEYAMA: "Charge Carrier Transporting Molecular Materials and Their Applications in Devices", CHEMICAL REV, AMERICAN CHEMICAL SOCIETY, US, Bd. 107, Nr. 4, 1. April 2007 (2007-04-01) , Seiten 953-1010, XP009141446, ISSN: 0009-2665, DOI: 10.1021/CR050143+ [gefunden am 2007-04-11]

## Beschreibung

Die vorliegende Anmeldung betrifft aromatische Verbindungen enthaltend eine Gruppe gewählt aus Aminogruppen, verbrückten Aminogruppen und Carbazolgruppen, gemäß den untenstehend definierten Formeln (I) und (II). Diese Verbindungen eignen sich zur Verwendung in elektronischen Vorrichtungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an einer Verbesserung der Leistungsdaten, insbesondere Lebensdauer, Effizienz und Betriebsspannung. In diesen Punkten konnte noch keine vollständig zufriedenstellende Lösung gefunden werden.

Weiterhin werden Materialien mit einer hohen Glasübergangstemperatur, einer geringen Neigung zur Kristallisation und einem hohen Brechungsindex gesucht, insbesondere zur Verwendung in lochtransportierenden Schichten von OLEDs.

Einen großen Einfluss auf die Leistungsdaten von elektronischen Vorrichtungen haben Emissionsschichten und Schichten mit lochtransportierender Funktion. Zur Verwendung in diesen Schichten werden weiterhin neue Verbindungen gesucht, insbesondere lochtransportierende Verbindungen und Verbindungen, die als Matrixmaterial, insbesondere für phosphoreszierende Emitter, in einer emittierenden Schicht dienen können.

Im Stand der Technik (z.B. WO 2006/122630 A1) sind verschiedene aromatische Verbindungen enthaltend eine Gruppe gewählt aus Aminogruppen, verbrückten Aminogruppen und Carbazolgruppen, als Lochtransportmaterialien und/oder Matrixmaterialien in elektronischen Vorrichtungen bekannt.

Es besteht jedoch weiterhin Bedarf an alternativen Verbindungen, die zur Verwendung in elektronischen Vorrichtungen geeignet sind. Es besteht auch Verbesserungsbedarf bezüglich der Leistungsdaten bei Verwendung in elektronischen Vorrichtungen, insbesondere bezüglich Lebensdauer, Betriebsspannung und Effizienz.

Nun wurde gefunden, dass sich bestimmte Verbindungen der oben genannten Strukturklasse hervorragend zur Verwendung in elektronischen Vorrichtungen eignen, insbesondere zur Verwendung in OLEDs, nochmals insbesondere darin zur Verwendung als Lochtransportmaterialien und zur Verwendung als Matrixmaterialien für phosphoreszierende Emitter. Die Verbindungen führen bevorzugt zu hoher Lebensdauer, hoher Effizienz und geringer Betriebsspannung der Vorrichtungen. Weiterhin bevorzugt weisen die Verbindungen eine geringe Kristallisationsneigung, eine hohe Glasübergangstemperatur und einen hohen Brechungsindex auf.

Gegenstand der vorliegenden Anmeldung ist eine Verbindung der folgenden Formel (I) oder (II) 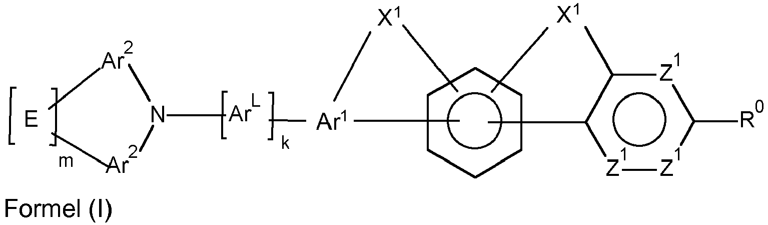 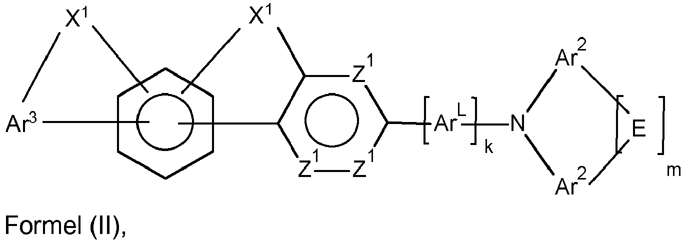 wobei für die auftretenden Variablen gilt:
Z¹ ist bei jedem Auftreten gleich oder verschieden gewählt aus CR¹ und CR³;
Ar¹ ist eine Arylgruppe mit 6 bis 20 aromatischen Ringatomen, die mit einem oder mehreren Resten R³ substituiert sein kann, oder eine Heteroarylgruppe mit 5 bis 20 aromatischen Ringatomen, die mit einem oder mehreren Resten R³ substituiert sein kann;
Ar² ist bei jedem Auftreten gleich oder verschieden gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
Ar³ ist eine Arylgruppe mit 6 bis 20 aromatischen Ringatomen, die mit einem oder mehreren Resten R² substituiert sein kann, oder eine Heteroarylgruppe mit 5 bis 20 aromatischen Ringatomen, die mit einem oder mehreren Resten R² substituiert sein kann;
X¹ ist bei jedem Auftreten gleich oder verschieden eine divalente Gruppe gewählt aus -C(R⁴)₂-, -C(R⁴)₂-C(R⁴)2-, -CR⁴=CR⁴-, -Si(R⁴)₂-, NR⁴, O und S;
Ar^{L} ist gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
E ist eine Einfachbindung oder eine divalente Gruppe gewählt aus C(R⁵)₂, Si(R⁵)₂, N(R⁵), O, und S;
R⁰ ist gewählt aus H, D, aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können;
R¹ ist bei jedem Auftreten gleich oder verschieden gewählt aus aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können;
R² ist bei jedem Auftreten gleich oder verschieden gewählt aus H, D, F, CN, Si(R⁶)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁰ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch - R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R³, R⁴, R⁵ bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ bzw. R⁴ bzw. R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R⁷ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁷ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
k ist gleich 0, 1, 2 oder 3, wobei im Fall k=0 die Gruppe Ar^{L} wegfällt und das Stickstoffatom der Gruppe der Formel (N) die Anbindungsposition darstellt;
m ist gleich 0 oder 1, wobei im Fall m=0 die Gruppe E wegfällt und die Gruppen Ar² nicht miteinander verbunden sind;
wobei in Formel (I) und (II) alle unsubstituiert gezeichneten Positionen jeweils mit einem Rest R³ substituiert sein können; und
wobei in Formel (I) und in Formel (II) jeweils mindestens eine Gruppe Z¹ vorliegt, die CR¹ ist.

Die Kreise, die in die Sechsringe der Formel (I) und (II) gezeichnet sind, bedeuten, dass die betreffenden Sechsringe Aromatizität aufweisen.

Die folgenden Definitionen gelten für die chemischen Gruppen, die in der vorliegenden Anmeldungen verwendet werden. Sie gelten, soweit keine spezielleren Definitionen angegeben sind.

Unter einer Arylgruppe im Sinne dieser Erfindung wird entweder ein einzelner aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einzelnen aromatischen Cyclen. Unter Kondensation zwischen Cyclen ist dabei zu verstehen, dass die Cyclen mindestens eine Kante miteinander teilen. Eine Arylgruppe im Sinne dieser Erfindung enthält 6 bis 40 aromatische Ringatome, von denen keines ein Heteroatom ist.

Unter einer Heteroarylgruppe im Sinne dieser Erfindung wird entweder ein einzelner heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einzelnen aromatischen oder heteroaromatischen Cyclen, wobei wenigstens einer der aromatischen und heteroaromatischen Cyclen ein heteroaromatischer Cyclus ist. Unter Kondensation zwischen Cyclen ist dabei zu verstehen, dass die Cyclen mindestens eine Kante miteinander teilen. Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S.

Unter einer Aryl- oder Heteroarylgruppe, die jeweils mit den oben genannten Resten substituiert sein kann werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung ist ein System, welches nicht notwendigerweise nur Arylgruppen enthält, sondern welches zusätzlich einen oder mehrere nicht-aromatische Ringe enthalten kann, die mit wenigstens einer Arylgruppe kondensiert sind. Diese nicht-aromatischen Ringe enthalten ausschließlich Kohlenstoffatome als Ringatome. Beispiele für Gruppen, die von dieser Definition umfasst sind, sind Tetrahydronaphthalin, Fluoren und Spirobifluoren. Weiterhin umfasst der Begriff aromatisches Ringsystem Systeme, die aus zwei oder mehr aromatischen Ringsystemen bestehen, die über Einfachbindungen miteinander verbunden sind, beispielsweise Biphenyl, Terphenyl, 7-Phenyl-2-fluorenyl, Quaterphenyl und 3,5-Diphenyl-1-phenyl. Ein aromatisches Ringsystem im Sinne dieser Erfindung enthält 6 bis 40 C-Atome und keine Heteroatome im Ringsystem. Die Definition von "aromatisches Ringsystem" umfasst nicht Heteroarylgruppen.

Ein heteroaromatisches Ringsystem entspricht der oben genannten Definition eines aromatischen Ringsystems, mit dem Unterschied dass es mindestens ein Heteroatom als Ringatom enthalten muss. Wie es beim aromatischen Ringsystem der Fall ist, muss das heteroaromatische Ringsystem nicht ausschließlich Arylgruppen und Heteroarylgruppen enthalten, sondern es kann zusätzlich einen oder mehrere nicht-aromatische Ringe enthalten, die mit wenigstens einer Aryl- oder Heteroarylgruppe kondensiert sind. Die nicht-aromatischen Ringe können ausschließlich C-Atome als Ringatome enthalten, oder sie können zusätzlich ein oder mehrere Heteroatome enthalten, wobei die Heteroatome bevorzugt gewählt sind aus N, O und S. Ein Beispiel für ein derartiges heteroaromatisches Ringsystem ist Benzopyranyl. Weiterhin werden unter dem Begriff "heteroaromatisches Ringsystem" Systeme verstanden, die aus zwei oder mehr aromatischen oder heteroaromatischen Ringsystemen bestehen, die miteinander über Einfachbindungen verbunden sind, wie beispielsweise 4,6-Diphenyl-2-triazinyl. Ein heteroaromatisches Ringsystem im Sinne dieser Erfindung enthält 5 bis 40 Ringatome, die gewählt sind aus Kohlenstoff und Heteroatomen, wobei mindestens eines der Ringatome ein Heteroatom ist. Die Heteroatome des heteroaromatischen Ringsystems sind bevorzugt ausgewählt aus N, O und S.

Die Begriffe "heteroaromatisches Ringsystem" und "aromatisches Ringsystem" gemäß der Definition der vorliegenden Anmeldung unterscheiden sich damit dadurch voneinander, dass ein aromatisches Ringsystem kein Heteroatom als Ringatom aufweisen kann, während ein heteroaromatisches Ringsystem mindestens ein Heteroatom als Ringatom aufweisen muss. Dieses Heteroatom kann als Ringatom eines nicht-aromatischen heterocyclischen Rings oder als Ringatom eines aromatischen heterocyclischen Rings vorliegen.

Entsprechend der obenstehenden Definitionen ist jede Arylgruppe vom Begriff "aromatisches Ringsystem" umfasst, und jede Heteroarylgruppe ist vom Begriff "heteroatomatisches Ringsystem" umfasst.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Die Verbindungen der Formel (I) und (II) enthalten bevorzugt nur eine Triarylaminogruppe. Besonders bevorzugt enthalten sie nur eine Aminogruppe. Unter einer Triarylaminogruppe wird dabei eine Aminogruppe verstanden, an die drei Gruppen gewählt aus aromatischen Ringsystemen und heteroaromatischen Ringsystemen gebunden sind.

Bevorzugt sind eine oder zwei Gruppen Z¹ gleich CR¹, und die anderen Gruppen Z¹ sind gleich CR³. Besonders bevorzugt ist eine Gruppe Z¹ gleich CR¹, und die anderen zwei Gruppen Z¹ sind gleich CR³.

Bevorzugt ist Ar¹ eine Arylgruppe mit 6 bis 14 aromatischen Ringatomen, die mit einem oder mehreren Resten R³ substituiert sein kann, besonders bevorzugt ist Ar¹ eine Benzolgruppe, die mit einem oder mehreren Resten R³ substituiert sein kann.

Bevorzugt ist Ar³ eine Arylgruppe mit 6 bis 14 aromatischen Ringatomen, die mit einem oder mehreren Resten R² substituiert sein kann, besonders bevorzugt ist Ar³ eine Benzolgruppe, die mit einem oder mehreren Resten R² substituiert sein kann.

Bevorzugt ist X¹ bei jedem Auftreten gleich gewählt. Bevorzugt ist X¹ bei jedem Auftreten gleich C(R⁴)₂ oder Si(R⁴)₂, besonders bevorzugt gleich C(R¹)₂.

Gruppen Ar^{L} sind bevorzugt gewählt aus aromatischen Ringsystemen mit 6 bis 20 aromatischen Ringatomen, die mit einem oder mehreren Resten R⁵ substituiert sein können, und heteroaromatischen Ringsystemen mit 5 bis 20 aromatischen Ringatomen, die mit einem oder mehreren Resten R⁵ substituiert sein können. Besonders bevorzugte Gruppen Ar^{L} sind gewählt aus divalenten Gruppen abgeleitet von Benzol, Biphenyl, Terphenyl, Naphthalin, Fluoren, Indenofluoren, Indenocarbazol, Spirobifluoren, Dibenzofuran, Dibenzothiophen, und Carbazol, die jeweils mit einem oder mehreren Resten R⁵ substituiert sein können. Ganz besonders bevorzugt ist Ar^{L} eine divalente Gruppe abgeleitet von Benzol, das jeweils mit einem oder mehreren Resten R⁵ substituiert sein kann. Gruppen Ar^{L} können bei jedem Auftreten gleich oder verschieden gewählt sein.

Bevorzugt ist k gewählt aus 0 oder 1, besonders bevorzugt ist k gleich 0.

Bevorzugte Gruppen -(Ar^{L})ₖ- entsprechen den folgenden Formeln:

| | | |
|---|---|---|
| 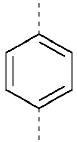 | 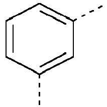 | 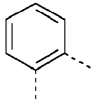 |
| Ar^{L}-1 | Ar^{L}-2 | Ar^{L}-3 |
| 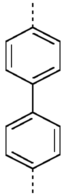 | 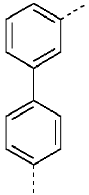 | 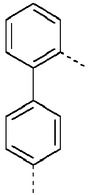 |
| Ar^{L}-4 | Ar^{L}-5 | Ar^{L}-6 |
| 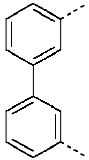 | 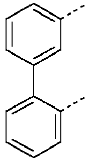 | 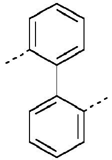 |
| Ar^{L}-7 | Ar^{L}-8 | Ar^{L}-9 |
| 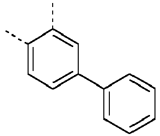 | 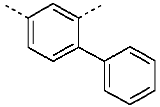 | 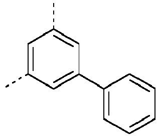 |
| Ar^{L}-10 | Ar^{L}-11 | Ar^{L}-12 |
| 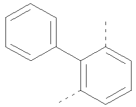 | 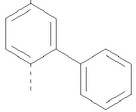 | 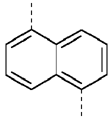 |
| Ar^{L}-13 | Ar^{L}-14 | Ar^{L}-15 |
| 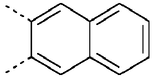 | 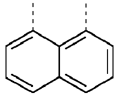 | 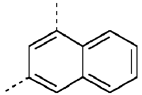 |
| Ar^{L}-16 | Ar^{L}-17 | Ar^{L}-18 |
| 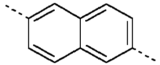 | 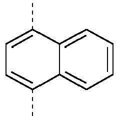 | 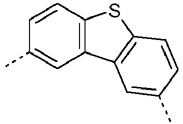 |
| Ar^{L}-19 | Ar^{L}-20 | Ar^{L}-21 |
| 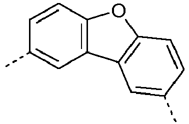 | 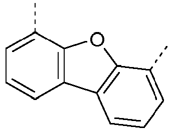 | 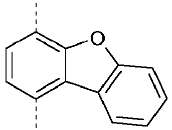 |
| Ar^{L}-22 | Ar^{L}-23 | Ar^{L}-24 |
| 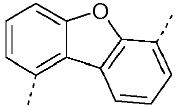 | 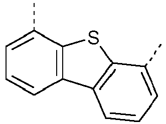 | 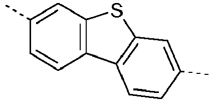 |
| Ar^{L}-25 | Ar^{L}-26 | Ar^{L}-27 |
| 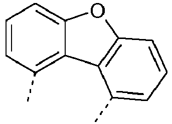 | 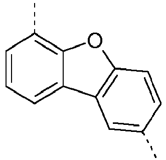 | 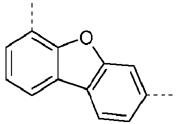 |
| Ar^{L}-28 | Ar^{L}-29 | Ar^{L}-30 |
| 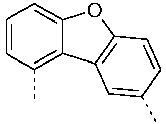 | 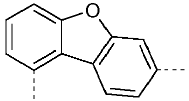 | 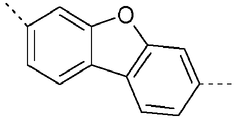 |
| Ar^{L}-31 | Ar^{L}-32 | Ar^{L}-33 |
| 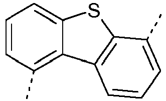 | 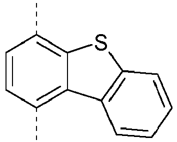 | 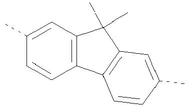 |
| Ar^{L}-34 | Ar^{L}-35 | Ar^{L}-36 |
| 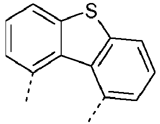 | 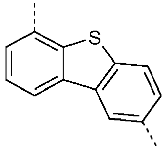 | 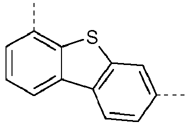 |
| Ar^{L}-37 | Ar^{L}-38 | Ar^{L}-39 |
| 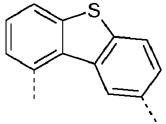 | 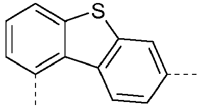 | 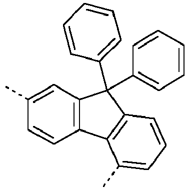 |
| Ar^{L}-40 | Ar^{L}-41 | Ar^{L}-42 |
| 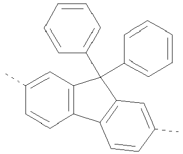 | 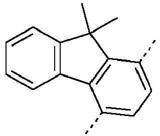 | 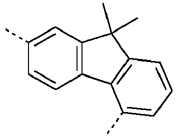 |
| Ar^{L}-43 | Ar^{L}-44 | Ar^{L}-45 |
| 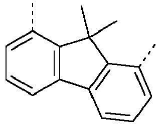 | 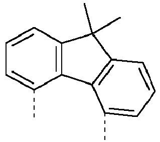 | 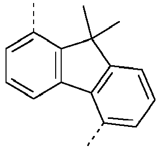 |
| Ar^{L}-46 | Ar^{L}-47 | Ar^{L}-48 |
| 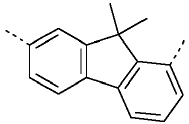 | 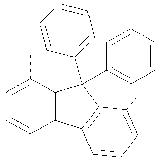 | 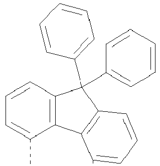 |
| Ar^{L}-49 | Ar^{L}-50 | Ar^{L}-51 |
| 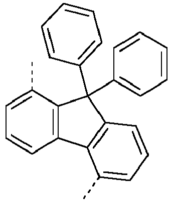 | 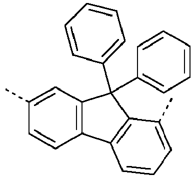 | 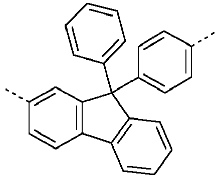 |
| Ar^{L}-52 | Ar^{L}-53 | Ar^{L}-54 |
| 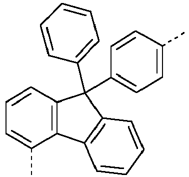 | 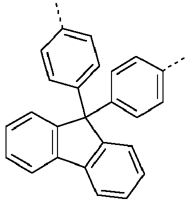 | 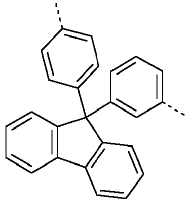 |
| Ar^{L}-55 | Ar^{L}-56 | Ar^{L}-57 |
| 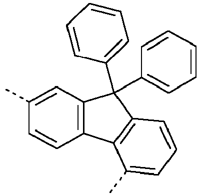 | 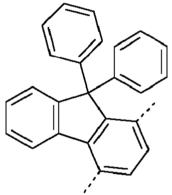 | 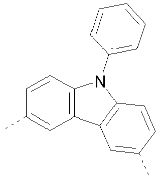 |
| Ar^{L}-58 | Ar^{L}-59 | Ar^{L}-60 |
| 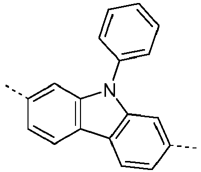 | 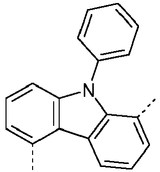 | 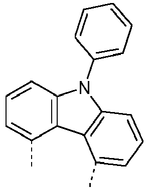 |
| Ar^{L}-61 | Ar^{L}-62 | Ar^{L}-63 |
| 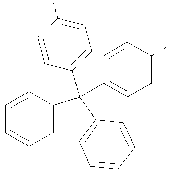 | 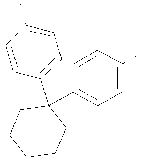 | 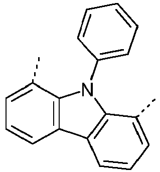 |
| Ar^{L}-64 | Ar^{L}-65 | Ar^{L}-66 |
| 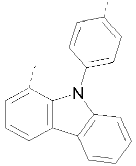 | 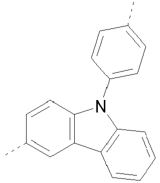 | 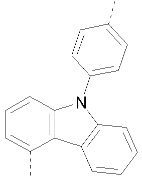 |
| Ar^{L}-67 | Ar^{L}-68 | Ar^{L}-69 |
| 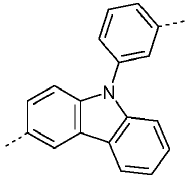 | 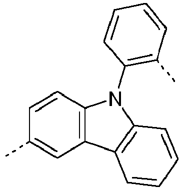 | 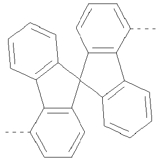 |
| Ar^{L}-70 | Ar^{L}-71 | Ar^{L}-72 |
| 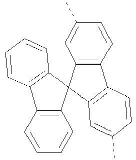 | 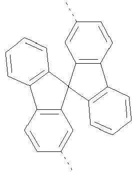 | 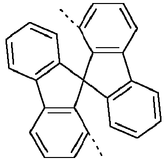 |
| Ar^{L}-73 | Ar^{L}-74 | Ar^{L}-75 |

wobei die gestrichelten Linien die Bindungen an den Rest der Formel (I) bzw. (II) darstellen.

Es ist bevorzugt, dass die direkt an das Stickstoffatom bindende Gruppe von Ar² ein aromatisches Ringsystem ist.

Bevorzugt sind Gruppen Ar² bei jedem Auftreten gleich oder verschieden gewählt aus monovalenten Gruppen abgeleitet von Benzol, Biphenyl, Terphenyl, Quaterphenyl, Naphthalin, Fluoren, insbesondere 9,9'-Dimethylfluoren und 9,9'-Diphenylfluoren, Benzofluoren, Spirobifluoren, Indenofluoren, Indenocarbazol, Dibenzofuran, Dibenzothiophen, Benzocarbazol, Carbazol, Benzofuran, Benzothiophen, Indol, Chinolin, Pyridin, Pyrimidin, Pyrazin, Pyridazin, und Triazin, wobei die monovalenten Gruppen jeweils mit einem oder mehreren Resten R⁵ substituiert sein können. Alternativ bevorzugt können die Gruppen Ar² bei jedem Auftreten gleich oder verschieden gewählt sein aus Kombinationen von Gruppen, die abgeleitet sind von Benzol, Biphenyl, Terphenyl, Quaterphenyl, Naphthalin, Fluoren, insbesondere 9,9'-Dimethylfluoren und 9,9'-Diphenylfluoren, Benzofluoren, Spirobifluoren, Indenofluoren, Indenocarbazol, Dibenzofuran, Dibenzothiophen, Carbazol, Benzofuran, Benzothiophen, Indol, Chinolin, Pyridin, Pyrimidin, Pyrazin, Pyridazin und Triazin, wobei die Gruppen jeweils mit einem oder mehreren Resten R⁵ substituiert sein können.

Besonders bevorzugte Gruppen Ar² sind bei jedem Auftreten gleich oder verschieden gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Carbazolyl, Benzofuranyl, Benzothiophenyl, benzokondensiertes Dibenzofuranyl, benzokondensiertes Dibenzothiophenyl, Naphthyl-substituiertes Phenyl, Fluorenyl-substituiertes Phenyl, Spirobifluorenyl-substituiertes Phenyl, Dibenzofuranyl-substituiertes Phenyl, Dibenzothiophenyl-substituiertes Phenyl, Carbazolyl-substituiertes Phenyl, Pyridyl-substituiertes Phenyl, Pyrimidyl-substituiertes Phenyl, und Triazinyl-substituiertes Phenyl, wobei die genannten Gruppen jeweils mit einem oder mehreren Resten R⁵ substituiert sein können.

Besonders bevorzugte Gruppen Ar² sind gewählt aus den folgenden Formeln:

| | | |
|---|---|---|
| 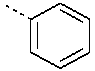 | 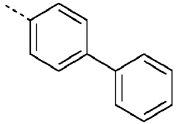 | 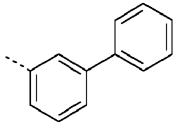 |
| Ar-1 | Ar-2 | Ar-3 |
| 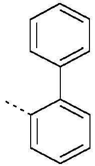 | 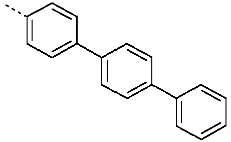 | 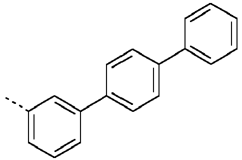 |
| Ar-4 | Ar-5 | Ar-6 |
| 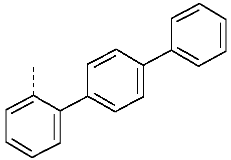 | 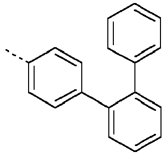 | 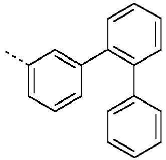 |
| Ar-7 | Ar-8 | Ar-9 |
| 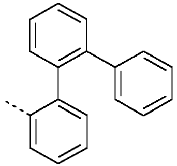 | 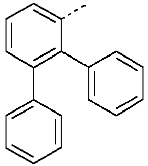 | 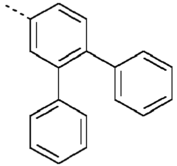 |
| Ar-10 | Ar-11 | Ar-12 |
| 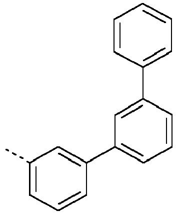 | 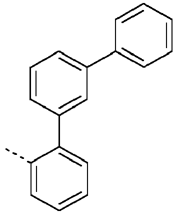 | 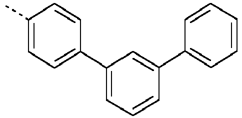 |
| Ar-13 | Ar-14 | Ar-15 |
| 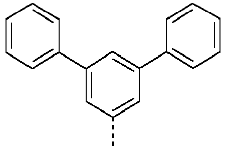 | 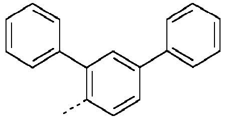 | 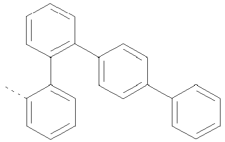 |
| Ar-16 | Ar-17 | Ar-18 |
| 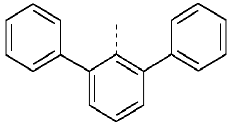 | | |
| | Ar-19 | |
| 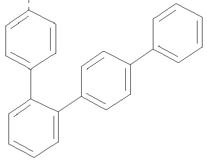 | 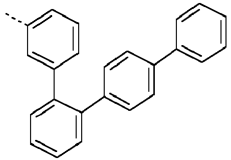 | 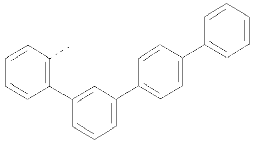 |
| Ar-20 | Ar-21 | Ar-22 |
| 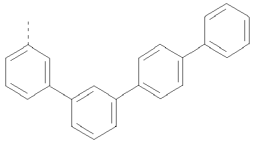 | 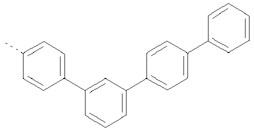 | 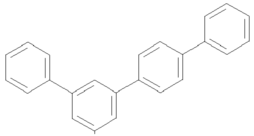 |
| Ar-23 | Ar-24 | Ar-25 |
| 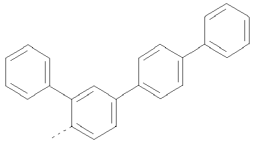 | 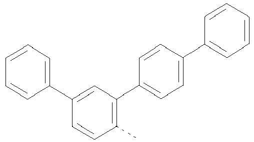 | 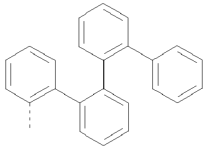 |
| Ar-26 | Ar-27 | Ar-28 |
| 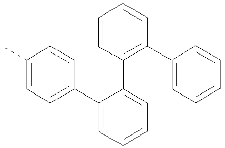 | 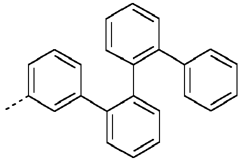 | 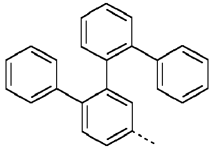 |
| Ar-29 | Ar-30 | Ar-31 |
| 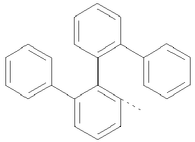 | 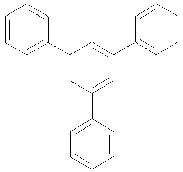 | 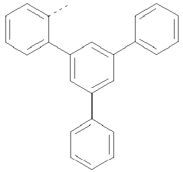 |
| Ar-32 | Ar-33 | Ar-34 |
| 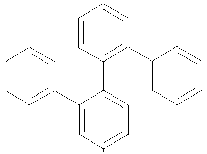 | 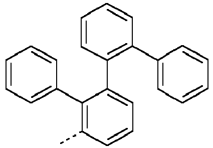 | 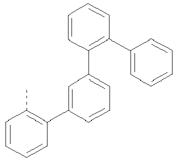 |
| Ar-35 | Ar-36 | Ar-37 |
| 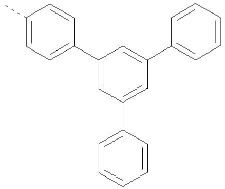 | 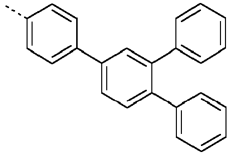 | 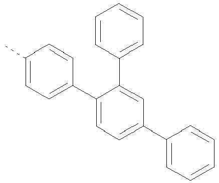 |
| Ar-38 | Ar-39 | Ar-40 |
| 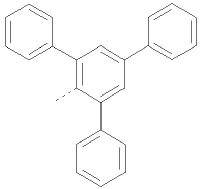 | 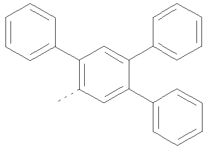 | 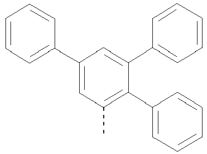 |
| Ar-41 | Ar-42 | Ar-43 |
| 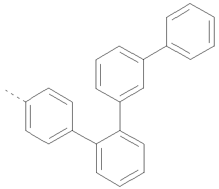 | 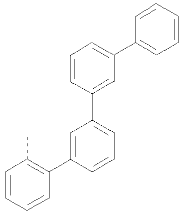 | 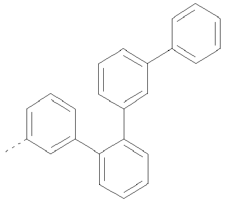 |
| Ar-44 | Ar-45 | Ar-46 |
| 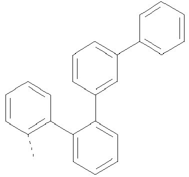 | | |
| | Ar-47 | |
| 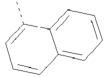 | 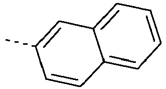 | 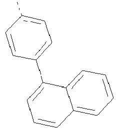 |
| Ar-48 | Ar-49 | Ar-50 |
| 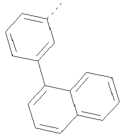 | 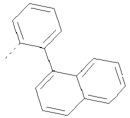 | 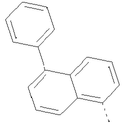 |
| Ar-51 | Ar-52 | Ar-53 |
| 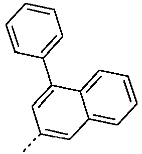 | 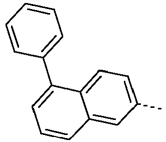 | 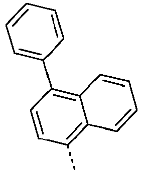 |
| Ar-54 | Ar-55 | Ar-56 |
| 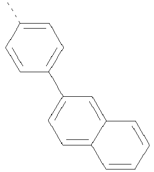 | 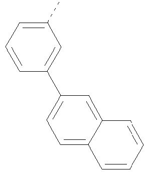 | 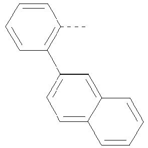 |
| Ar-57 | Ar-58 | Ar-59 |
| 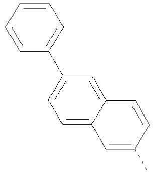 | 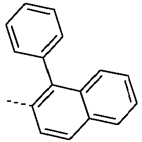 | 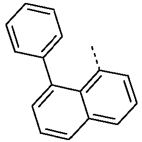 |
| Ar-60 | Ar-61 | Ar-62 |
| 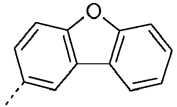 | 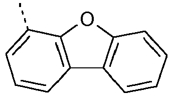 | 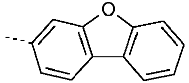 |
| Ar-63 | Ar-64 | Ar-65 |
| 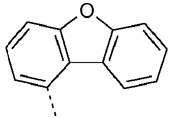 | 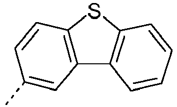 | 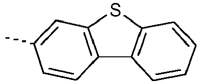 |
| Ar-66 | Ar-67 | Ar-68 |
| 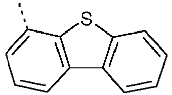 | 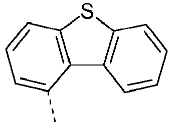 | 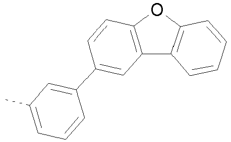 |
| Ar-69 | Ar-70 | Ar-71 |
| 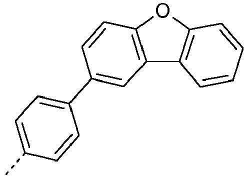 | 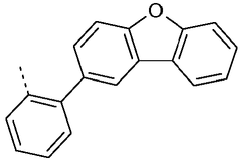 | 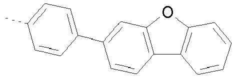 |
| Ar-72 | Ar-73 | Ar-74 |
| 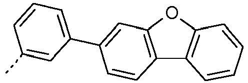 | 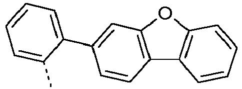 | 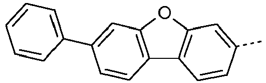 |
| Ar-75 | Ar-76 | Ar-77 |
| 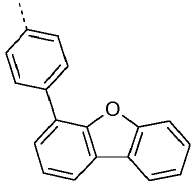 | 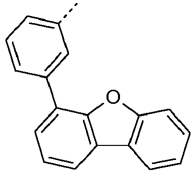 | 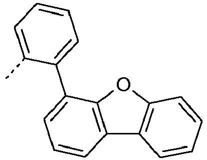 |
| Ar-78 | Ar-79 | Ar-80 |
| 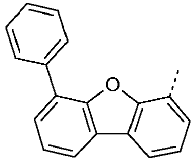 | 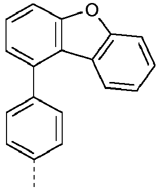 | 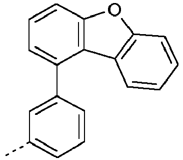 |
| Ar-81 | Ar-82 | Ar-83 |
| 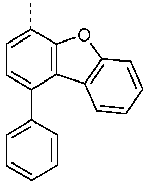 | 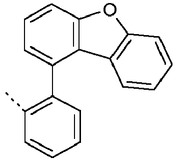 | 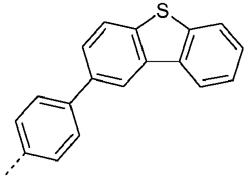 |
| Ar-84 | Ar-85 | Ar-86 |
| 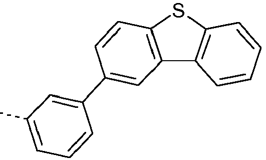 | 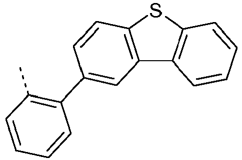 | 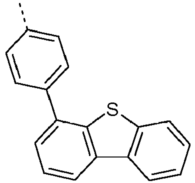 |
| Ar-87 | Ar-88 | Ar-89 |
| 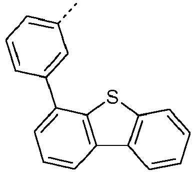 | 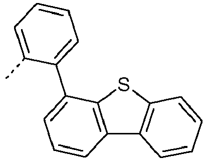 | 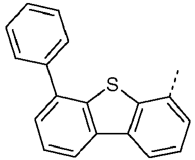 |
| Ar-90 | Ar-91 | Ar-92 |
| 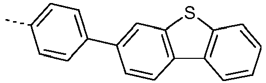 | 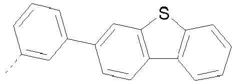 | 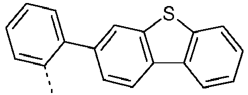 |
| Ar-93 | Ar-94 | Ar-95 |
| 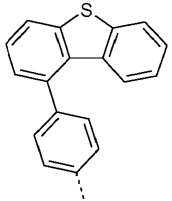 | 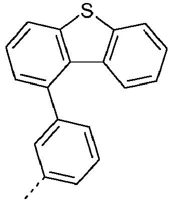 | 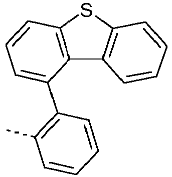 |
| Ar-96 | Ar-97 | Ar-98 |
| 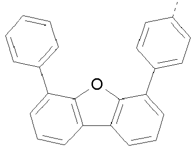 | 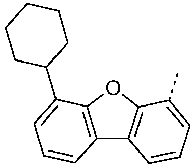 | 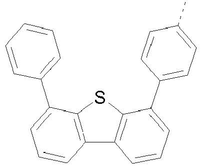 |
| Ar-99 | Ar-100 | Ar-101 |
| 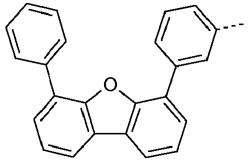 | 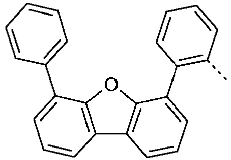 | 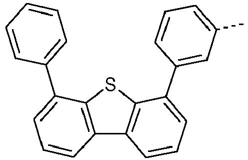 |
| Ar-102 | Ar-103 | Ar-104 |
| 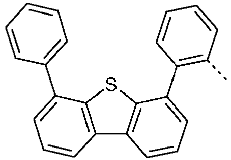 | | |
| Ar-105 | | |
| 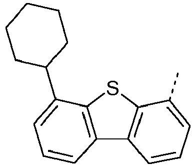 | 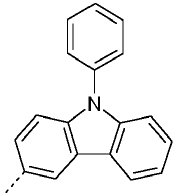 | 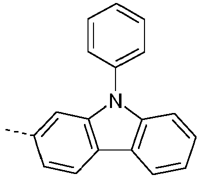 |
| Ar-106 | Ar-107 | Ar-108 |
| 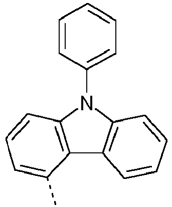 | 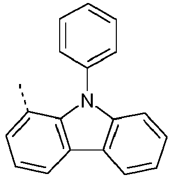 | 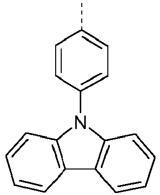 |
| Ar-109 | Ar-110 | Ar-111 |
| 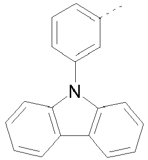 | 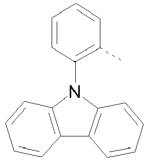 | |
| Ar-112 | Ar-113 | |
| 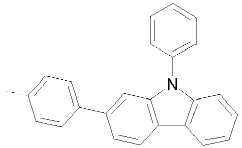 | 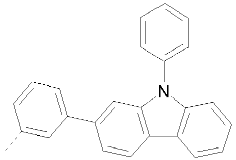 | 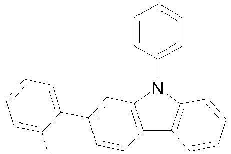 |
| Ar-114 | Ar-115 | Ar-116 |
| 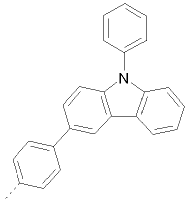 | 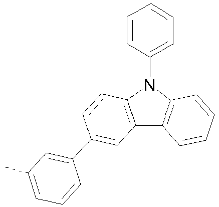 | 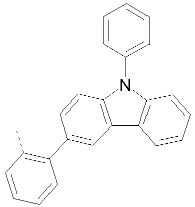 |
| Ar-117 | Ar-118 | Ar-119 |
| 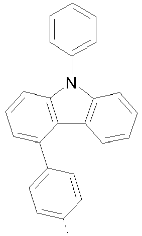 | 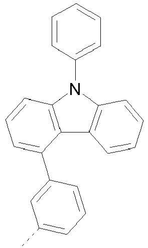 | 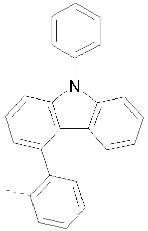 |
| Ar-120 | Ar-121 | Ar-122 |
| 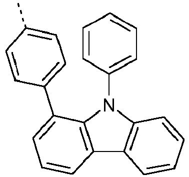 | 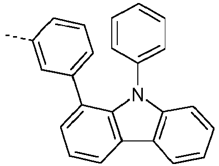 | 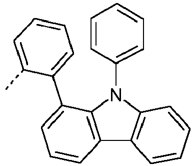 |
| Ar-123 | Ar-124 | Ar-125 |
| 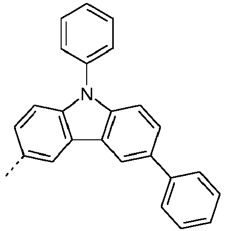 | 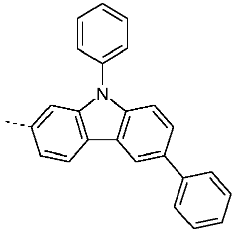 | 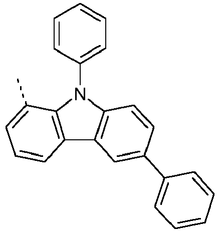 |
| Ar-126 | Ar-127 | Ar-128 |
| 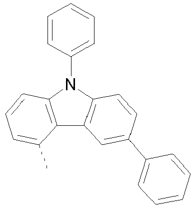 | 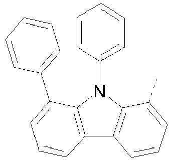 | 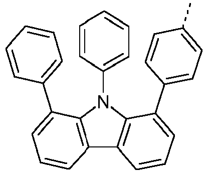 |
| Ar-129 | Ar-130 | Ar-131 |
| 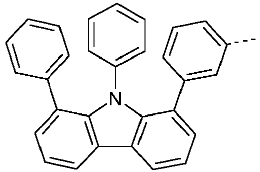 | 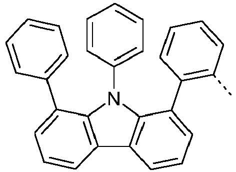 | |
| Ar-132 | Ar-133 | |
| 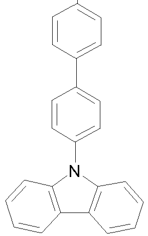 | 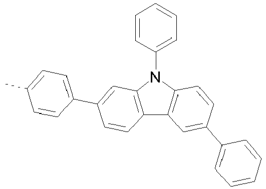 | 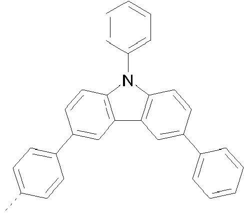 |
| Ar-134 | Ar-135 | Ar-136 |
| 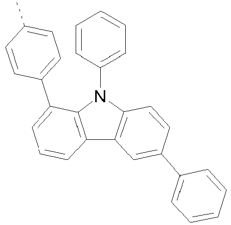 | 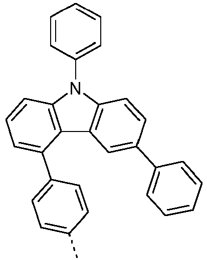 | 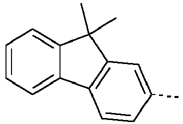 |
| Ar-137 | Ar-138 | Ar-139 |
| 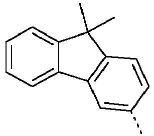 | 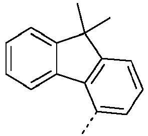 | 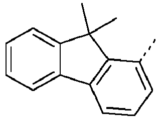 |
| Ar-140 | Ar-141 | Ar-142 |
| 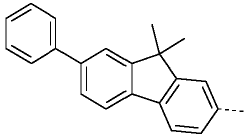 | 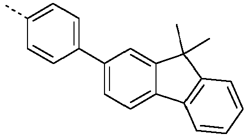 | 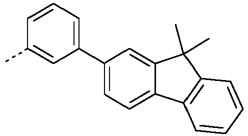 |
| Ar-143 | Ar-144 | Ar-145 |
| 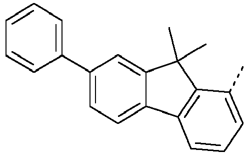 | 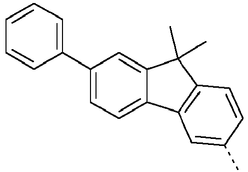 | 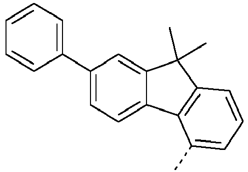 |
| Ar-146 | Ar-147 | Ar-148 |
| 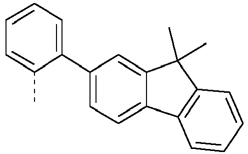 | 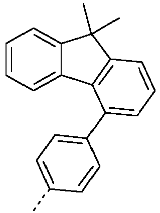 | 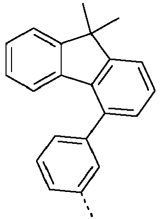 |
| Ar-149 | Ar-150 | Ar-151 |
| 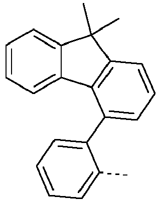 | 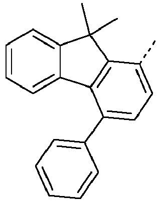 | 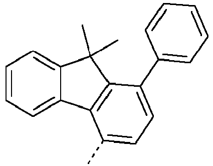 |
| Ar-152 | Ar-153 | Ar-154 |
| 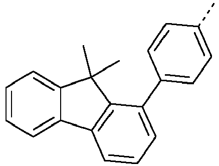 | 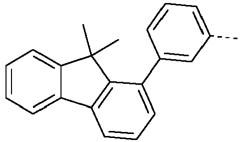 | 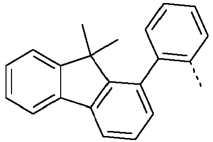 |
| Ar-155 | Ar-156 | Ar-157 |
| 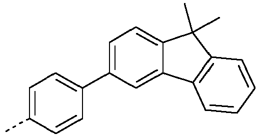 | 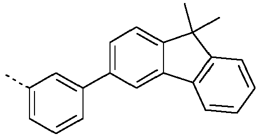 | 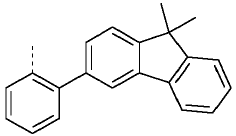 |
| Ar-158 | Ar-159 | Ar-160 |
| 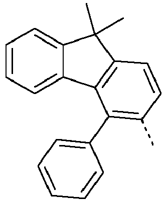 | 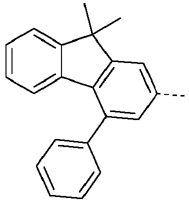 | 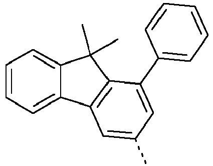 |
| Ar-161 | Ar-162 | Ar-163 |
| 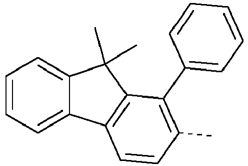 | 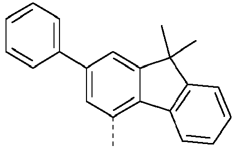 | 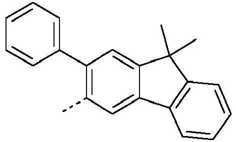 |
| Ar-164 | Ar-165 | Ar-166 |
| 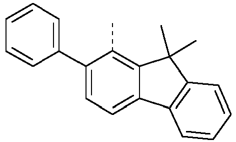 | 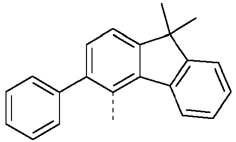 | 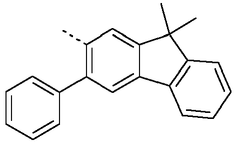 |
| Ar-167 | Ar-168 | Ar-169 |
| 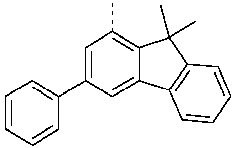 | 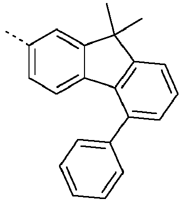 | 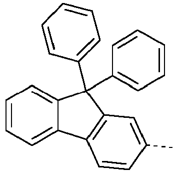 |
| Ar-170 | Ar-171 | Ar-172 |
| 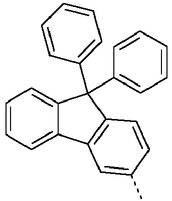 | 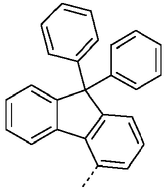 | 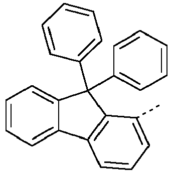 |
| Ar-173 | Ar-174 | Ar-175 |
| 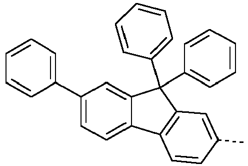 | 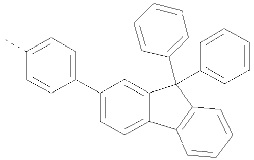 | 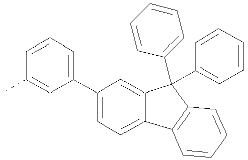 |
| Ar-176 | Ar-177 | Ar-178 |
| 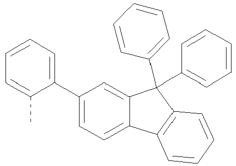 | 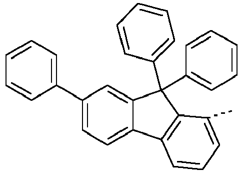 | 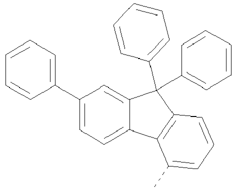 |
| Ar-179 | Ar-180 | Ar-181 |
| 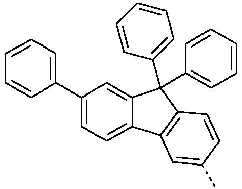 | 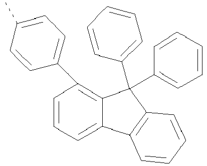 | 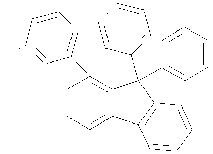 |
| Ar-182 | Ar-183 | Ar-184 |
| 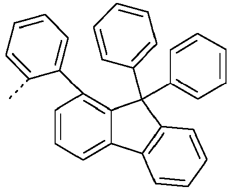 | 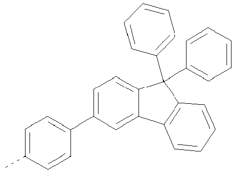 | 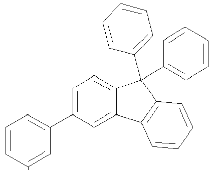 |
| Ar-185 | Ar-186 | Ar-187 |
| 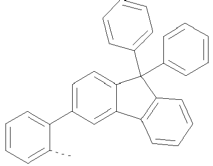 | 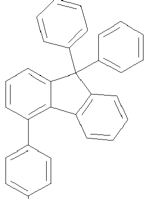 | 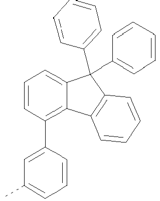 |
| Ar-188 | Ar-189 | Ar-190 |
| 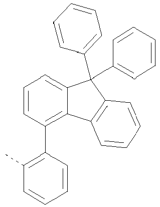 | | |
| Ar-191 | | |
| 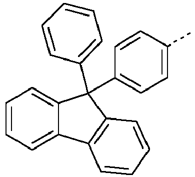 | 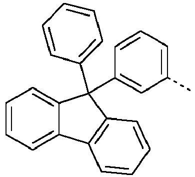 | 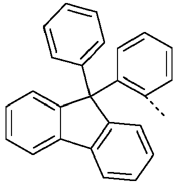 |
| Ar-192 | Ar-193 | Ar-194 |
| 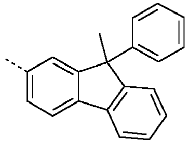 | 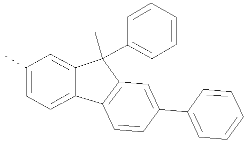 | 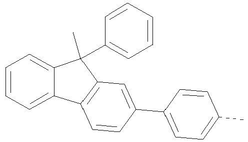 |
| Ar-195 | Ar-196 | Ar-197 |
| 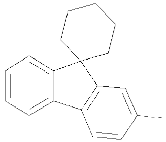 | 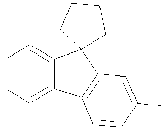 | 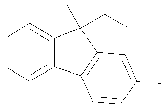 |
| Ar-198 | Ar-199 | Ar-200 |
| 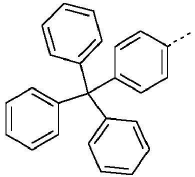 | 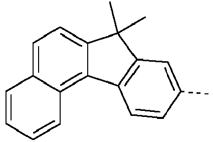 | 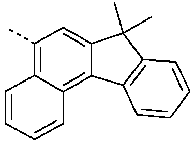 |
| Ar-201 | Ar-202 | Ar-203 |
| 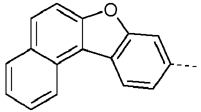 | 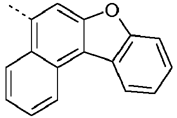 | 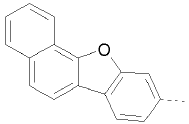 |
| Ar-204 | Ar-205 | Ar-206 |
| 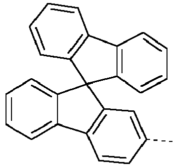 | 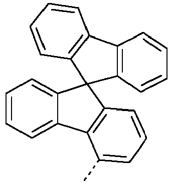 | 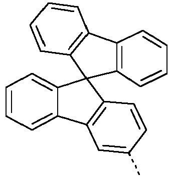 |
| Ar-207 | Ar-208 | Ar-209 |
| 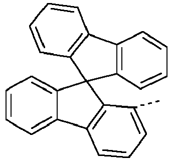 | 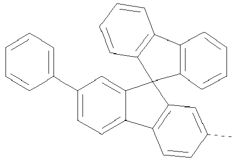 | 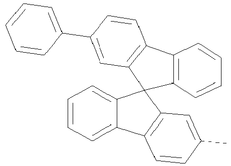 |
| Ar-210 | Ar-211 | Ar-212 |
| 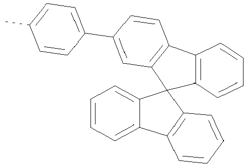 | 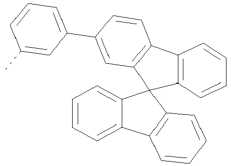 | 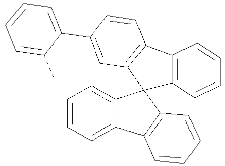 |
| Ar-213 | Ar-214 | Ar-215 |
| 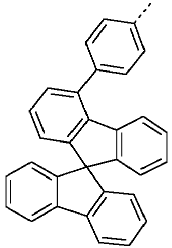 | 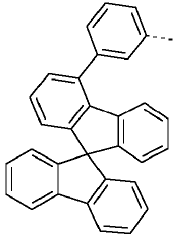 | 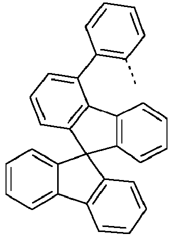 |
| Ar-216 | Ar-217 | Ar-218 |
| 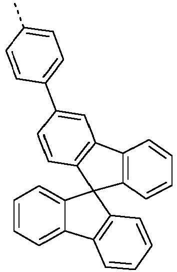 | 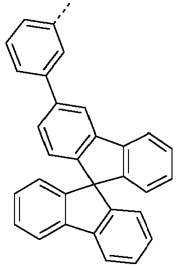 | 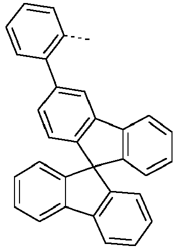 |
| Ar-219 | Ar-220 | Ar-221 |
| 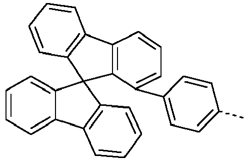 | 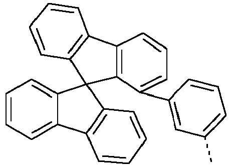 | 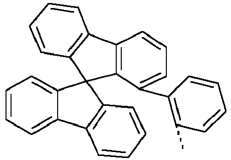 |
| Ar-222 | Ar-223 | Ar-224 |
| 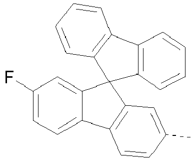 | 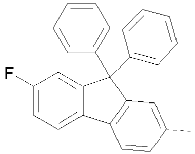 | 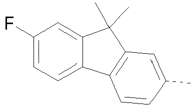 |
| Ar-225 | Ar-226 | Ar-227 |
| 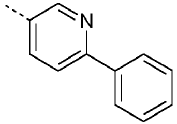 | 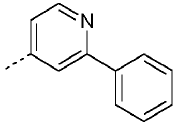 | 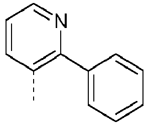 |
| Ar-228 | Ar-229 | Ar-230 |
| 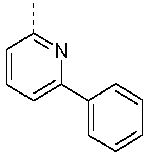 | 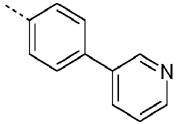 | 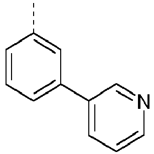 |
| Ar-231 | Ar-232 | Ar-233 |
| 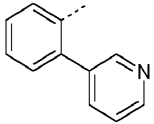 | 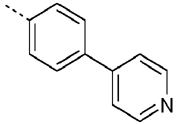 | 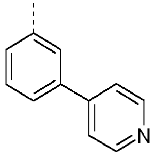 |
| Ar-234 | Ar-235 | Ar-236 |
| 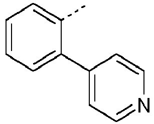 | 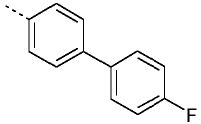 | 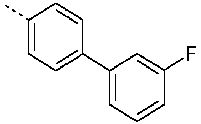 |
| Ar-237 | Ar-238 | Ar-239 |
| 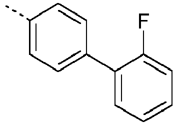 | 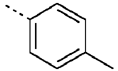 | 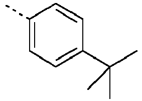 |
| Ar-240 | Ar-241 | Ar-242 |
| 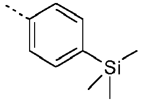 | 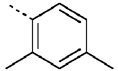 | 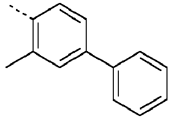 |
| Ar-243 | Ar-244 | Ar-245 |
| 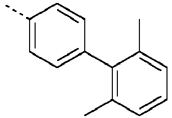 | 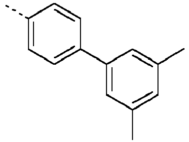 | 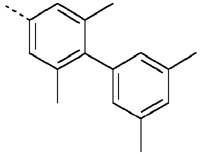 |
| Ar-246 | Ar-247 | Ar-248 |
| 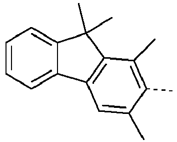 | 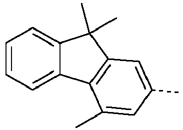 | 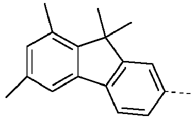 |
| Ar-250 | Ar-251 | Ar-252 |
| 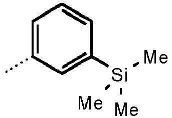 | 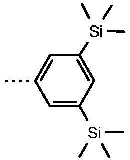 | 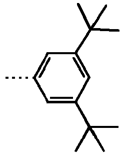 |
| Ar-253 | Ar-254 | Ar-255 |
| 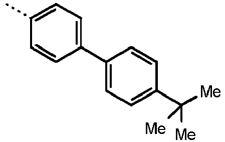 | 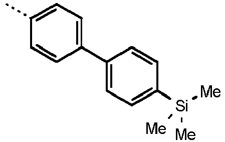 | 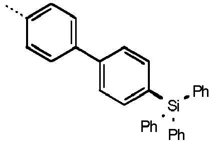 |
| Ar-256 | Ar-257 | Ar-258 |
| 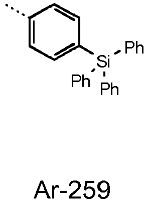 | 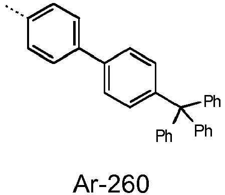 | |
| Ar-259 | Ar-260 | |

wobei die Gruppen an allen freien Positionen jeweils mit einem Rest R⁵ substituiert sein können, und wobei die gestrichelte Bindung die Bindung an das Amin-Stickstoffatom ist.

Es ist bevorzugt, dass an ein Amin-Stickstoffatom jeweils zwei unterschiedliche Gruppen Ar² gebunden sind.

Die Gruppe E ist bevorzugt eine Einfachbindung oder eine Gruppe C(R⁴)₂, besonders bevorzugt eine Einfachbindung.

Bevorzugt ist m=0, so dass keine Gruppe E vorhanden ist.

Gemäß einer alternativen Ausführungsform, die ebenfalls bevorzugt ist, ist m=1, so dass die Gruppen Ar² miteinander über eine Gruppe E verbunden sind. In diesem Fall ist es bevorzugt, dass die Gruppen Ar² gewählt sind aus Phenyl und Fluorenyl, welche jeweils mit einem oder mehreren Resten R⁵ substituiert sein können. Weiterhin ist es in diesem Fall bevorzugt, dass die Gruppe E, welche die beiden Gruppen Ar² miteinander verbindet, an den betreffenden Gruppen Ar² in ortho-Position zur Bindung der Gruppe Ar² an das Amin-Stickstoffatom gebunden ist. Weiterhin ist es bevorzugt, dass die Gruppe E mit den Gruppen Ar² einen Sechsring bildet, falls E gewählt ist aus C(R⁵)₂, Si(R⁵)₂, NR⁵, O and S; und einen Fünfring bildet, falls E eine Einfachbindung ist.

Bevorzugte Ausführungsformen der Einheit 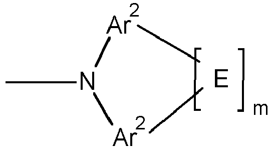 für m=1 sind die im Folgenden abgebildeten Gruppen:

| | | |
|---|---|---|
| 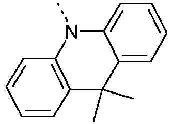 | 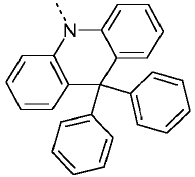 | 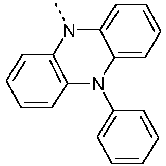 |
| N-1 | N-2 | N-3 |
| 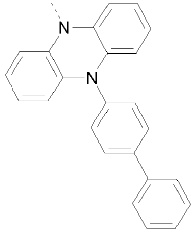 | 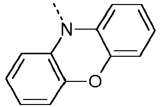 | 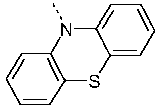 |
| N-4 | N-5 | N-6 |
| 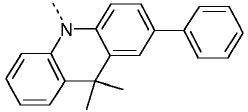 | 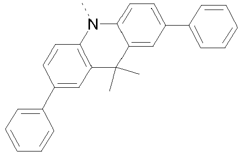 | 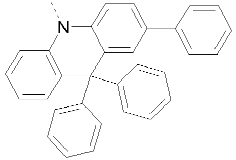 |
| N-7 | N-8 | N-9 |
| 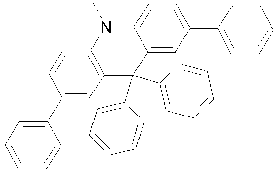 | 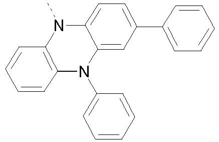 | 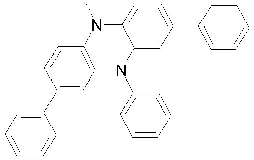 |
| N-10 | N-11 | N-12 |
| 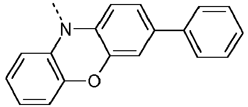 | 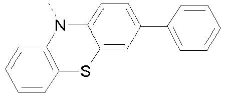 | 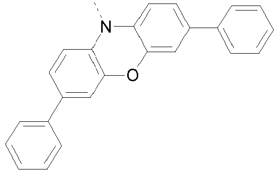 |
| N-13 | N-14 | N-15 |
| 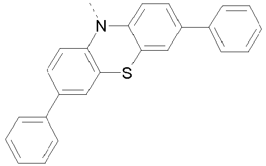 | 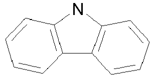 | 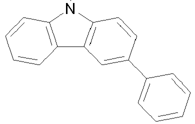 |
| N-16 | N-17 | N-18 |
| 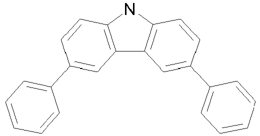 | 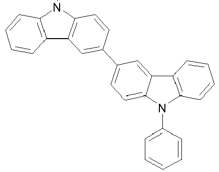 | 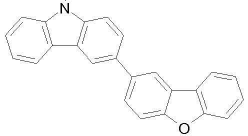 |
| N-19 | N-20 | N-21 |
| 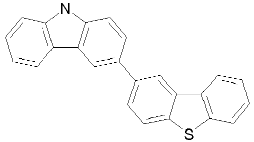 | 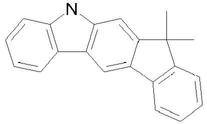 | 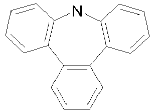 |
| N-22 | N-23 | N-24 |
| 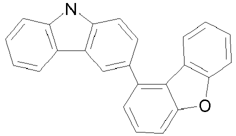 | 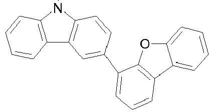 | 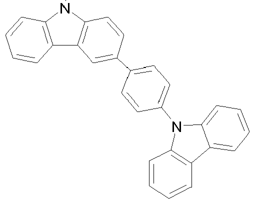 |
| N-25 | N-26 | N-27 |
| 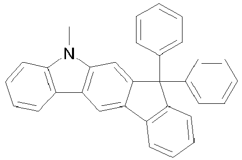 | 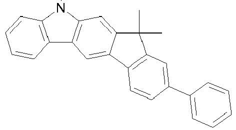 | 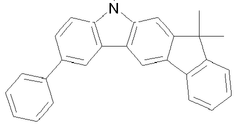 |
| N-28 | N-29 | N-30 |
| 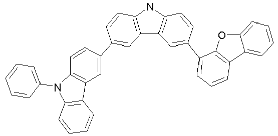 | 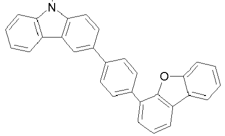 | |
| N-31 | N-32 | |

wobei die Gruppen an ihren freien Positionen jeweils mit einem Rest R⁵ substituiert sein können, und bevorzugt in den freien Positionen unsubstituiert sind, und wobei die gestrichelten Bindungen die Bindungen an den Rest der Formel darstellen.

Bevorzugte Ausführungsformen der Gruppe 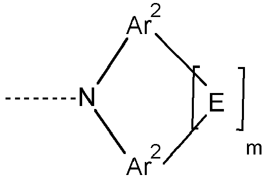 für m=0 sind die im Folgenden abgebildeten Gruppen:

| | |
|---|---|
| 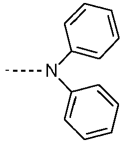 | 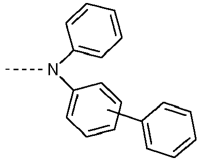 |
| A-1 | A-2 |
| 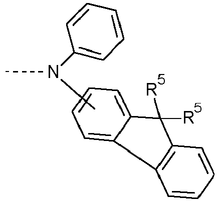 | 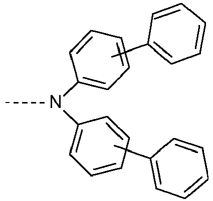 |
| A-3 | A-4 |
| 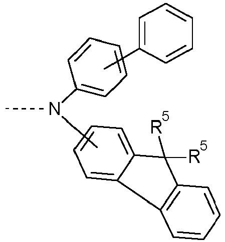 | 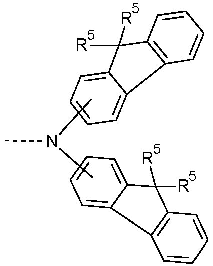 |
| A-5 | A-6 |
| 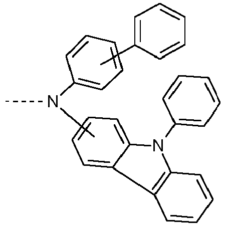 | 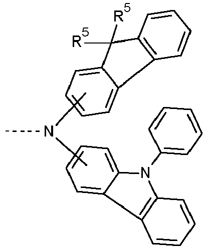 |
| A-7 | A-8 |
| 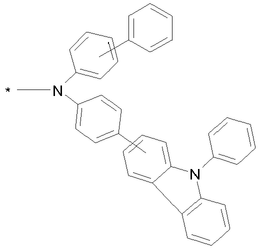 | 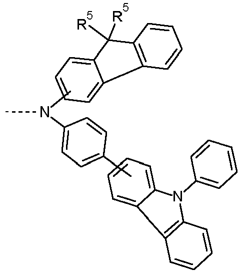 |
| A-9 | A-10 |
| 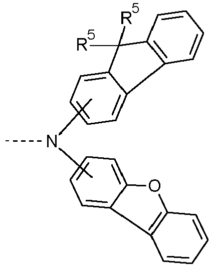 | 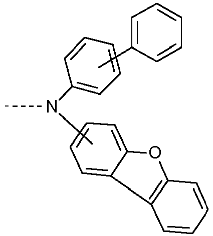 |
| A-11 | A-12 |
| 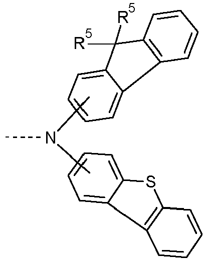 | 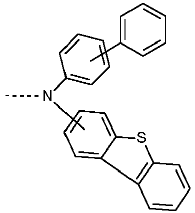 |
| A-13 | A-14 |
| 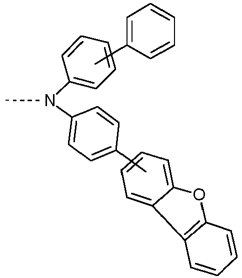 | 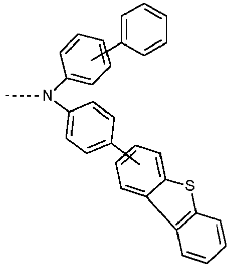 |
| A-15 | A-16 |
| 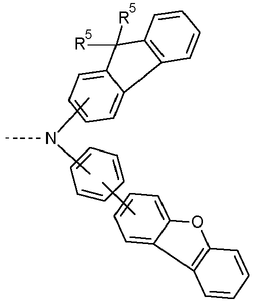 | 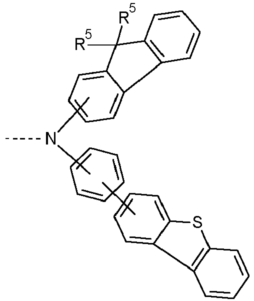 |
| A-17 | A-18 |
| 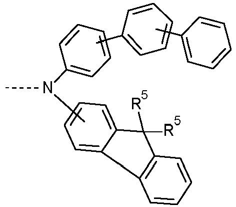 | 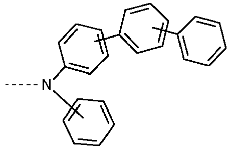 |
| A-19 | A-20 |
| 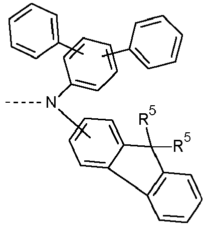 | 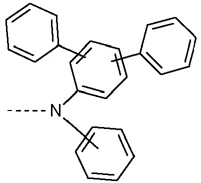 |
| A-21 | A-22 |
| 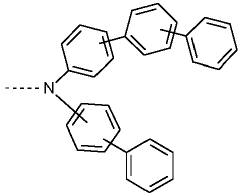 | 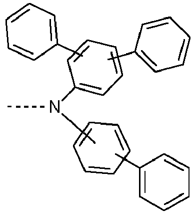 |
| A-23 | A-24 |
| 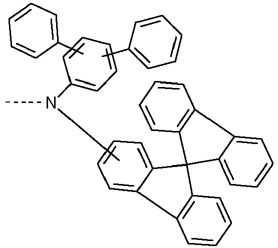 | 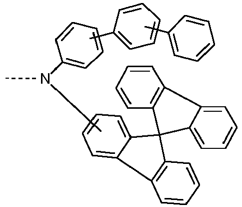 |
| A-25 | A-26 |
| 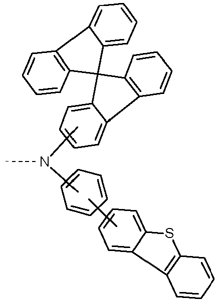 | 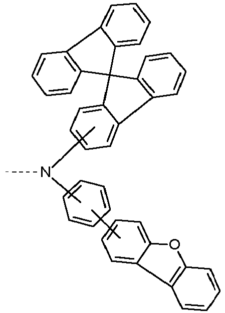 |
| A-27 | A-28 |
| 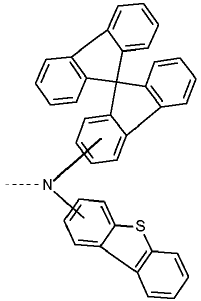 | 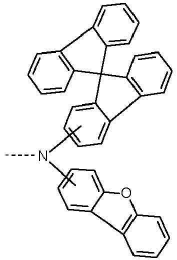 |
| A-29 | A-30 |
| 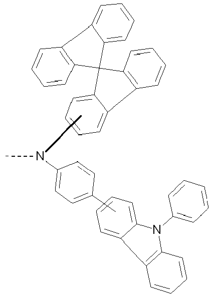 | 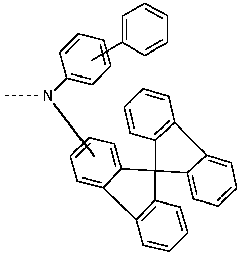 |
| A-31 | A-32 |
| 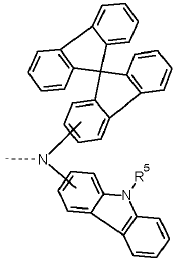 | 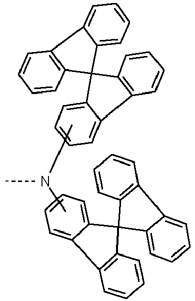 |
| A-33 | A-34 |
| 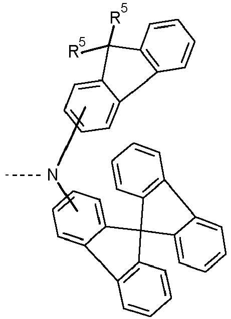 | 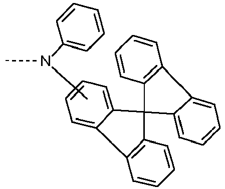 |
| A-35 | A-36 |
| 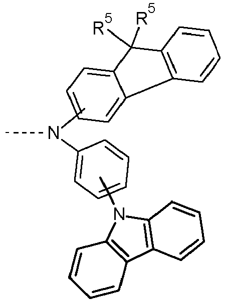 | 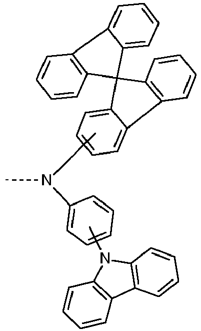 |
| A-37 | A-38 |
| 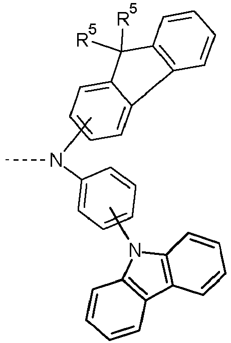 | |
| A-39 | |

wobei die Gruppen an ihren freien Positionen jeweils mit einem Rest R⁵ substituiert sein können, und bevorzugt in den freien Positionen unsubstituiert sind, und wobei die gestrichelten Bindungen die Bindungen an den Rest der Formel darstellen.

R⁰ ist bevorzugt H.

R¹ ist bevorzugt bei jedem Auftreten gleich oder verschieden gewählt aus monovalenten Gruppen abgeleitet von Benzol, Biphenyl, Terphenyl, Quaterphenyl, Naphthalin, Fluoren, insbesondere 9,9'-Dimethylfluoren und 9,9'-Diphenylfluoren, Benzofluoren, Spirobifluoren, Indenofluoren, Indenocarbazol, Dibenzofuran, Dibenzothiophen, Benzocarbazol, Carbazol, Benzofuran, Benzothiophen, Indol, Chinolin, Pyridin, Pyrimidin, Pyrazin, Pyridazin, und Triazin, wobei die monovalenten Gruppen jeweils mit einem oder mehreren Resten R⁶ substituiert sein können. Wenn Reste R⁶ in den Gruppen vorliegen, ist es bevorzugt, wenn ein oder zwei Reste R⁶ gleich N(R⁷)₂ sind, wobei R⁷ gewählt ist aus aromatischen Ringsystemen mit 6 bis 40 aromatischen Ringatomen und heteroaromatischen Ringsystemen mit 5 bis 40 aromatischen Ringatomen. Besonders bevorzugte Gruppen R¹ sind gewählt aus Phenyl, mit einer oder zwei Gruppen -N(R⁷)₂ substituiertes Phenyl, Biphenyl, N-gebundenes Carbazolyl, C-gebundenes Carbazolyl, Naphthyl, Dibenzofuranyl und Dibenzothiophenyl, welche jeweils mit einem oder mehreren Resten R⁶ substituiert sein können und bevorzugt unsubstituiert sind.

R² ist bevorzugt bei jedem Auftreten gleich oder verschieden gewählt aus H, D, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen, wobei die genannten Alkylgruppen und die genannten aromatischen Ringsysteme und heteroaromatischen Ringsysteme jeweils mit einem oder mehreren Resten R⁶ substituiert sein können. Besonders bevorzugt ist R² gleich H.

R³ ist bevorzugt bei jedem Auftreten gleich oder verschieden gewählt aus H, D, F, CN, Si(R⁶)₃, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei in den genannten Alkylgruppen eine oder mehrere CH₂-Gruppen durch -C=C-, -R⁶C=CR⁶-, Si(R⁶)₂, C=O, C=NR⁶, -NR⁶-, -O-, -S-, -C(=O)O- oder- C(=O)NR⁶- ersetzt sein können. Besonders bevorzugt ist R³ gleich H.

R⁴, R⁵ sind bevorzugt bei jedem Auftreten gleich oder verschieden gewählt aus H, D, F, CN, Si(R⁶)₃, N(R⁶)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁶ substituiert sein können; und wobei in den genannten Alkylgruppen eine oder mehrere CH₂-Gruppen durch -C=C-, -R⁶C=CR⁶-, Si(R⁶)₂, C=O, C=NR⁶, -NR⁶-, -O-, -S-, -C(=O)O- oder -C(=O)NR⁶- ersetzt sein können.

Reste R⁴, die an eine Gruppe X¹ = C(R⁴)₂ gebunden sind, sind bevorzugt gewählt aus Alkylgruppen mit 1 bis 20 C-Atomen, aromatischen

Ringsystemen mit 6 bis 20 aromatischen Ringatomen und heteroaromatischen Ringsystemen mit 5 bis 20 aromatischen Ringatomen, wobei die die genannten Alkylgruppen, die genannten aromatischen Ringsysteme und die genannten heteroaromatischen Ringsysteme jeweils mit einem oder mehreren Resten R⁶ substituiert sein können. Gemäß einer bevorzugten Ausführungsform bilden die zwei Reste R⁴ einer Gruppe X¹ = C(R⁴)₂ miteinander einen Ring, so dass das C-Atom der Gruppe X¹ = C(R⁴)₂ ein Spiro-Atom ist. Der gebildete Ring ist in diesem Fall bevorzugt ein Cycloalkyl-Ring oder ein Ring der folgenden Struktur 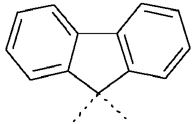 wobei die gestrichelten Bindungen die Bindungen von der Gruppe X¹ zum Rest der Verbindung kennzeichnen.

Bevorzugt ist R⁶ bei jedem Auftreten gleich oder verschieden gewählt aus H, D, F, CN, Si(R⁷)₃, N(R⁷)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁷ substituiert sein können; und wobei in den genannten Alkyl- oder Alkoxygruppen eine oder mehrere CH₂-Gruppen durch -C=C-, - R⁷C=CR⁷-, Si(R⁷)₂, C=O, C=NR⁷, -NR⁷-, -O-, -S-, -C(=O)O- oder -C(=O)NR⁷- ersetzt sein können.

Bevorzugte Ausführungsformen der Formel (I) entsprechen den folgenden Formeln

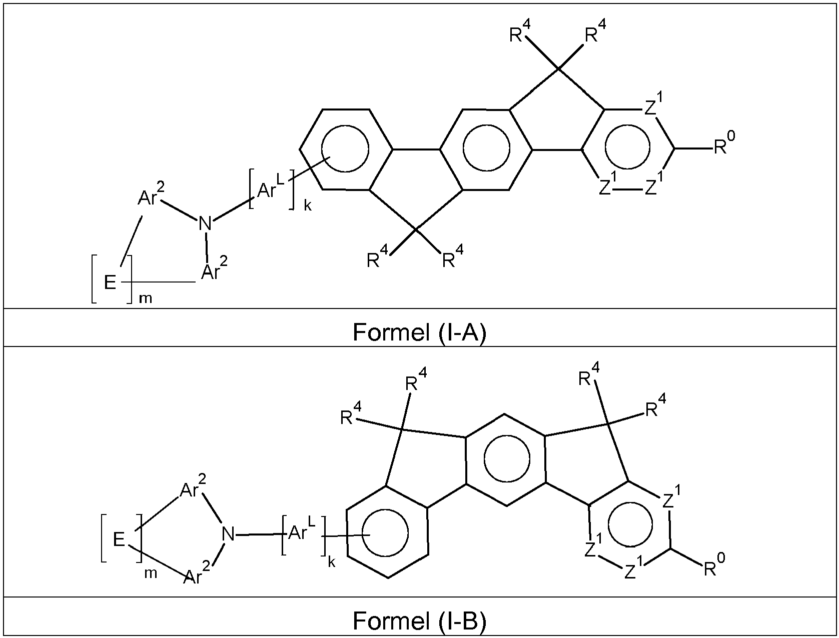

wobei alle unsubstituiert gezeichneten Positionen an den Benzolringen jeweils mit einem Rest R³ substituiert sein können.

Bevorzugt ist in den Formeln (I-A) und (I-B) die Gruppe 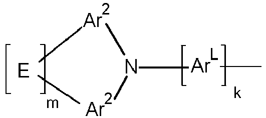 jeweils in der Position para zur Bindung an die benachbarte Benzolgruppe gebunden, was Formeln (I-A-a) bzw. (I-B-a) entspricht:

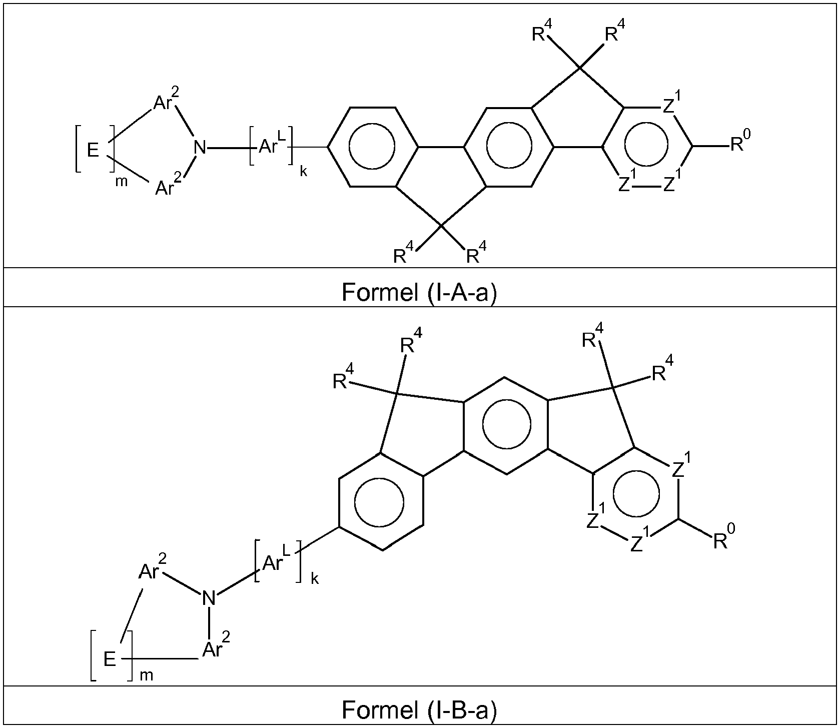

wobei alle unsubstituiert gezeichneten Positionen an den Benzolringen jeweils mit einem Rest R³ substituiert sein können.

Bevorzugte Ausführungsformen der Formel (II) entsprechen den folgenden Formeln

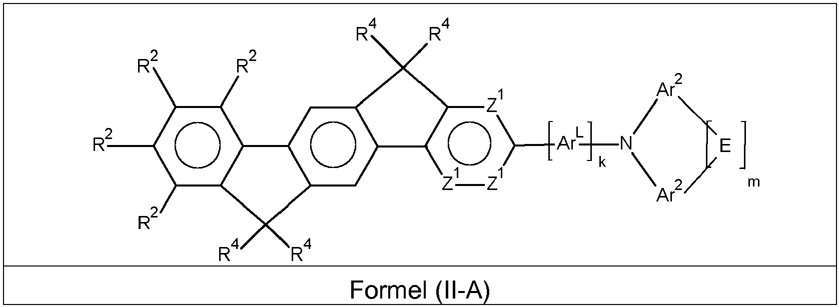

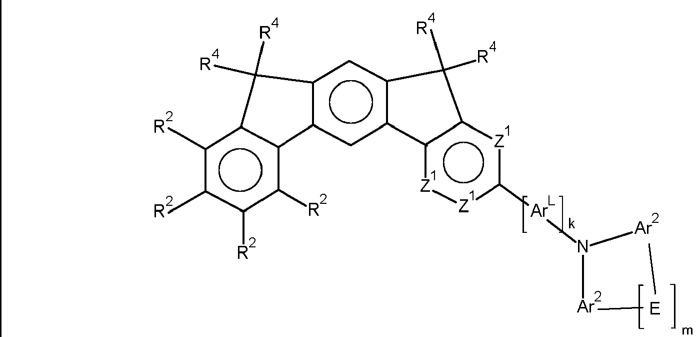

wobei alle unsubstituiert gezeichneten Positionen an den Benzolringen jeweils mit einem Rest R³ substituiert sein können.

Weitere bevorzugte Ausführungsformen der Formel (I) entsprechen einer der folgenden Formeln

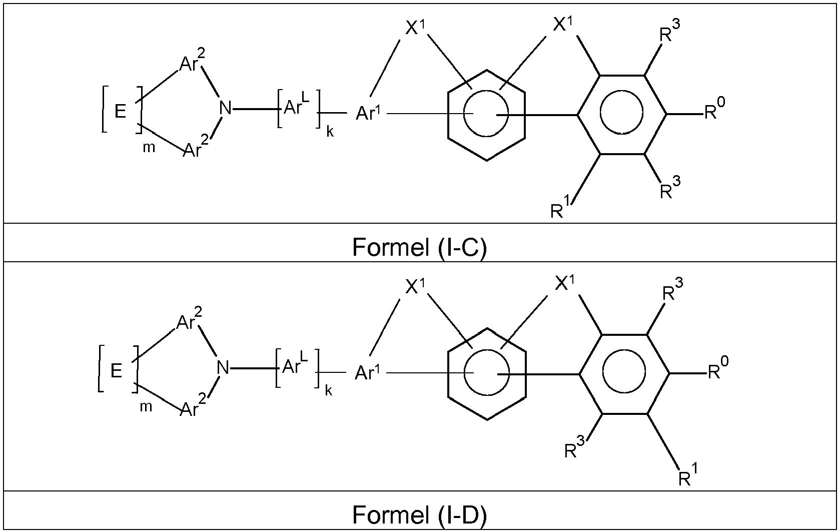

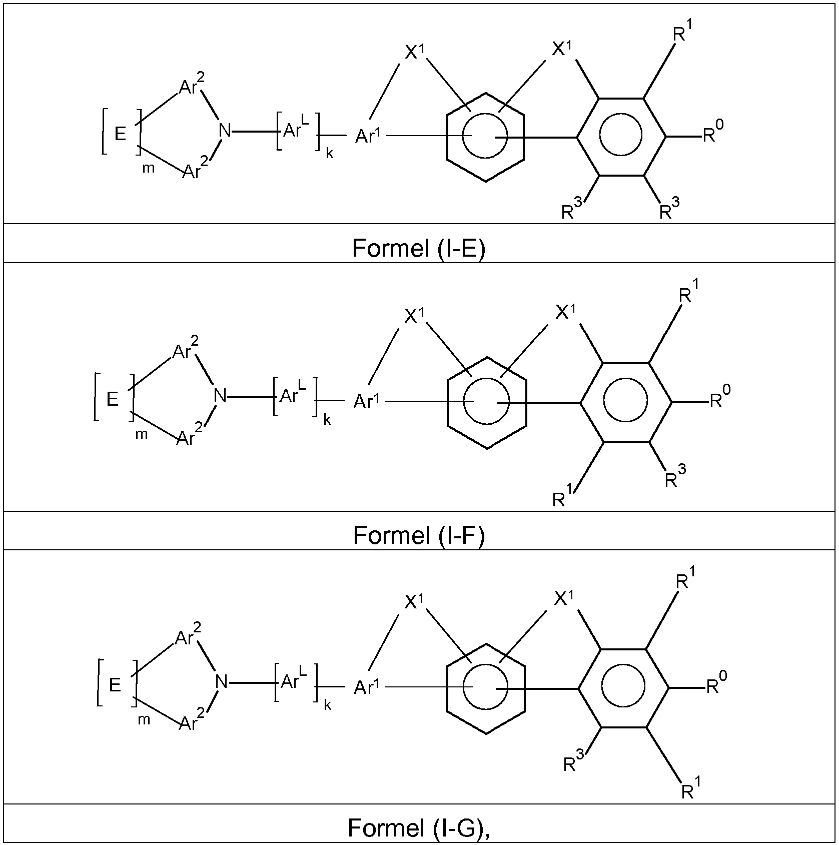

wobei die auftretenden Variablen definiert sind wie oben, und wobei R⁰ und R³ bevorzugt gleich H sind. Weiterhin bevorzugt ist in diesen Formeln k=0 und m=0, und Ar¹ ist eine Phenylgruppe, die mit einem oder mehreren Resten R³ substituiert sein kann.

Bevorzugte Ausführungsformen der Formel (I-A) und (I-B) entsprechen den folgenden Formeln

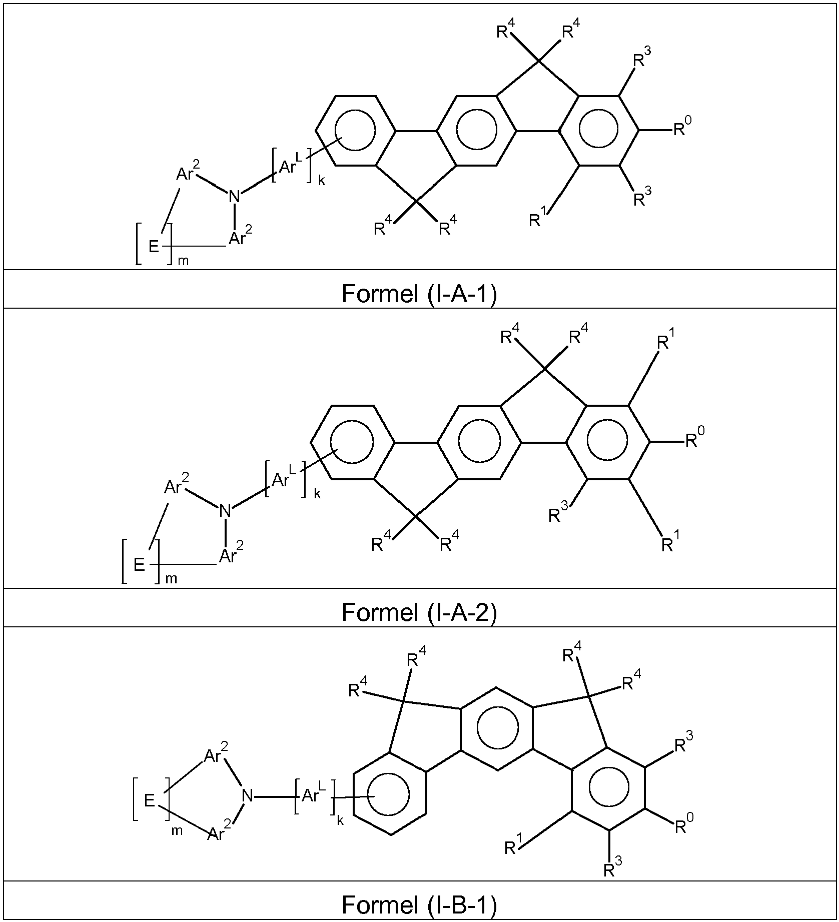

wobei die auftretenden Variablen definiert sind wie oben. Bevorzugt ist in diesen Formeln k=0 und m=0. Weiterhin bevorzugt ist in diesen Formeln R³ und R⁰ gleich H. Bevorzugt ist in den Formeln (I-A-1), (I-A-2) und (I-B-1) die Gruppe 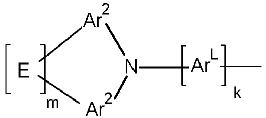 jeweils in der Position para zur Bindung an die benachbarte Benzolgruppe gebunden, was den folgenden Formeln entspricht:

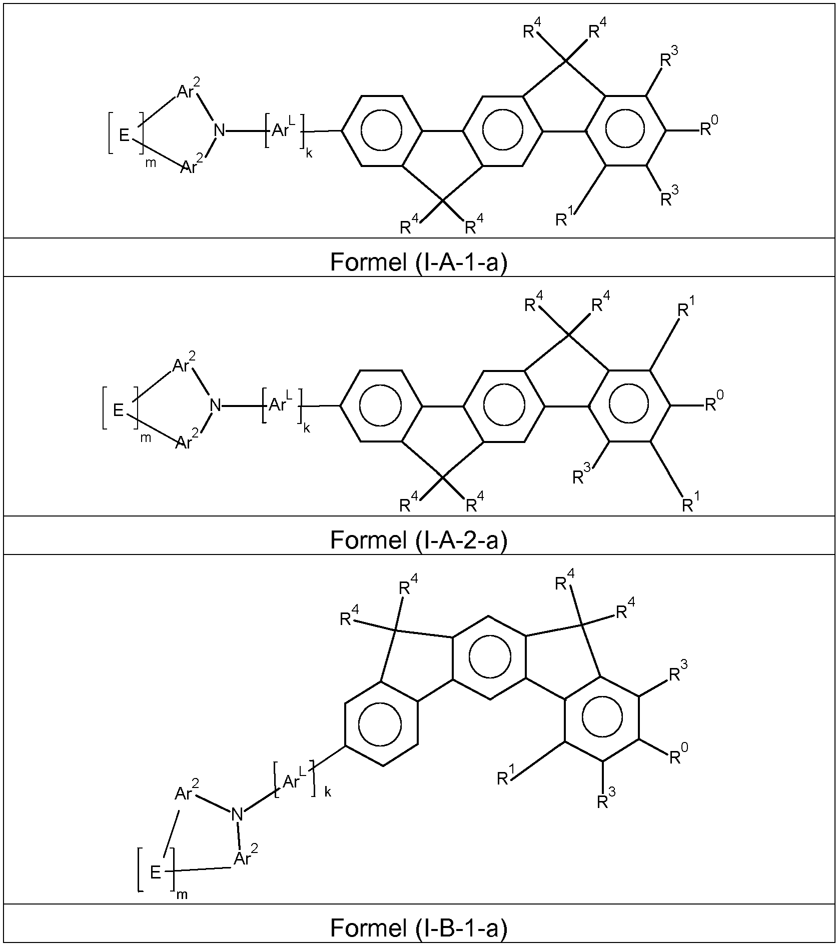

wobei die auftretenden Variablen definiert sind wie oben.

Bevorzugte Ausführungsformen der Formeln (I-A-1), (I-A-2) und (I-B-1) sind die folgenden Formeln

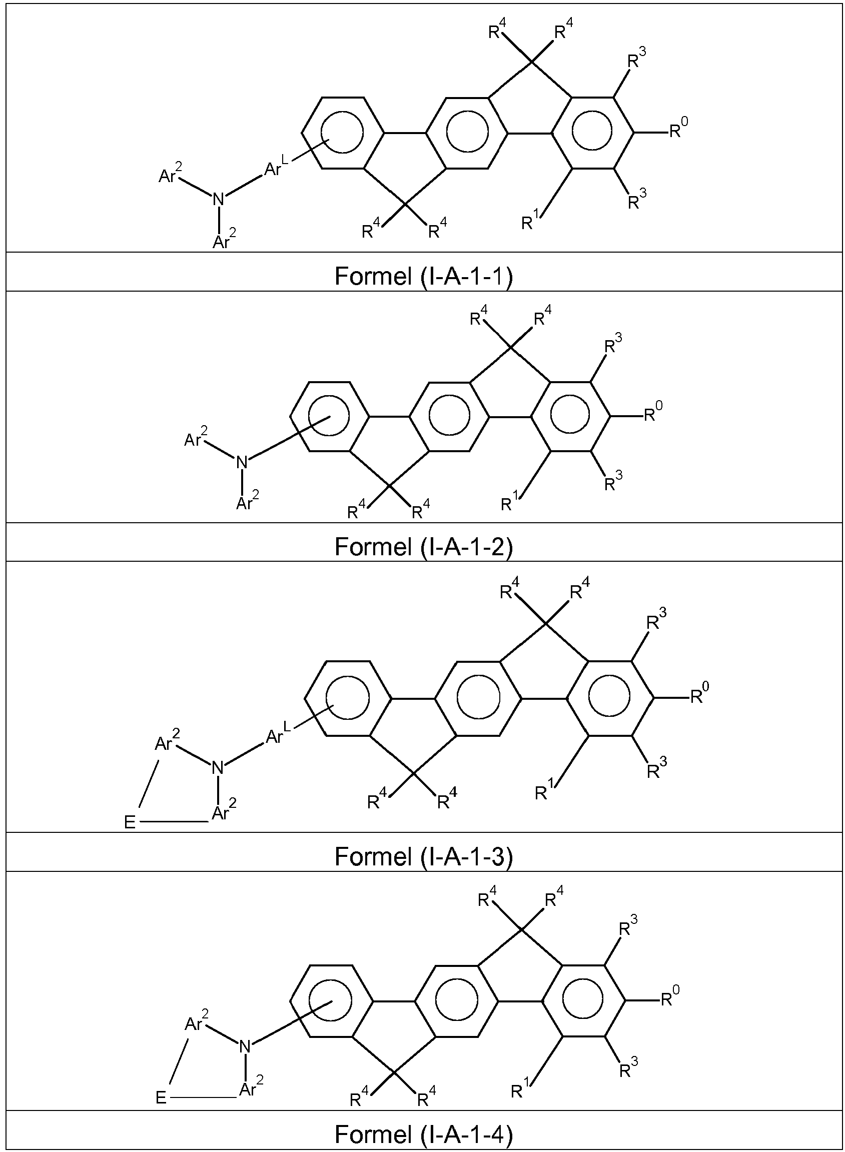

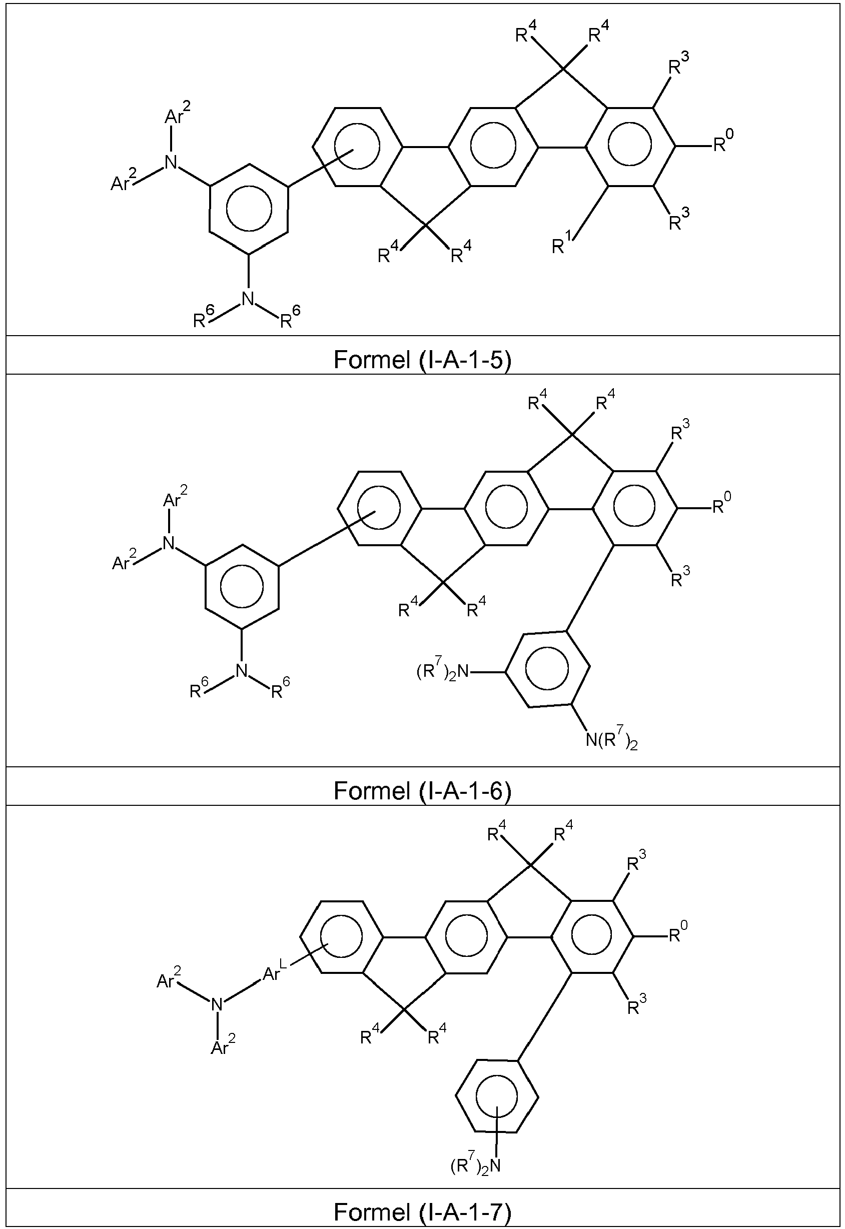

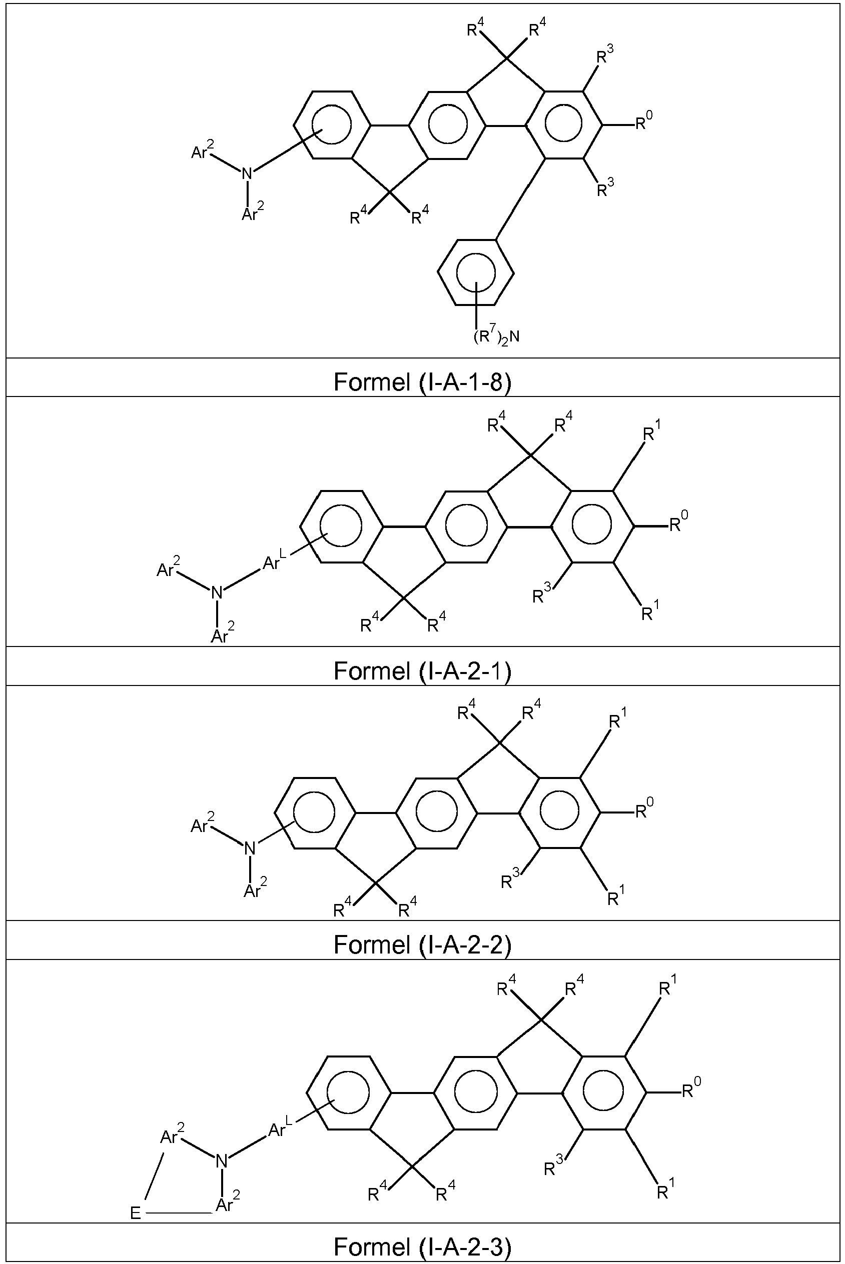

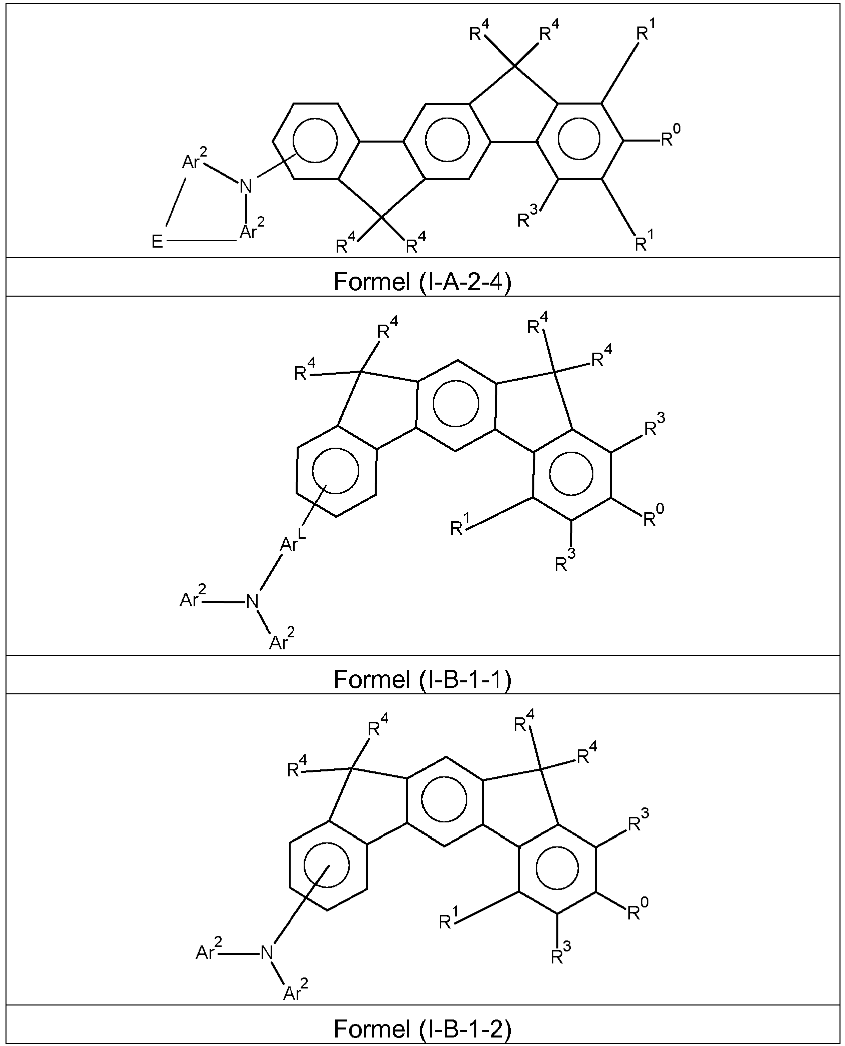

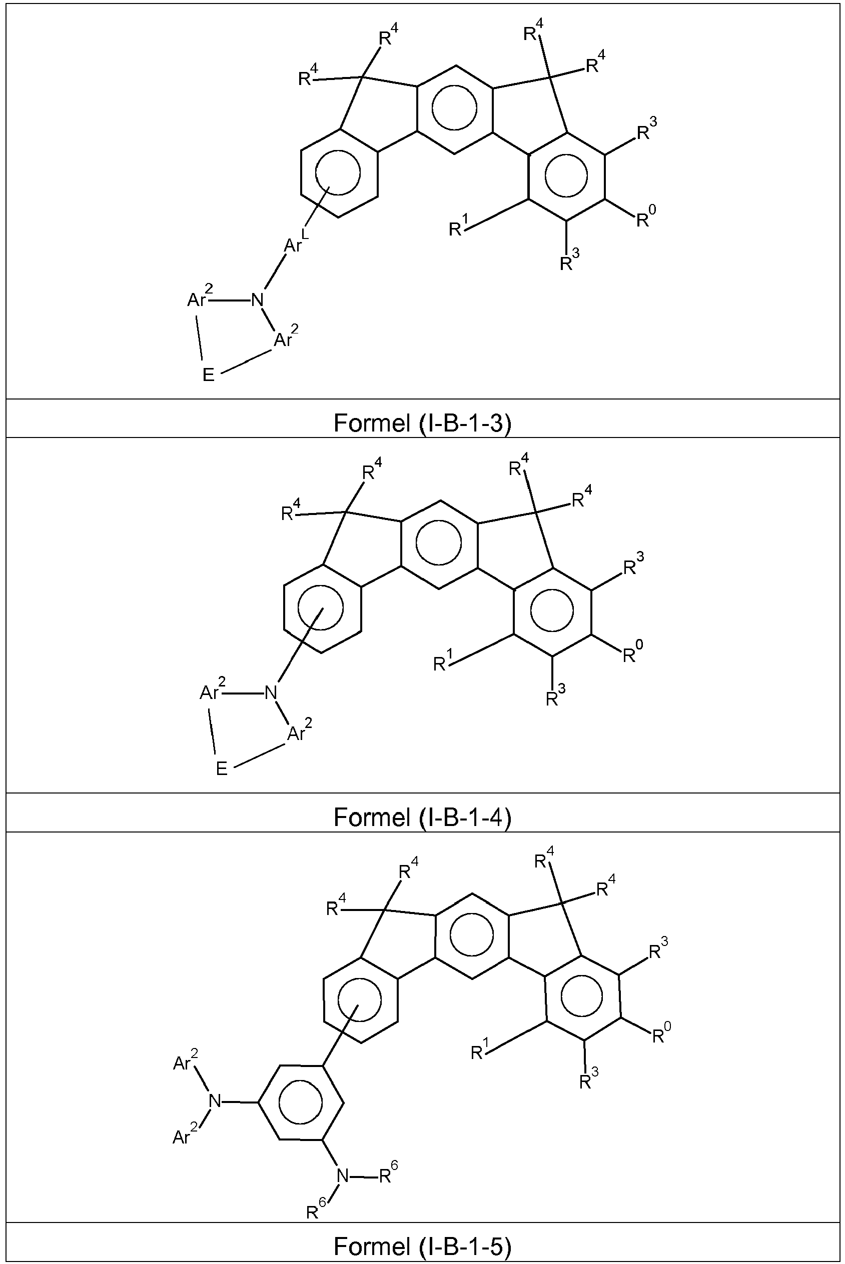

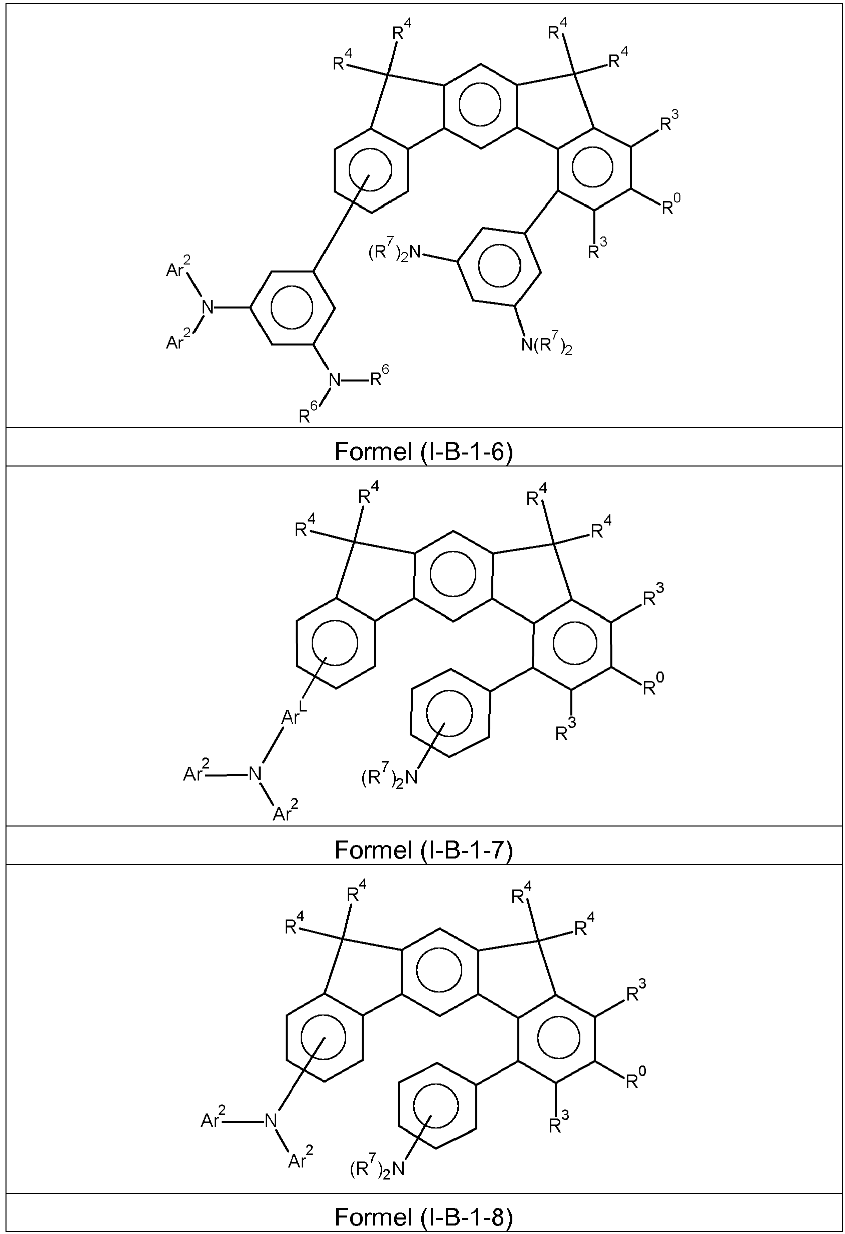

wobei die auftretenden Variablen definiert sind wie oben, und wobei R⁰ und R³ bevorzugt gleich H sind. In Formeln (I-A-1-3), (I-A-1-4), (I-A-2-3), (I-A-2-4), (I-B-1-3) und (I-B-1-4) ist E bevorzugt gewählt aus Einfachbindung und C(R⁴)₂, besonders bevorzugt ist E eine Einfachbindung. Ar^{L} ist in Formeln (I-A-1-1), (I-A-1-3), (I-A-1-7), (I-A-2-1), (I-A-2-3), (I-B-1-1), (I-B-1-3) und (I-B-1-7) bevorzugt eine Phenylgruppe, die mit einem oder mehreren Resten R⁵ substituiert sein kann. Bevorzugt ist in den oben genannten Formeln die Gruppe gewählt aus den folgenden Gruppen

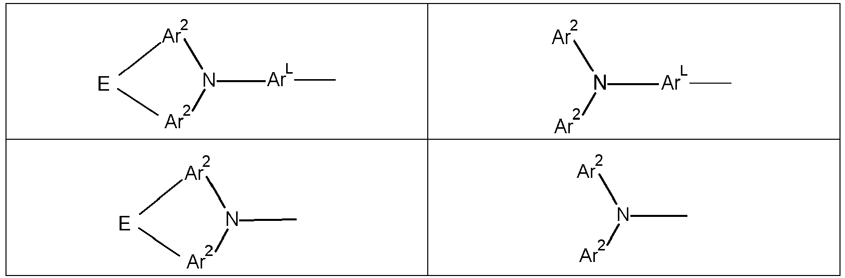

soweit vorhanden, jeweils in der Position para zur Bindung an die benachbarte Benzolgruppe gebunden.

Besonders bevorzugt unter den oben genannten Formeln sind Formeln (I-A-1-1), (I-A-1-2), (I-A-2-1), (I-A-2-2), (I-B-1-1) und (I-B-1-2), davon besonders (I-A-1-1) und (I-A-1-2), davon ganz besonders (I-A-1-2). Ganz besonders bevorzugte Formeln sind entsprechend die folgenden Ausführungsformen von Formel (I-A-1-1) und (I-A-1-2)

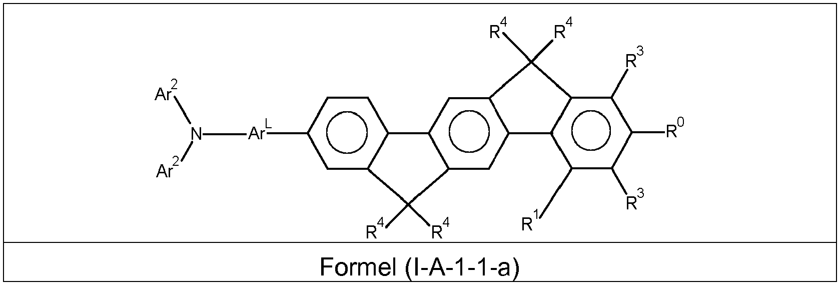

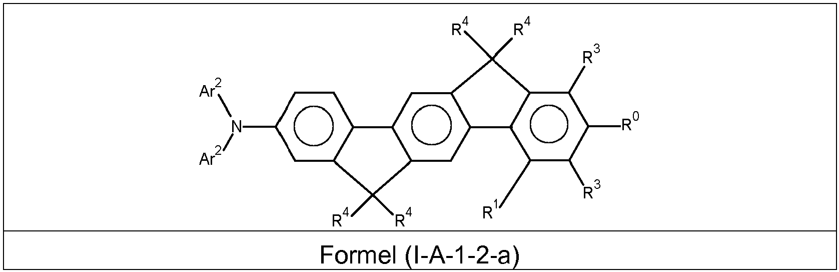

wobei Formel (I-A-1-2-a) am stärksten bevorzugt ist.

Bevorzugte Ausführungsformen der Formel (II-A) entsprechen den folgenden Formeln

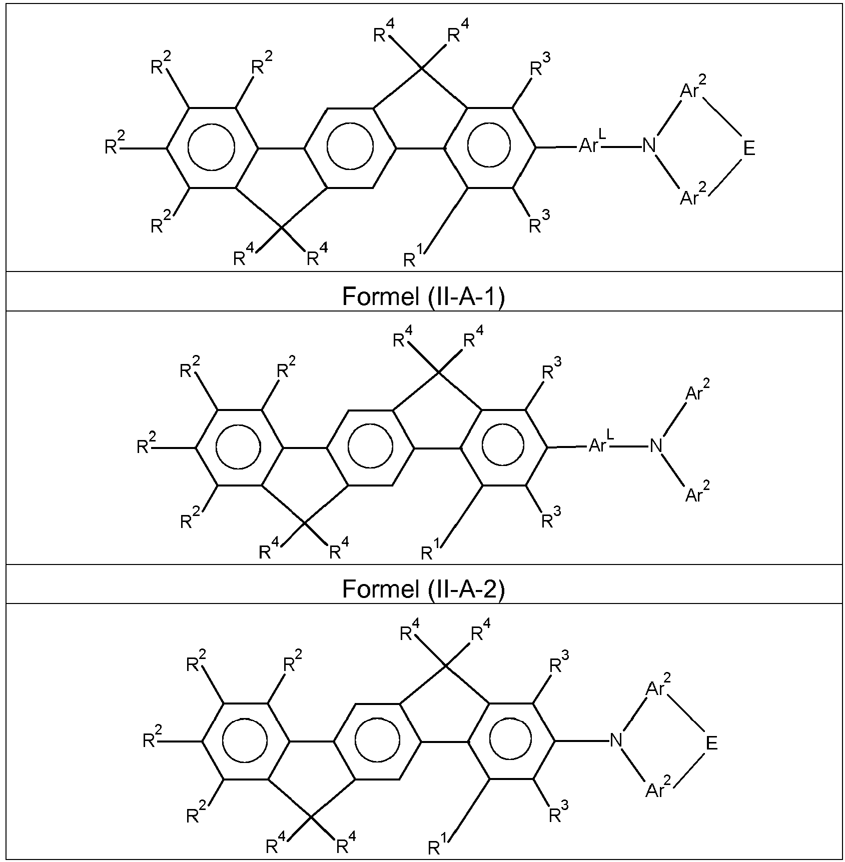

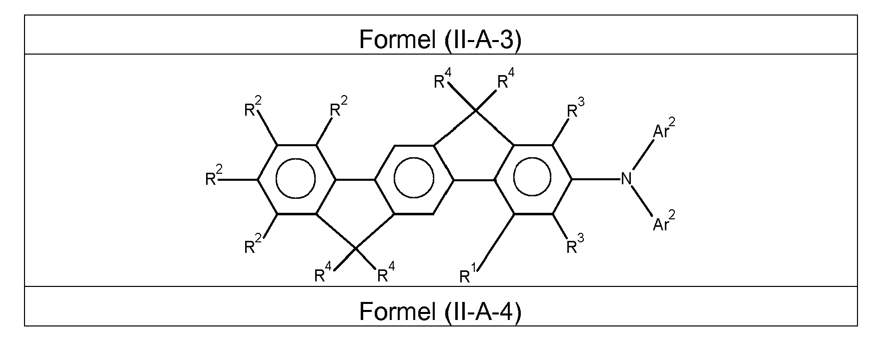

wobei die auftretenden Variablen definiert sind wie oben, und wobei R² und R³ bevorzugt gleich H sind. In Formeln (II-A-1) und (II-A-3) ist E bevorzugt gewählt aus Einfachbindung und C(R⁴)₂, besonders bevorzugt ist E eine Einfachbindung. Ar^{L} ist in Formeln (II-A-1) und (II-A-2) bevorzugt eine Phenylgruppe, die mit einem oder mehreren Resten R⁵ substituiert sein kann. Besonders bevorzugt unter den oben genannten Formeln ist Formel (II-A-4).

Bevorzugte Verbindungen gemäß der vorliegenden Erfindung sind im Folgenden abgebildet:

| | | |
|---|---|---|
| 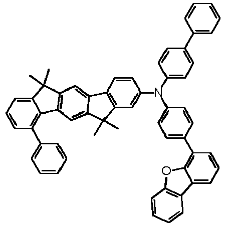 | 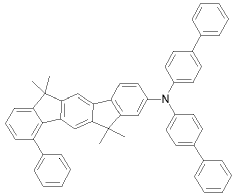 | 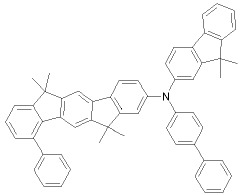 |
| 1 | 2 | 3 |
| 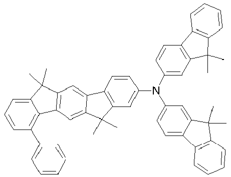 | 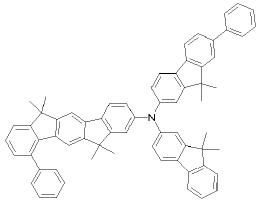 | 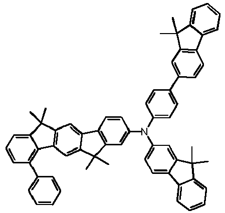 |
| 4 | 5 | 6 |
| 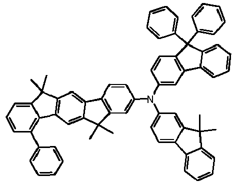 | 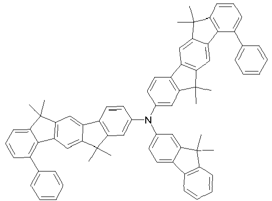 | 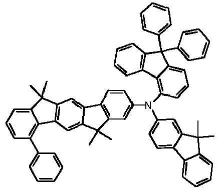 |
| 7 | 8 | 9 |
| 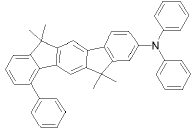 | 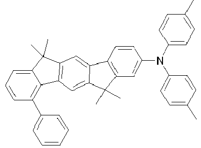 | 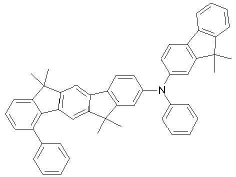 |
| 10 | 11 | 12 |
| 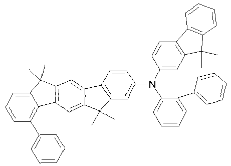 | 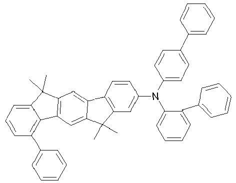 | 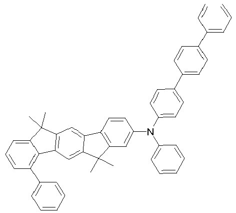 |
| 13 | 14 | 15 |
| 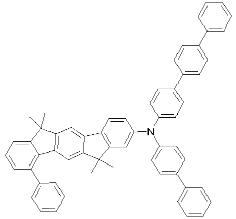 | 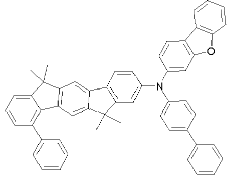 | 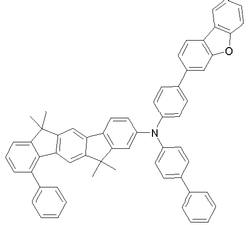 |
| 16 | 17 | 18 |
| 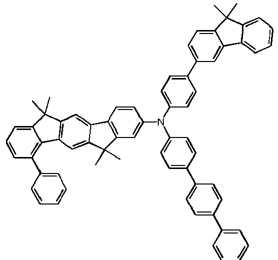 | 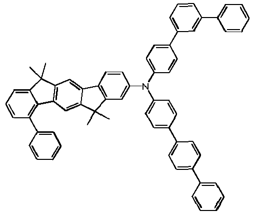 | 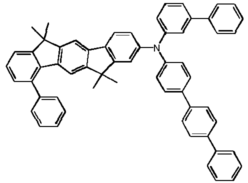 |
| 19 | 20 | 21 |
| 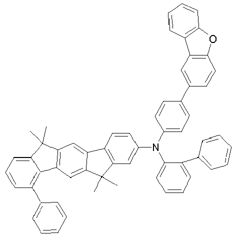 | 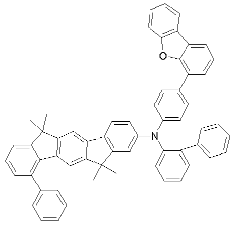 | 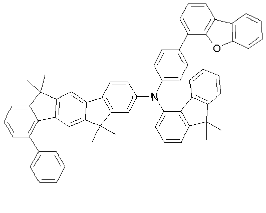 |
| 22 | 23 | 24 |
| 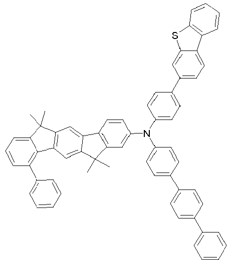 | 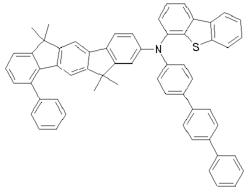 | 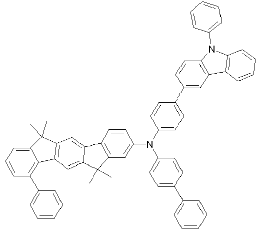 |
| 25 | 26 | 27 |
| 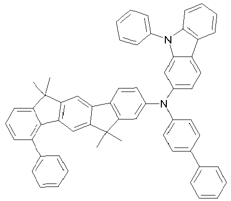 | 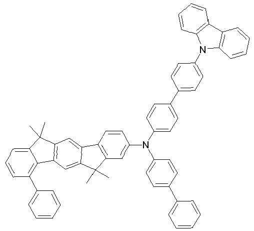 | 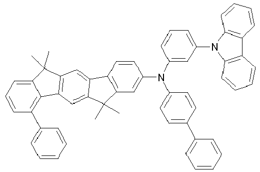 |
| 28 | 29 | 30 |
| 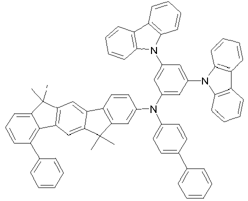 | 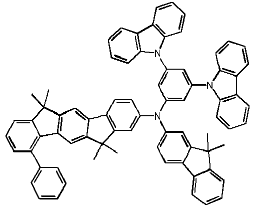 | 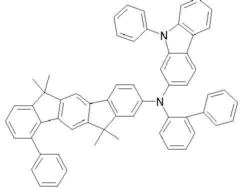 |
| 31 | 32 | 33 |
| 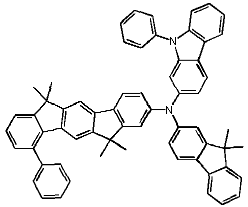 | 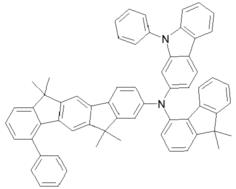 | 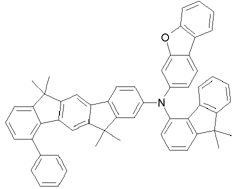 |
| 34 | 35 | 36 |
| 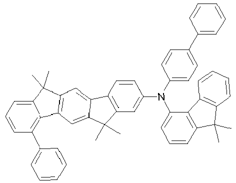 | 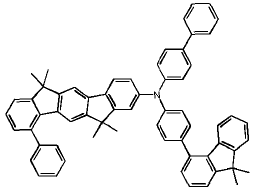 | 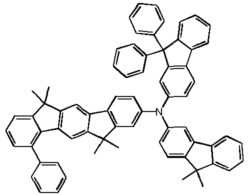 |
| 37 | 38 | 39 |
| 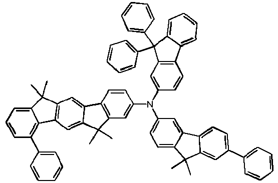 | 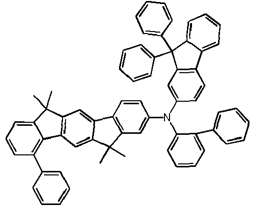 | 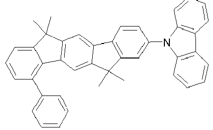 |
| 40 | 41 | 42 |
| 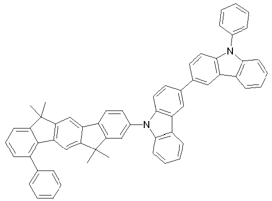 | 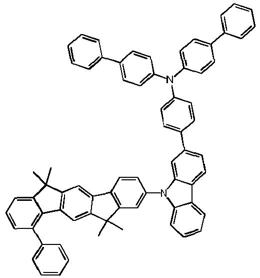 | 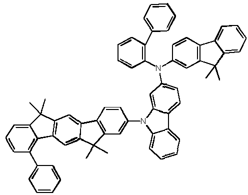 |
| 43 | 44 | 45 |
| 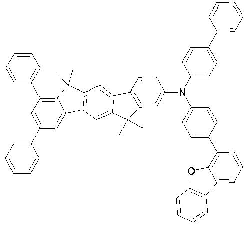 | 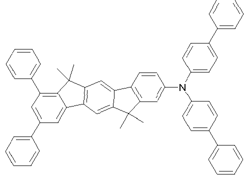 | 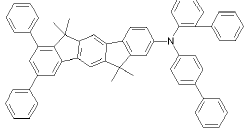 |
| 46 | 47 | 48 |
| 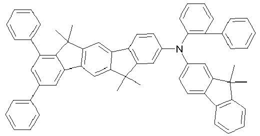 | 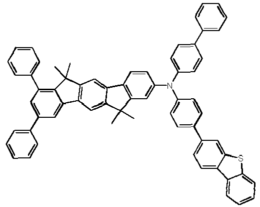 | 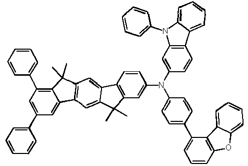 |
| 49 | 50 | 51 |
| 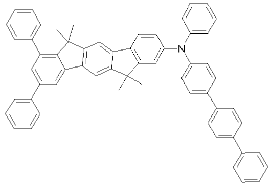 | 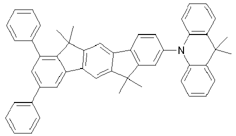 | 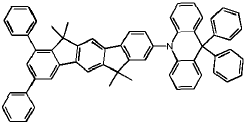 |
| 52 | 53 | 54 |
| 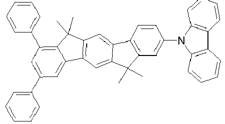 | 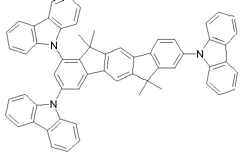 | |
| 55 | 56 | |
| 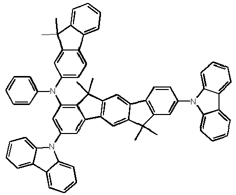 | 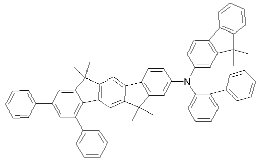 | 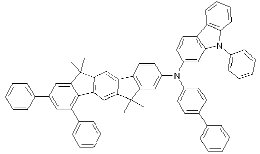 |
| 57 | 58 | 59 |
| 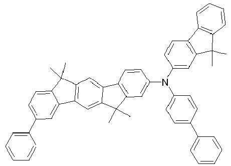 | 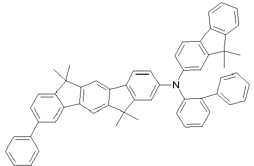 | 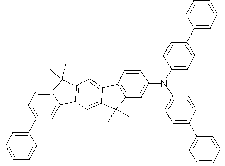 |
| 60 | 61 | 62 |
| 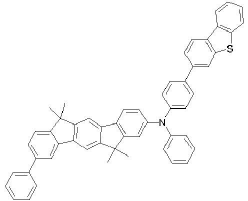 | 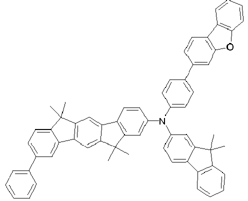 | 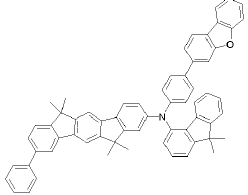 |
| 63 | 64 | 65 |
| 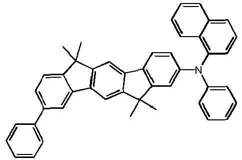 | 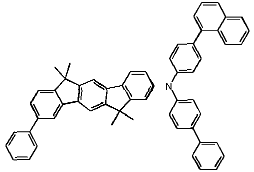 | 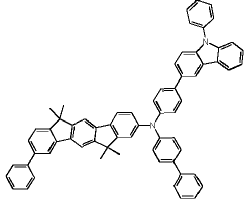 |
| 66 | 67 | 68 |
| 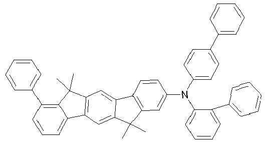 | 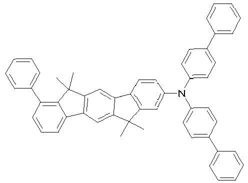 | 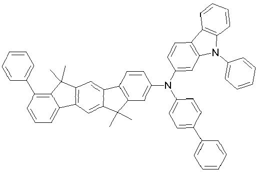 |
| 69 | 70 | 71 |
| 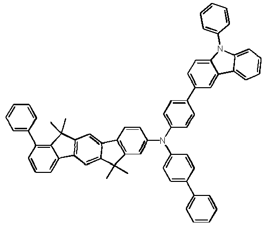 | 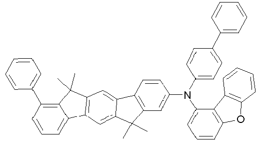 | 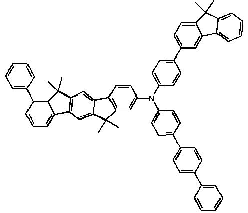 |
| 72 | 73 | 74 |
| 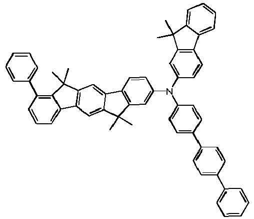 | 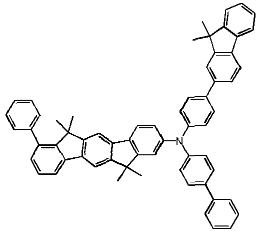 | 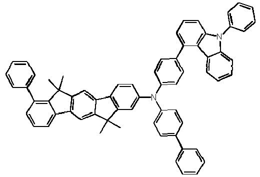 |
| 75 | 76 | 77 |
| 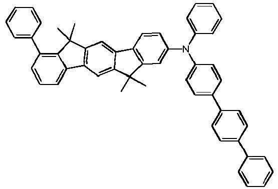 | 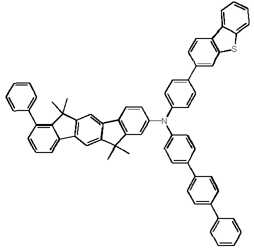 | 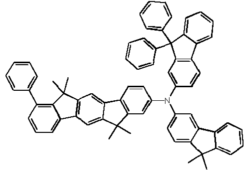 |
| 78 | 79 | 80 |
| 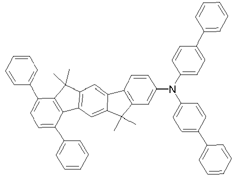 | 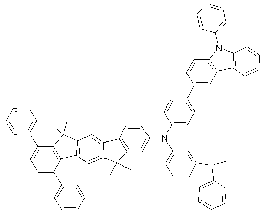 | 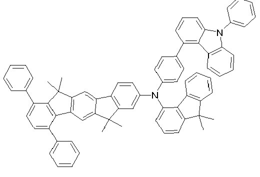 |
| 81 | 82 | 83 |
| 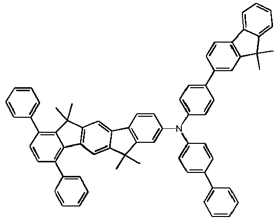 | 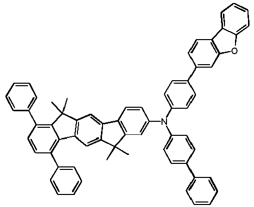 | 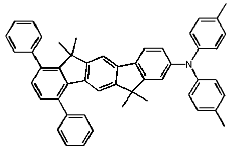 |
| 84 | 85 | 86 |
| 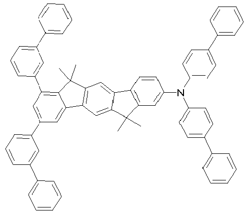 | 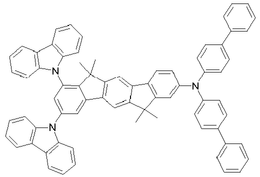 | 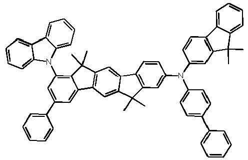 |
| 87 | 88 | 89 |
| 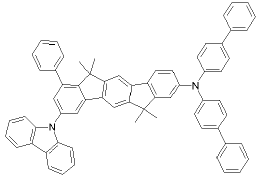 | 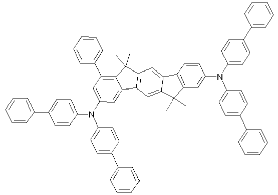 | 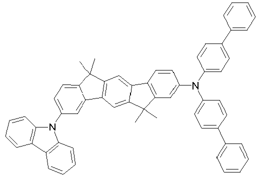 |
| 90 | 91 | 92 |
| 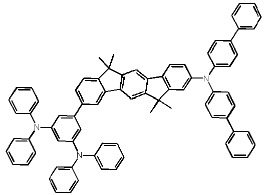 | 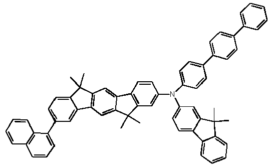 | 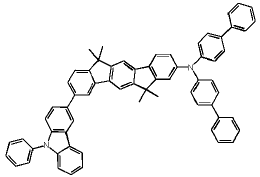 |
| 93 | 94 | 95 |
| 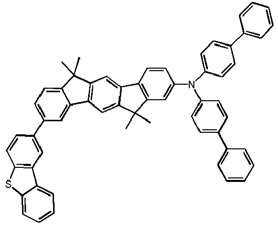 | 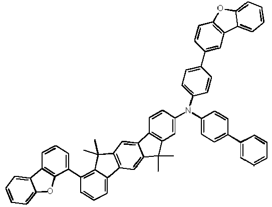 | 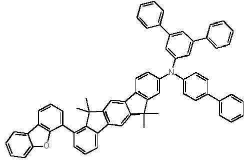 |
| 96 | 97 | 98 |
| 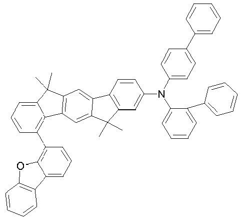 | 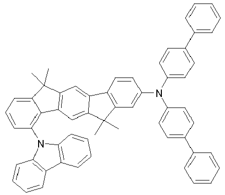 | 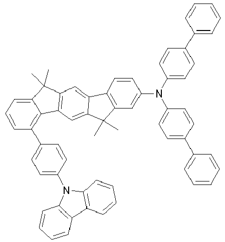 |
| 99 | 100 | 101 |
| 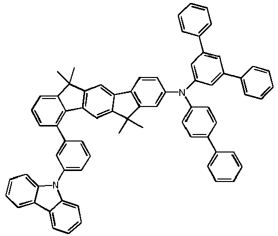 | 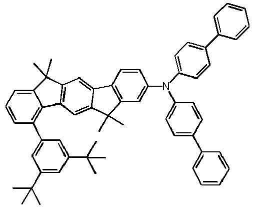 | 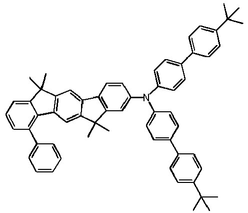 |
| 102 | 103 | 104 |
| 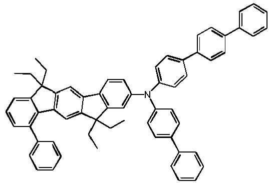 | 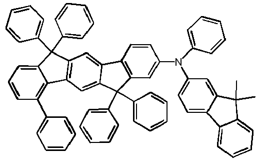 | 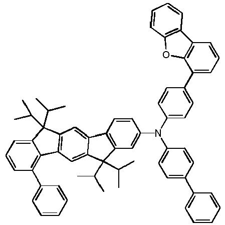 |
| 105 | 106 | 107 |
| 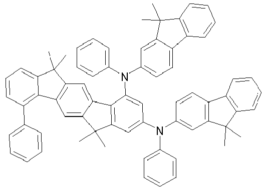 | 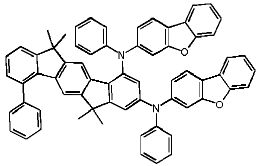 | 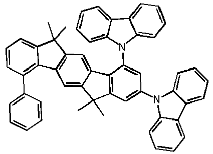 |
| 108 | 109 | 110 |
| 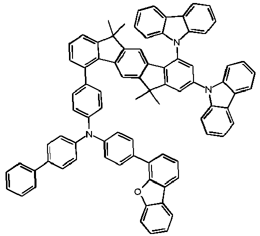 | 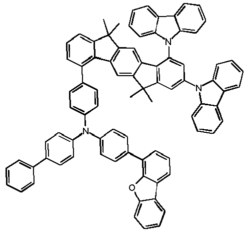 | 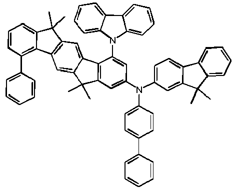 |
| 111 | 112 | 113 |
| 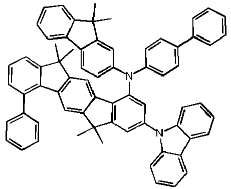 | 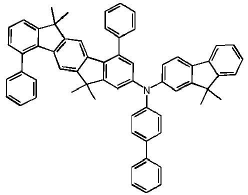 | 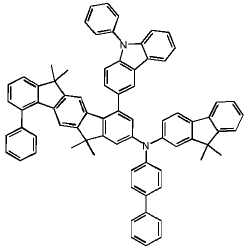 |
| 114 | 115 | 116 |
| 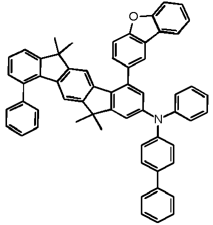 | 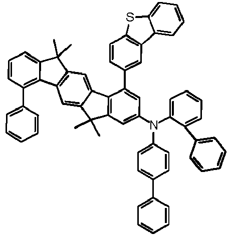 | 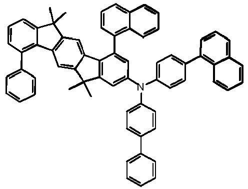 |
| 117 | 118 | 119 |
| 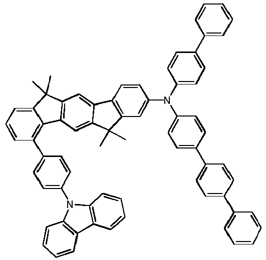 | 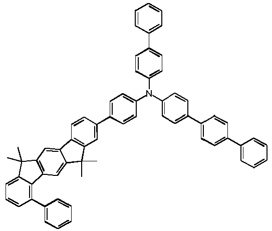 | 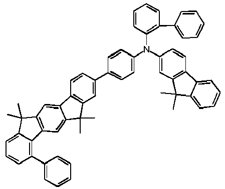 |
| 120 | 121 | 122 |
| 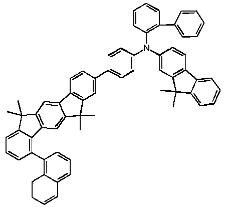 | 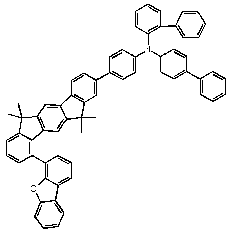 | 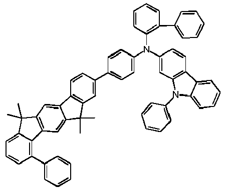 |
| 123 | 124 | 125 |
| 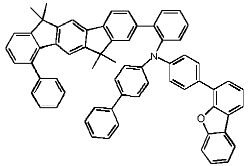 | 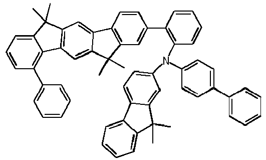 | 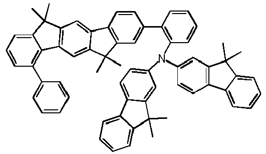 |
| 126 | 127 | 128 |
| 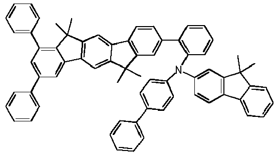 | 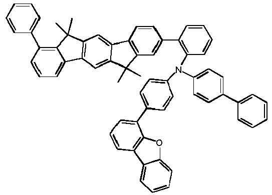 | 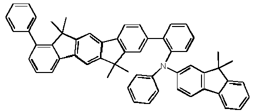 |
| 129 | 130 | 131 |
| 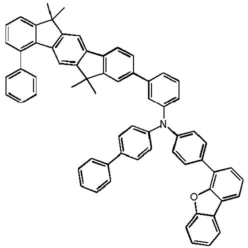 | 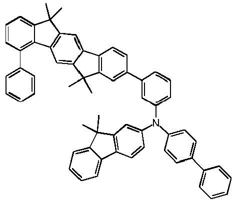 | 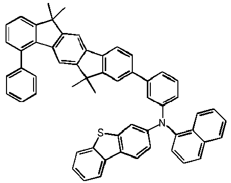 |
| 132 | 133 | 134 |
| 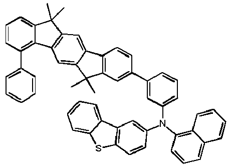 | 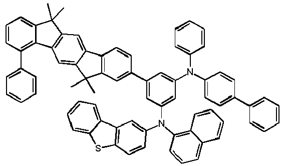 | 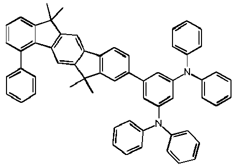 |
| 135 | 136 | 137 |
| 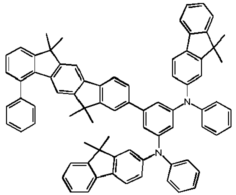 | 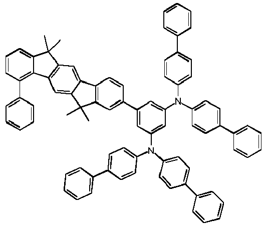 | 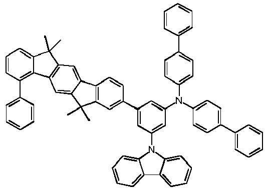 |
| 138 | 139 | 140 |
| 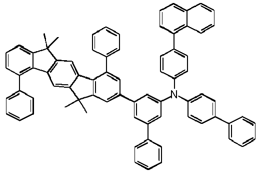 | 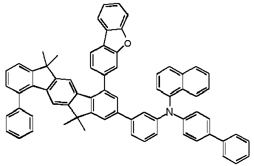 | 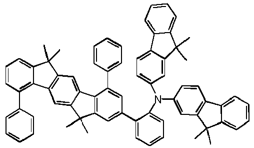 |
| 141 | 142 | 143 |
| 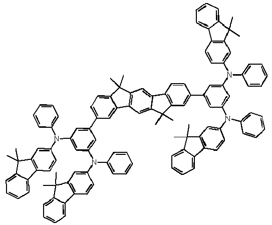 | 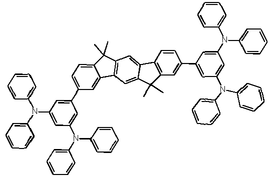 | 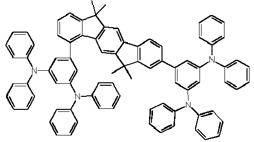 |
| 144 | 145 | 146 |
| 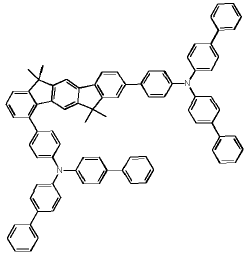 | 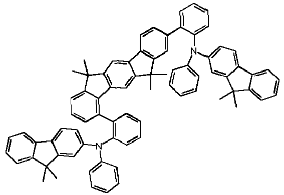 | 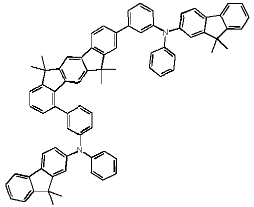 |
| 147 | 148 | 149 |
| 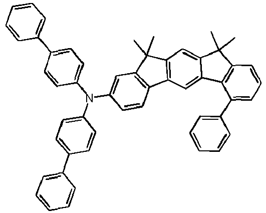 | 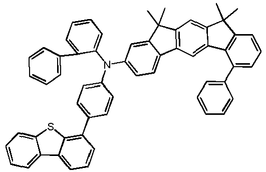 | 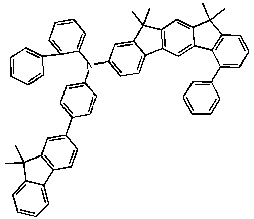 |
| 150 | 151 | 152 |
| 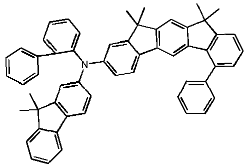 | 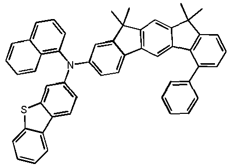 | 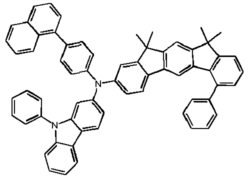 |
| 153 | 154 | 155 |
| 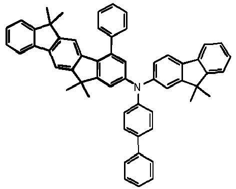 | 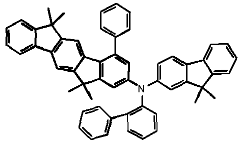 | 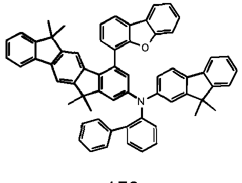 |
| 156 | 157 | 158 |
| 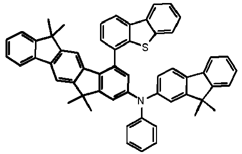 | 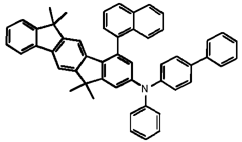 | 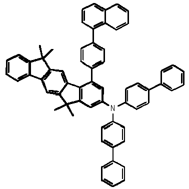 |
| 159 | 160 | 161 |
| 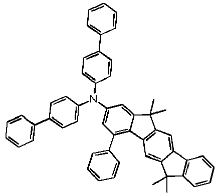 | 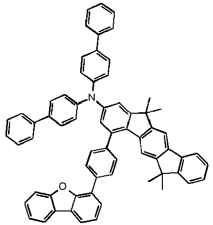 | 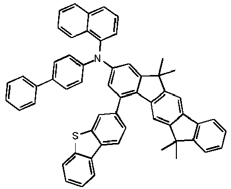 |
| 162 | 163 | 164 |
| 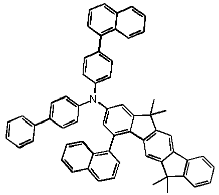 | 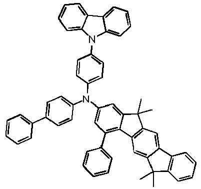 | 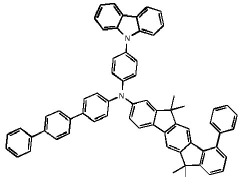 |
| 165 | 166 | 167 |
| 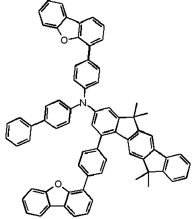 | 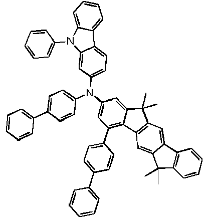 | 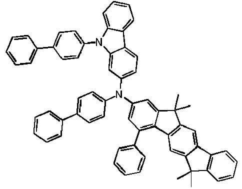 |
| 168 | 169 | 170 |
| 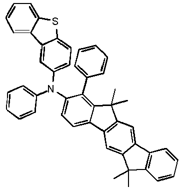 | 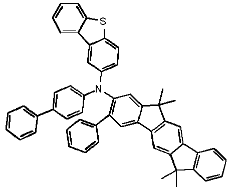 | 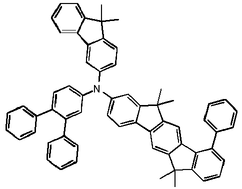 |
| 171 | 172 | 173 |

Die Verbindungen gemäß Formel (I) und (II) können mittels bekannten organischen Reaktionen hergestellt werden, insbesondere mittels Suzuki-Reaktionen, Hartwig-Buchwald-Reaktionen, und Zyklisierungsreaktionen. Gemäß einem bevorzugten Verfahren (Schema 1) wird ausgehend von einer Benzolverbindung, die zwei reaktive Gruppen X und zwei Carbonsäureestergruppen trägt, über zwei sequenzielle Suzuki-Kupplungen ein Intermediat hergestellt, in dem an die zentrale Benzolgruppe an einer Seite eine Benzolgruppe mit einer Aminogruppe A gebunden ist, und an der anderen Seite eine Benzolgruppe mit einem aromatischen Substituenten Ar gebunden ist. Der aromatische Substituent befindet sich in ortho- oder in meta-Stellung zur Bindung zwischen den beiden Benzolgruppen.

Anschließend werden die Carbonsäureestergruppen dieser Verbindung in tertiäre Alkoxygruppen überführt, durch Reaktion mit einer Metallalkylverbindung, bevorzugt einer Lithium-Alkylverbindung oder einer Grignard-Alkylverbindung. Diese tertiären Alkoxygruppen zyklisieren zu Ringen unter Einwirkung von Säure, so dass die Verbindung der Formel (I) erhalten wird.

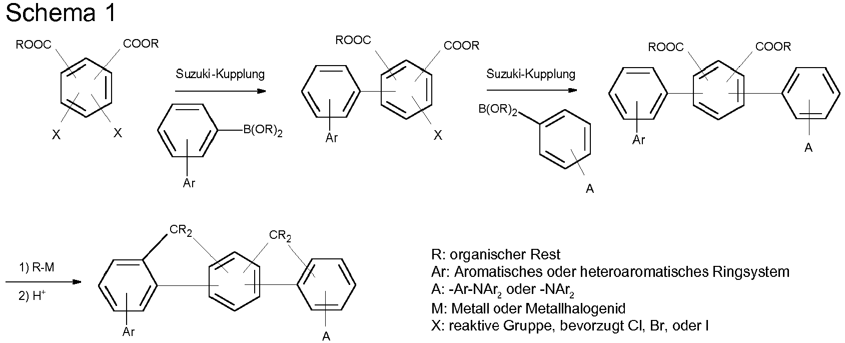

Gemäß einem alternativen bevorzugten Verfahren (Schema 2) wird ausgehend von einer Benzolverbindung, die zwei reaktive Gruppen X und zwei Carbonsäureestergruppen trägt, über zwei sequenzielle Suzuki-Kupplungen ein Intermediat hergestellt, in dem an die zentrale Benzolgruppe an einer Seite eine Benzolgruppe mit einer reaktiven Gruppe X gebunden ist, und an der anderen Seite eine Benzolgruppe mit einem aromatischen Substituenten Ar gebunden ist. Der aromatische Substituent befindet sich in ortho- oder in meta-Stellung zur Bindung zwischen den beiden Benzolgruppen.

Anschließend werden die Carbonsäureestergruppen dieser Verbindung in tertiäre Alkoxygruppen überführt, durch Reaktion mit einer Metallalkylverbindung, bevorzugt einer Lithium-Alkylverbindung oder einer Grignard-Alkylverbindung. Diese tertiären Alkoxygruppen zyklisieren zu Ringen unter Einwirkung von Säure. Schließlich wird über eine Buchwald-Kupplung eine Aminogruppe eingeführt, oder es wird durch Suzuki-Reaktion eine Diarylamino-Aryl- oder eine Diarylamino-Heteroaryl-Gruppe eingeführt, so dass die Verbindung der Formel (I) erhalten wird.

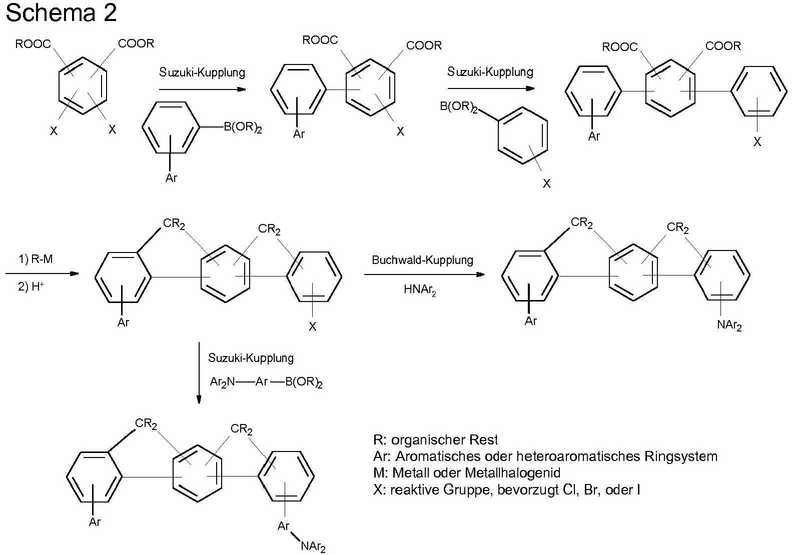

Gemäß einem alternativen bevorzugten Verfahren (Schema 3) wird ausgehend von einer Benzolverbindung, die zwei reaktive Gruppen X und zwei Carbonsäureestergruppen trägt, über zwei sequenzielle Suzuki-Kupplungen ein Intermediat hergestellt, in dem an die zentrale Benzolgruppe an einer Seite eine Benzolgruppe mit einer reaktiven Gruppe X gebunden ist, und an der anderen Seite eine Benzolgruppe mit einer Aminogruppe A gebunden ist. Die reaktive Gruppe X befindet sich in ortho- oder in meta-Stellung zur Bindung zwischen den beiden Benzolgruppen.

Anschließend werden die Carbonsäureestergruppen dieser Verbindung in tertiäre Alkoxygruppen überführt, durch Reaktion mit einer

Metallalkylverbindung, bevorzugt einer Lithium-Alkylverbindung oder einer Grignard-Alkylverbindung. Diese tertiären Alkoxygruppen zyklisieren zu Ringen unter Einwirkung von Säure. Schließlich wird über eine Suzuki-Reaktion ein aromatischer Substituent Ar eingeführt, so dass die Verbindung der Formel (I) erhalten wird.

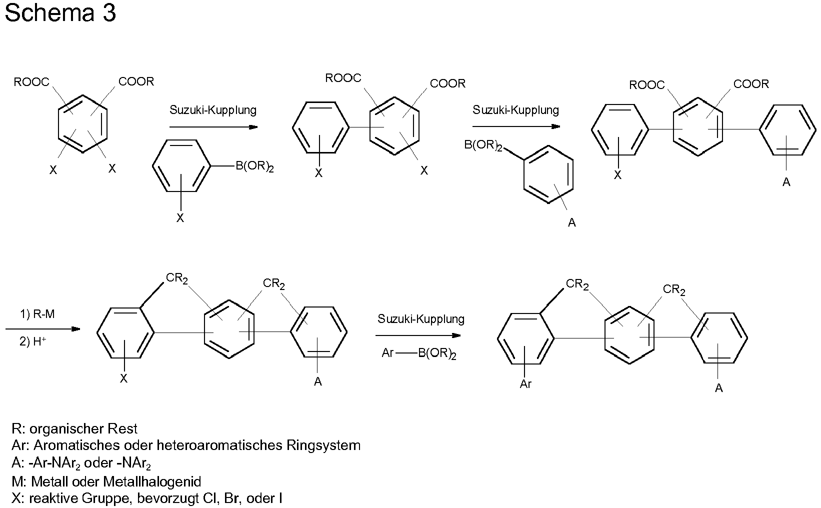

Ein bevorzugtes Verfahren zur Herstellung von Verbindungen gemäß Formel (II) ist im folgenden Schema 4 gezeigt. Dabei wird zunächst ausgehend von einer Benzolverbindung, die zwei reaktive Gruppen X und zwei Carbonsäureestergruppen trägt, über zwei sequenzielle Suzuki-Kupplungen ein Intermediat hergestellt, in dem an die zentrale Benzolgruppe an einer Seite eine Benzolgruppe gebunden ist, und an der anderen Seite eine Benzolgruppe mit zwei reaktiven Gruppen X gebunden ist. Mindestens eine der beiden reaktiven Gruppen X befindet sich in ortho- oder in meta-Stellung zur Bindung zwischen den beiden Benzolgruppen. Anschließend werden die Carbonsäureestergruppen dieser Verbindung in tertiäre Alkoxygruppen überführt, durch Reaktion mit einer Metallalkylverbindung, bevorzugt einer Lithium-Alkylverbindung oder einer Grignard-Alkylverbindung. Diese tertiären Alkoxygruppen zyklisieren zu Ringen unter Einwirkung von Säure. Dann wird über eine Buchwald-Kupplung eine Aminogruppe eingeführt, oder es wird durch Suzuki-Reaktion eine Diarylamino-Aryl- oder eine Diarylamino-Heteroaryl-Gruppe eingeführt. Schließlich wird über eine Suzuki-Reaktion ein aromatischer Substituent Ar eingeführt, so dass die Verbindung der Formel (II) erhalten wird.

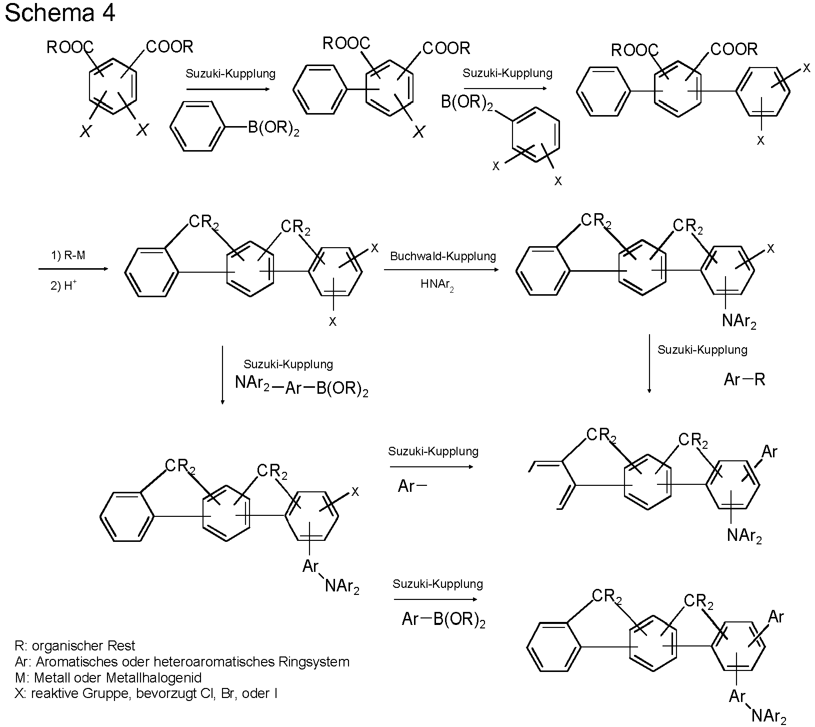

Weiterer Gegenstand der vorliegenden Anmeldung ist damit ein Verfahren zur Herstellung einer Verbindung gemäß Formel (I) oder (II), dadurch gekennzeichnet, dass eine Benzolverbindung, die zwei Carbonsäureestergruppen und mindestens eine reaktive Gruppe trägt, mit einer Benzolverbindung umgesetzt wird, die eine Boronsäuregruppe und mindestens eine Gruppe gewählt aus reaktiven Gruppen X und aromatischen oder heteroaromatischen Gruppen Ar enthält. Dabei steht die Boronsäuregruppe und die mindestens eine Gruppe gewählt aus Gruppen X und Ar in ortho- oder in meta-Stellung am Benzolring zueinander.

Bevorzugt ist die Gruppe X gewählt aus Cl, Br, I, Mesylat und Tosylat. Bevorzugt ist die Reaktion, in der die Benzolverbindung, die zwei Carbonsäureestergruppen und zwei reaktive Gruppen trägt, mit der Benzolverbindung, die eine Boronsäuregruppe und eine Gruppe gewählt aus reaktiven Gruppen X und aromatischen oder heteroaromatischen Gruppen Ar enthält, umgesetzt wird, eine Suzuki-Reaktion.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I) oder (II), wobei die Bindung(en) zum Polymer, Oligomer oder Dendrimer an beliebigen, in Formel (I) oder (II) mit R¹, R², R³, R⁴ oder R⁵ substituierten Positionen lokalisiert sein können. Je nach Verknüpfung der Verbindung ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) oder (II) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) oder (II) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) oder (II) in Oligomeren, Dendrimeren und Polymeren gelten dieselben Bevorzugungen wie oben für Verbindungen gemäß Formel (I) oder (II) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen, Spirobifluorenen, Paraphenylenen, Carbazolen, Thiophenen, Dihydrophenanthrenen, cis- und trans-Indenofluorenen, Ketonen, Phenanthrenen oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine oder phosphoreszierende Metallkomplexe, und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) oder (II) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder (II) sowie mindestens ein Lösungsmittel, bevorzugt ein organisches Lösungsmittel. Wie solche Lösungen hergestellt werden können, ist dem Fachmann bekannt.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) oder (II) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I) oder (II). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine lochtransportierende Schicht oder eine andere Schicht sein kann, mindestens eine Verbindung gemäß Formel (I) oder (II) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und/oder organischen oder anorganischen p/n-Übergängen.

Die Abfolge der Schichten der organischen Elektrolumineszenzvorrichtung enthaltend die Verbindung der Formel (I) oder (II) ist bevorzugt die folgende:
Anode-Lochinjektionsschicht-Lochtransportschicht-wahlweise weitere Lochtransportschicht(en)-wahlweise Elektronenblockierschichtemittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen. Die erfindungsgemäßen Verbindungen sind dabei bevorzugt in einer Lochtransportschicht, Lochinjektionsschicht, Elektronenblockierschicht, und/oder emittierenden Schicht vorhanden, besonders bevorzugt in einer emittierenden Schicht als Matrixmaterial, und/oder in einer Elektronenblockierschicht.

Es ist erfindungsgemäß bevorzugt, wenn die Verbindung gemäß Formel (I) oder (II) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht, und/oder einer emittierenden Schicht enthalten sein. Besonders bevorzugt ist sie in einer Elektronenblockierschicht oder in einer emittierenden Schicht in Kombination mit einer phosphoreszierenden emittierenden Verbindung enthalten. In letzterem Fall ist die phosphoreszierende emittierende Verbindung bevorzugt gewählt aus rot oder grün phosphoreszierenden emittierenden Verbindungen. Ganz besonders bevorzugt ist sie in einer Elektronenblockierschicht enthalten. Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) oder (II) in organischen Elektrolumineszenzvorrichtungen einsetzen. Weitere Beispiele sind in der folgenden Tabelle aufgeführt:

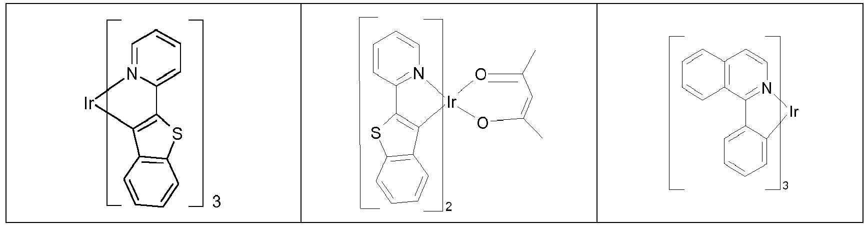

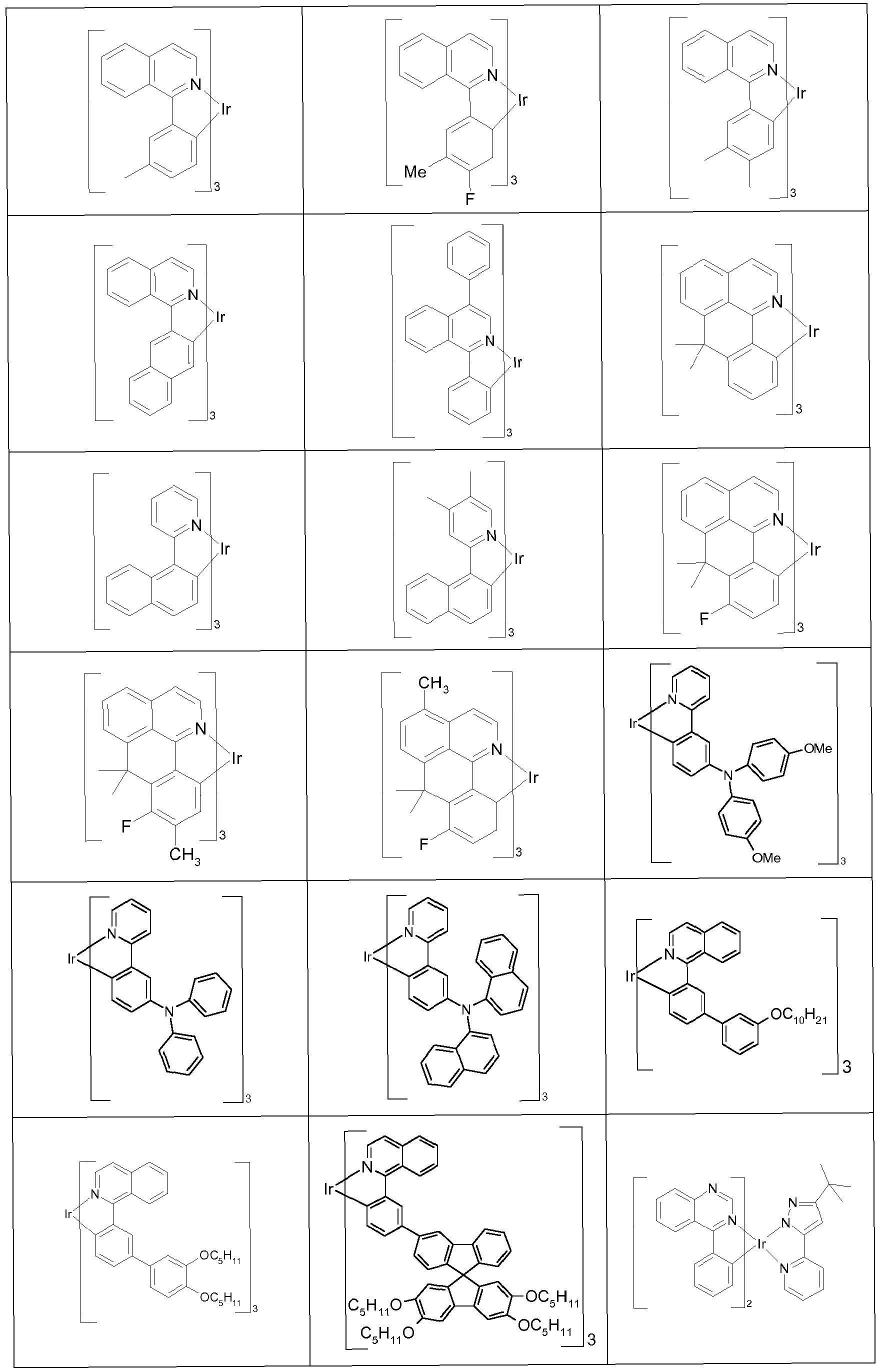

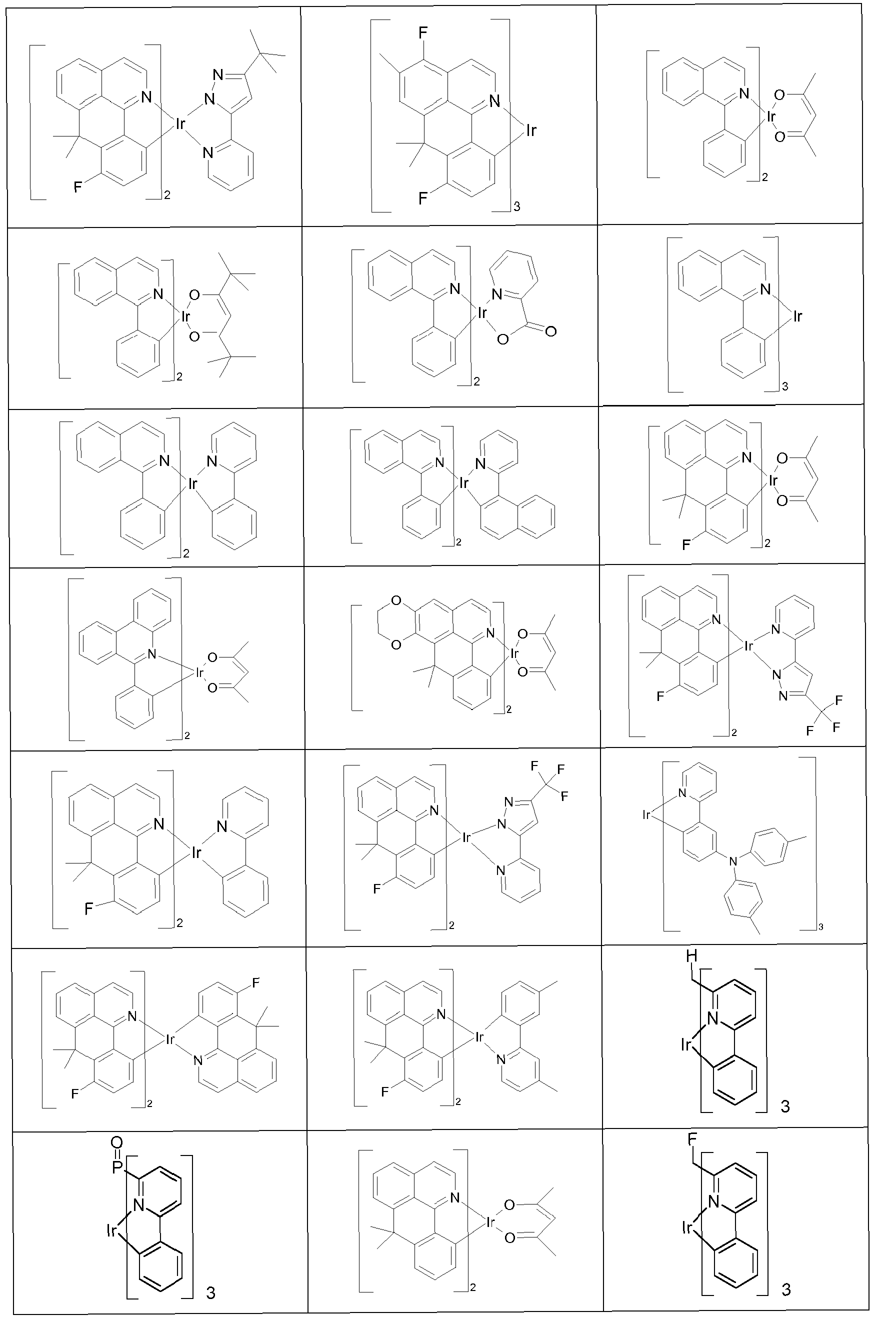

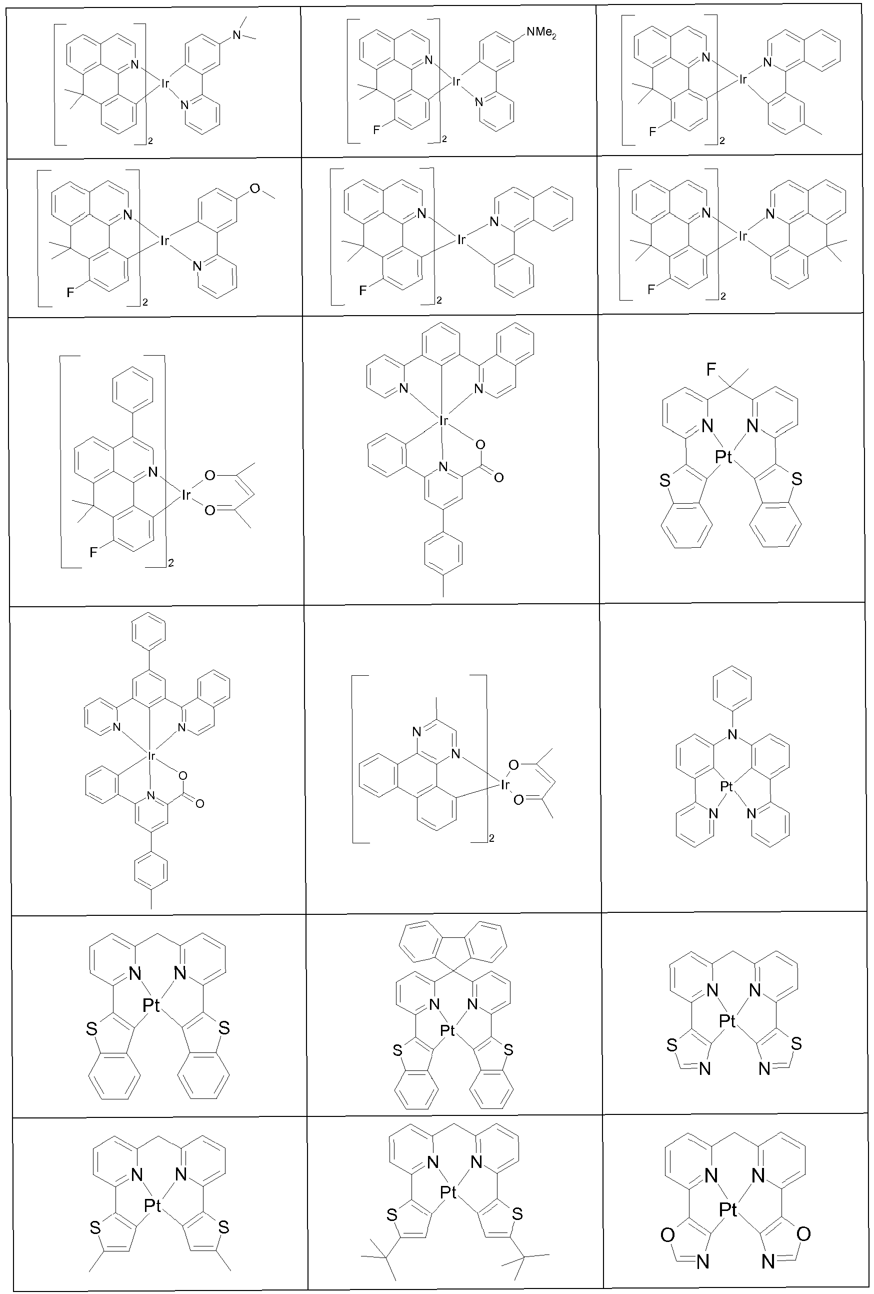

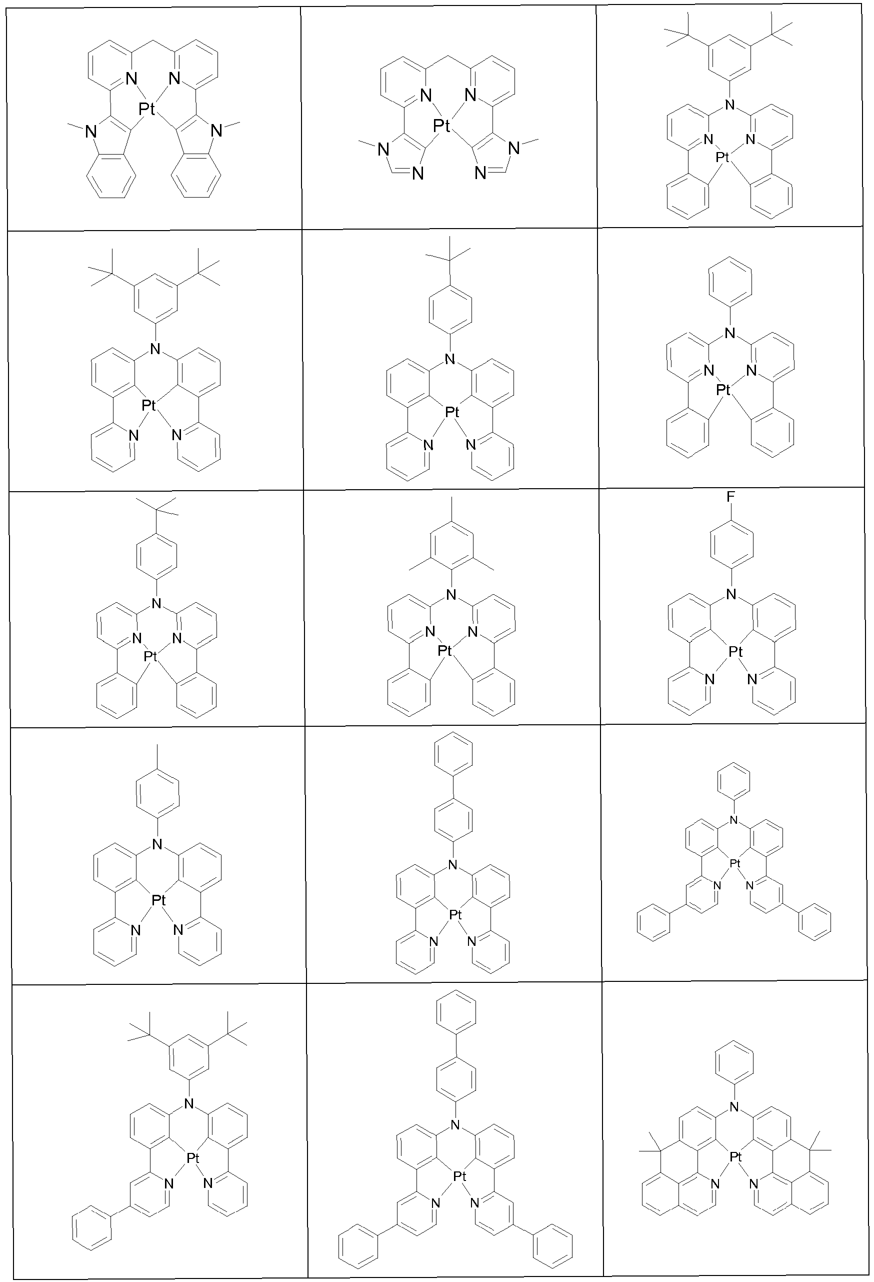

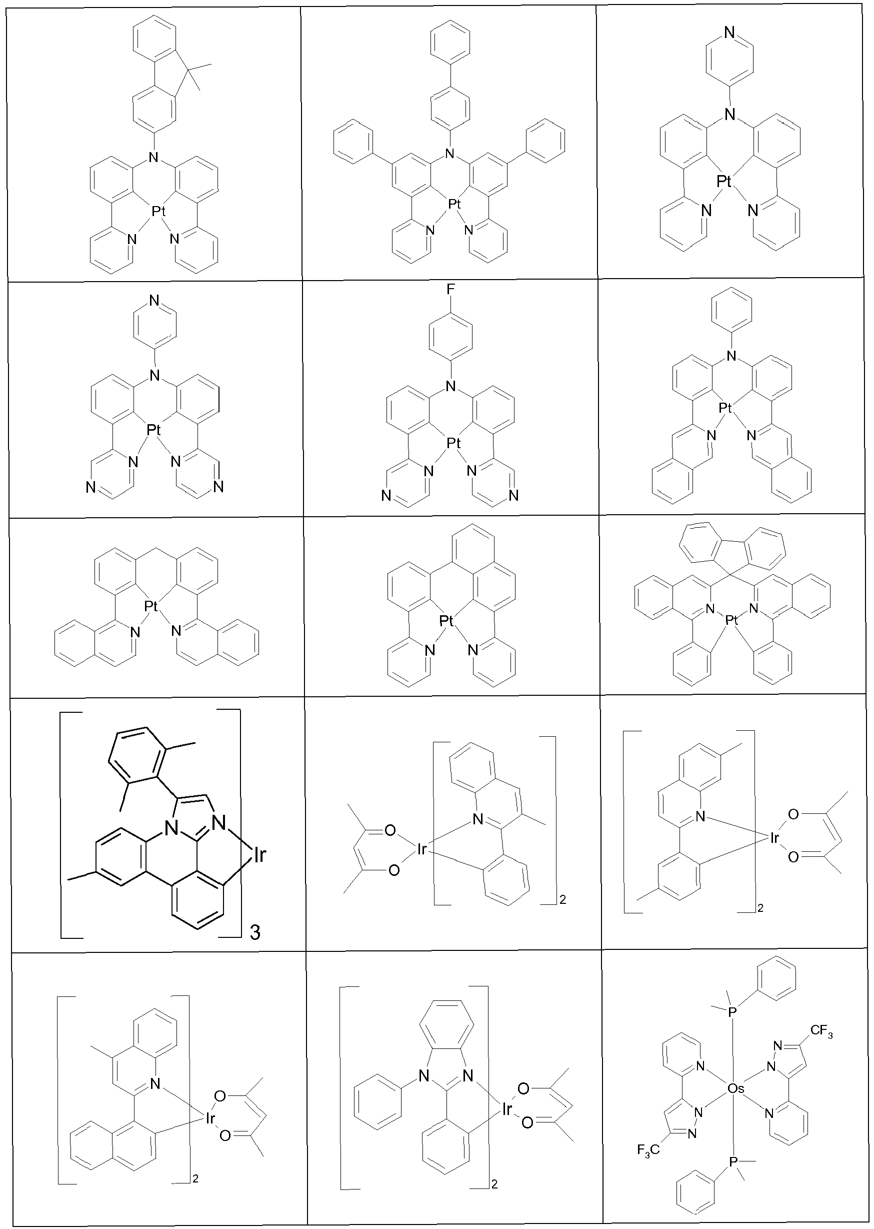

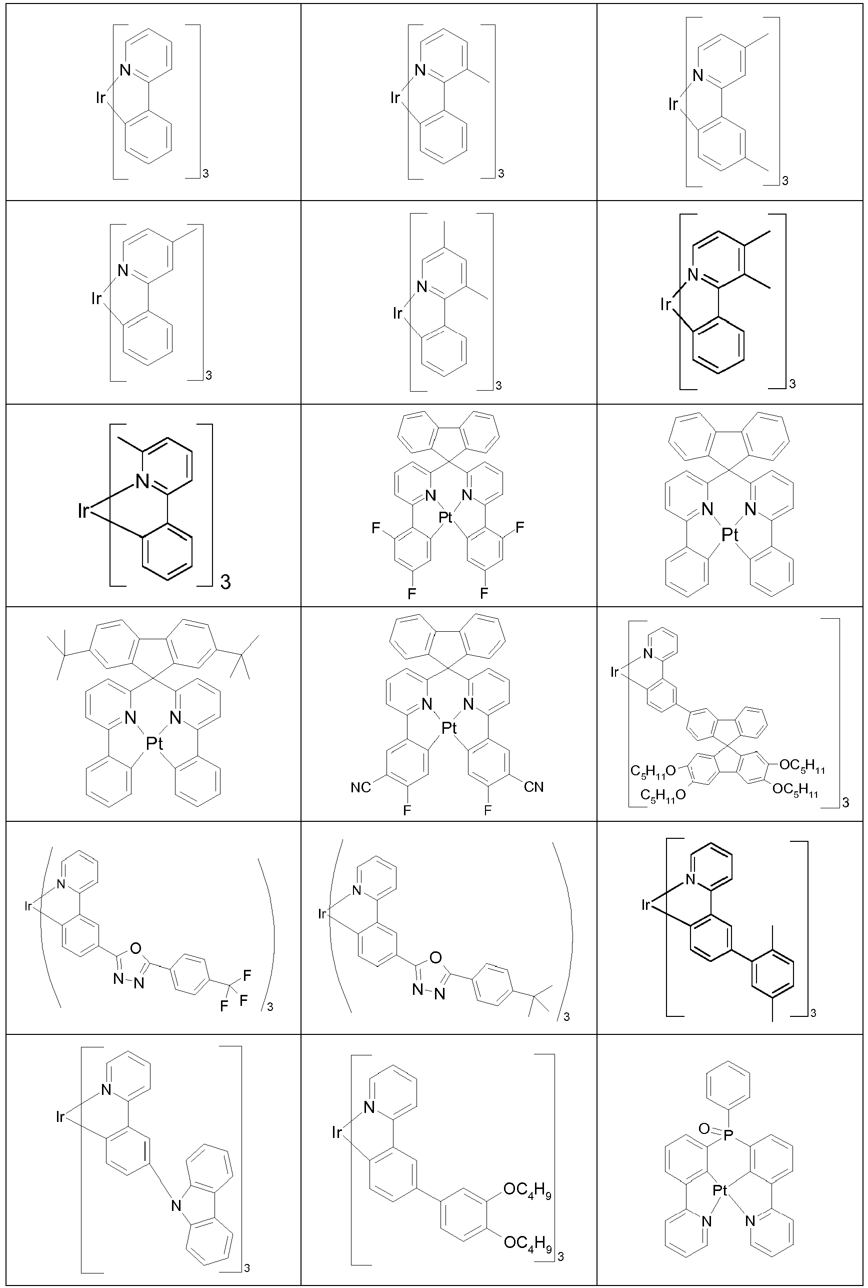

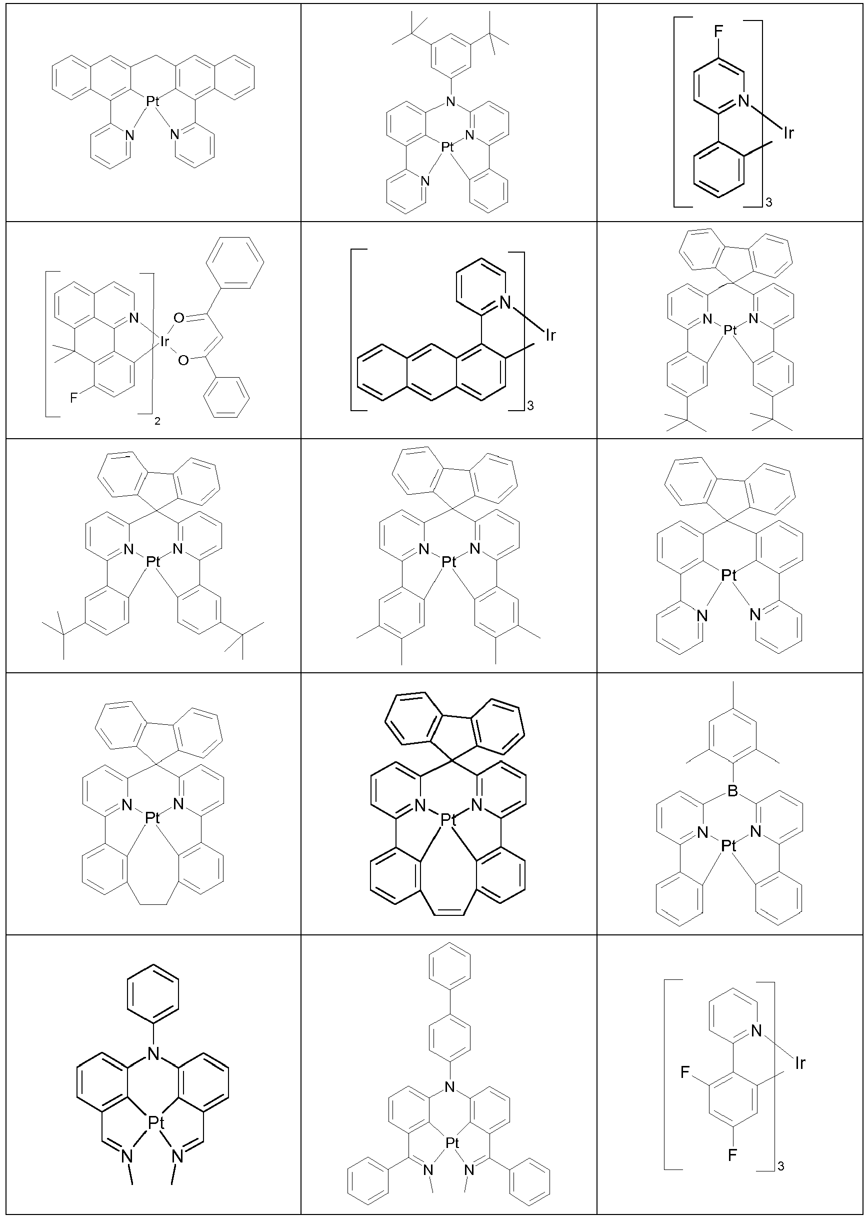

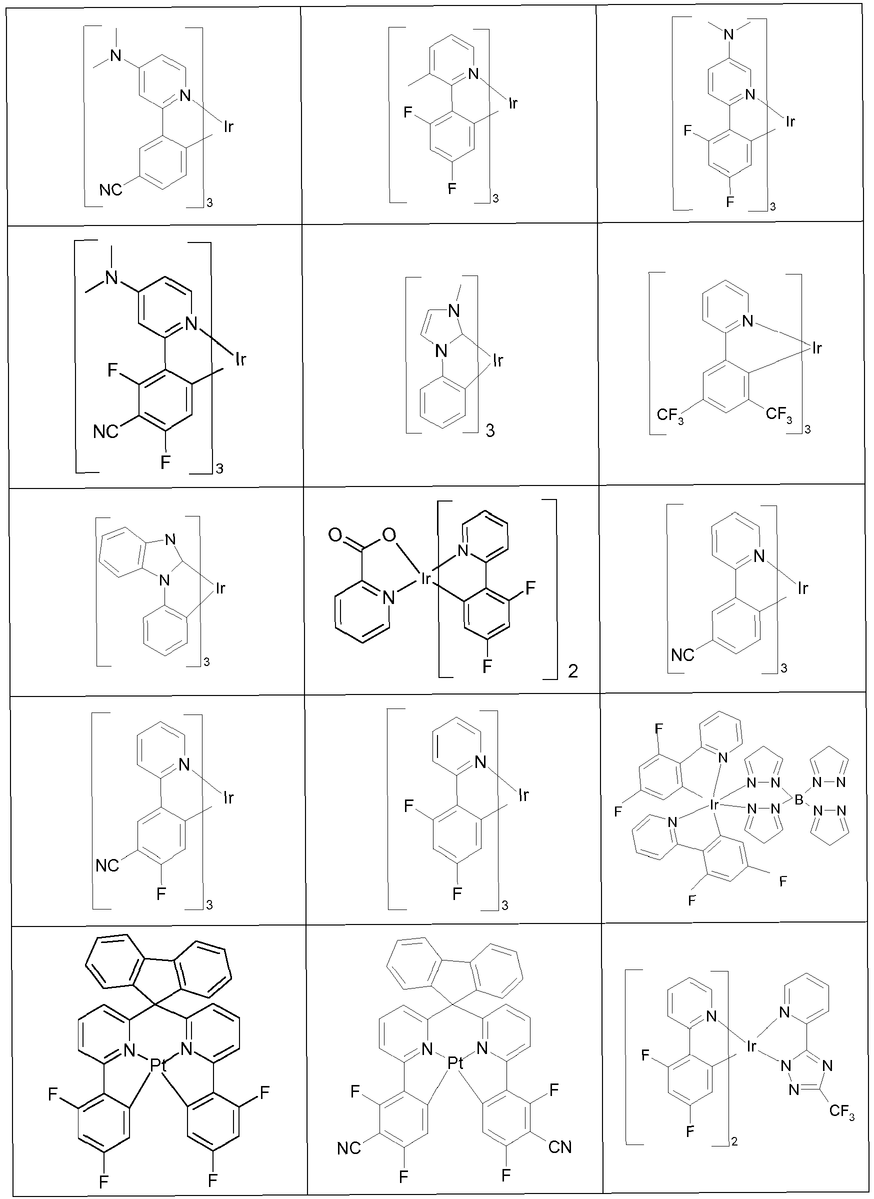

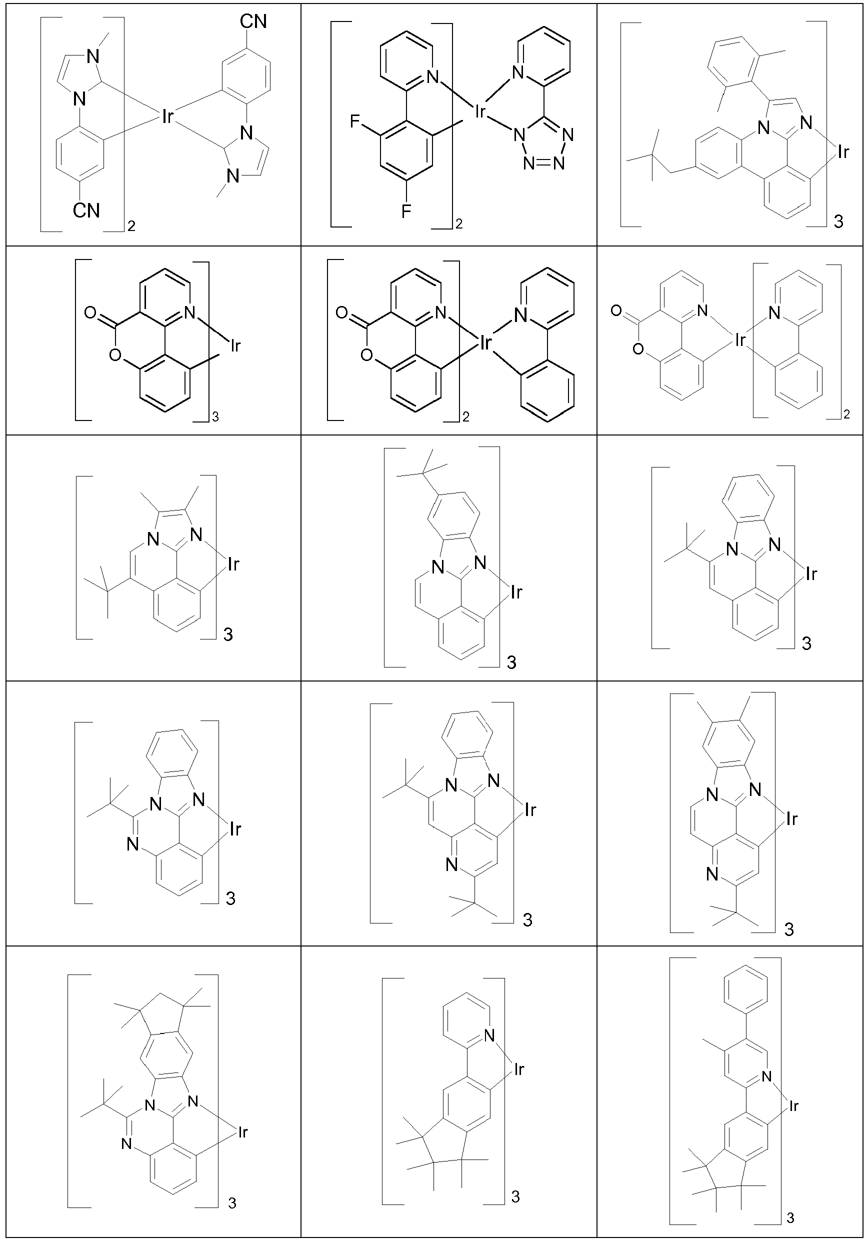

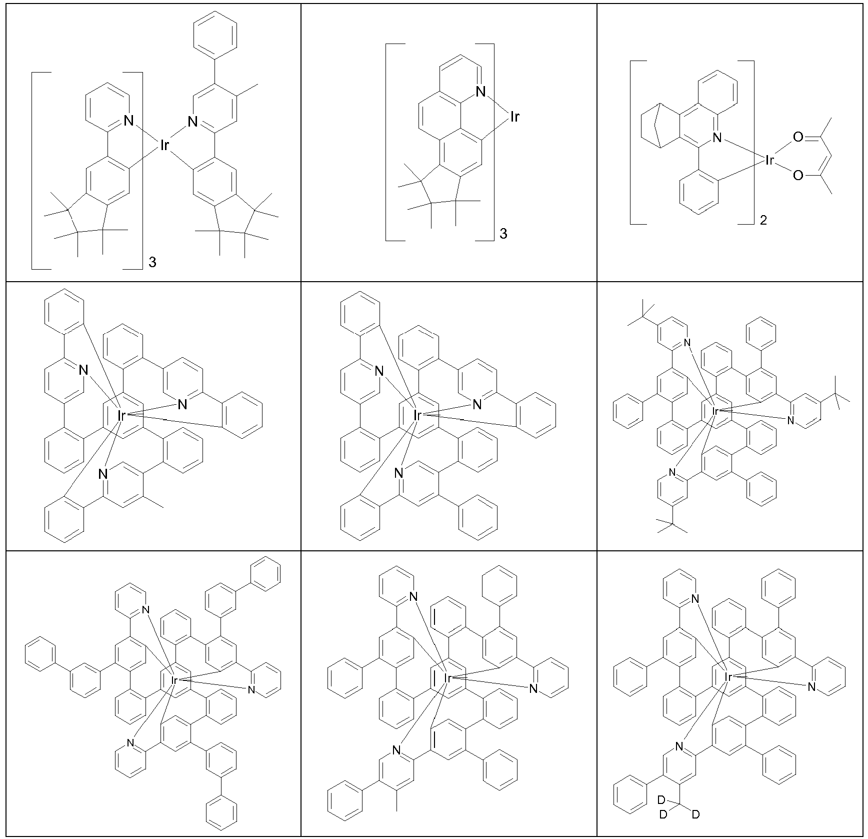

In einer bevorzugten Ausführungsform der Erfindung werden die Verbindungen gemäß Formel (I) oder (II) als lochtransportierendes Material eingesetzt. Die Verbindungen liegen dann bevorzugt in einer lochtransportierenden Schicht vor. Bevorzugte Ausführungsformen von lochtransportierenden Schichten sind Lochtransportschichten, Elektronenblockierschichten und Lochinjektionsschichten. Wenn die Verbindung der Formel (I) oder (II) in einer lochtransportierenden Schicht vorliegt, ist diese bevorzugt eine elektronenblockierende Schicht. Diese grenzt bevorzugt anodenseitig direkt an die emittierende Schicht an.

Eine Lochtransportschicht gemäß der vorliegenden Anmeldung ist eine Schicht mit lochtransportierender Funktion, welche sich zwischen Anode und emittierender Schicht befindet. Insbesondere ist es eine lochtransportierende Schicht, die keine Lochinjektionsschicht und keine Elektronenblockierschicht ist.

Lochinjektionsschichten und Elektronenblockierschichten werden im Sinne der vorliegenden Anmeldung als spezielle Ausführungsformen von lochtransportierenden Schichten verstanden. Eine Lochinjektionsschicht ist dabei im Fall von mehreren lochtransportierenden Schichten zwischen Anode und emittierender Schicht eine lochtransportierende Schicht, welche sich direkt an die Anode anschließt oder nur durch eine einzelne Beschichtung der Anode von ihr getrennt ist. Eine Elektronenblockierschicht ist im Fall von mehreren lochtransportierenden Schichten zwischen Anode und emittierender Schicht diejenige lochtransportierende Schicht, welche sich direkt anodenseitig an die emittierende Schicht anschließt. Bevorzugt enthält die erfindungsgemäße OLED zwei, drei oder vier lochtransportierende Schichten zwischen Anode und emittierender Schicht, von denen bevorzugt mindestens eine eine Verbindung gemäß Formel (I) oder (II) enthält, besonders bevorzugt genau eine oder zwei eine Verbindung gemäß Formel (I) oder (II) enthalten.

Wird die Verbindung gemäß Formel (I) oder (II) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) oder (II) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

| | | |
|---|---|---|
| 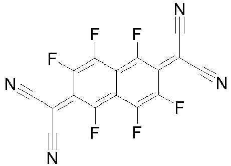 | 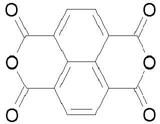 | 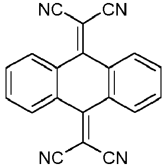 |
| (D-1) | (D-2) | (D-3) |
| 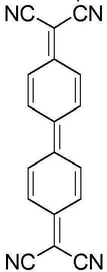 | 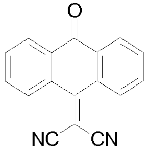 | 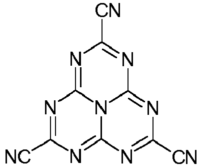 |
| (D-4) | (D-5) | (D-6) |
| 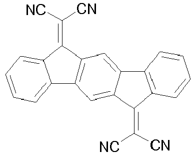 | 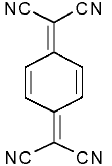 | 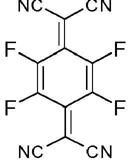 |
| (D-7) | (D-8) | (D-9) |
| 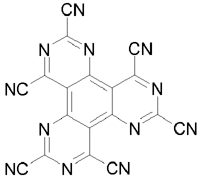 | 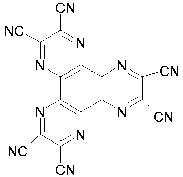 | 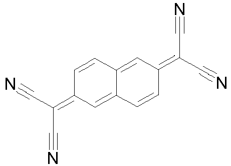 |
| (D-10) | (D-11) | (D-12) |
| 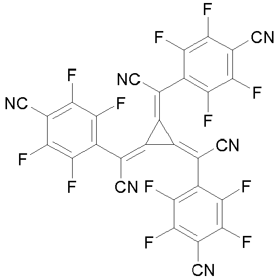 | | |
| (D-13) | | |

In einer weiteren bevorzugten Ausführungsform der Erfindung wird die Verbindung gemäß Formel (I) oder (II) als Lochtransportmaterial in Kombination mit einem Hexaazatriphenylenderivat in einer OLED verwendet. Besonders bevorzugt wird das Hexaazatriphenylenderivat dabei in einer separaten Schicht eingesetzt.

In einer bevorzugten Ausführungsform der vorliegenden Erfindung wird die Verbindung der Formel (I) oder (II) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierenden emittierenden Verbindungen sind dabei bevorzugt gewählt aus rot phosphoreszierenden und grün phosphoreszierenden Verbindungen.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) oder (II) als eine Komponente von Mixed-Matrix-Systemen, bevorzugt für phosphoreszierende Emitter, verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) oder (II) stellt dabei bevorzugt das Matrixmaterial mit lochtransportierenden Eigenschaften dar. Entsprechend ist, wenn die Verbindung der Formel (I) oder (II) als Matrixmaterial für einen phosphoreszierenden Emitter in der emittierenden Schicht einer OLED eingesetzt wird, eine zweite Matrixverbindung in der emittierenden Schicht vorhanden, die elektronentransportierende Eigenschaften aufweist. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen, darunter insbesondere aus denjenigen, die elektronentransportierende Eigenschaften aufweisen.

Im Folgenden werden bevorzugte Ausführungsformen für die verschiedenen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, Benzoindenofluorenamine bzw. -diamine, und Dibenzoindenofluorenamine bzw. -diamine, sowie Indenofluorenderivate mit kondensierten Arylgruppen. Ebenfalls bevorzugt sind Pyren-Arylamine, Benzoindenofluoren-Amine, Benzofluoren-Amine, erweiterte Benzoindenofluorene, Phenoxazine, und Fluoren-Derivate, die mit Furan-Einheiten oder mit Thiophen-Einheiten verbunden sind.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren oder Dinaphthylanthracen), insbesondere der Oligoarylene enthaltend kondensierte aromatische Gruppen, der Oligoarylenvinylene (z. B. DPVBi oder Spiro-DPVBi), der polypodalen Metallkomplexe, der lochleitenden Verbindungen, der elektronenleitenden Verbindungen, insbesondere Ketone, Phosphinoxide und Sulfoxide, der Atropisomere, der Boronsäurederivate oder der Benzanthracene. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende emittierende Verbindungen sind neben den Verbindungen der Formel (I) oder (II) aromatische Ketone, aromatische Phosphinoxide oder aromatische Sulfoxide oder Sulfone, Triarylamine, Carbazolderivate, Indolocarbazolderivate, Indenocarbazolderivate, Azacarbazolderivate, bipolare Matrixmaterialien, Silane, Azaborole oder Boronester, Triazinderivate, Zinckomplexe, Diazasilol- bzw. Tetraazasilol-Derivate, Diazaphosphol-Derivate, überbrückte Carbazol-Derivate, Triphenylenderivate und Lactame.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) oder (II) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugte Materialien für lochtransportierende Schichten der OLEDs sind die folgenden Materialien:

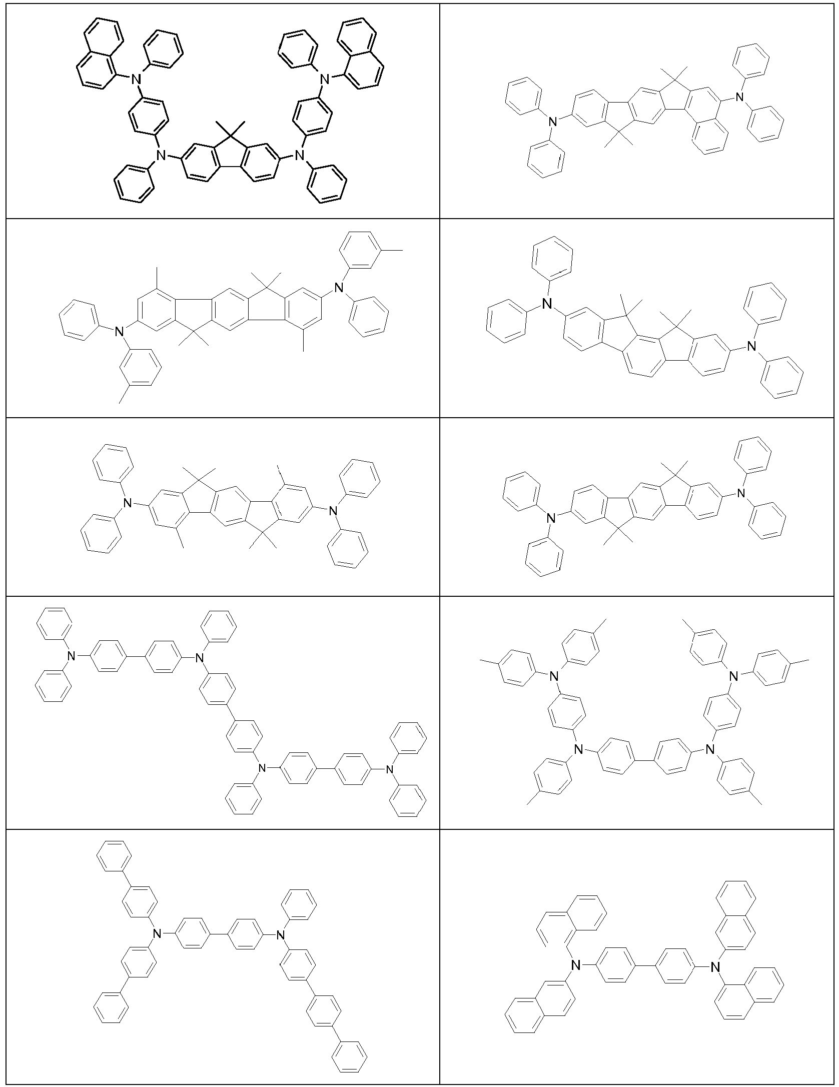

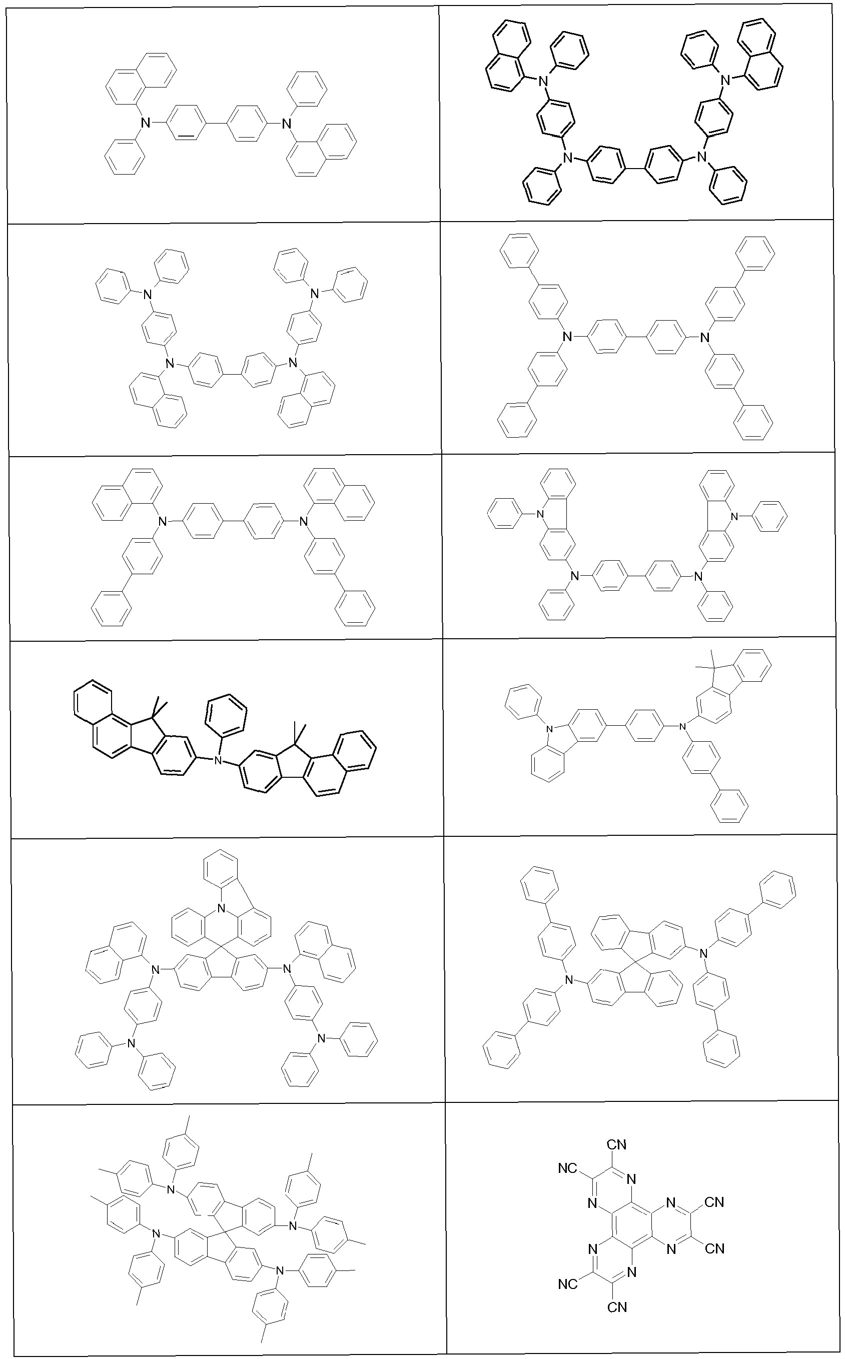

Bevorzugt umfasst die erfindungsgemäße OLED zwei oder mehr unterschiedliche lochtransportierende Schichten. Die Verbindung der Formel (I) oder (II) kann dabei in einer oder in mehreren oder in allen lochtransportierenden Schichten eingesetzt werden. Gemäß einer bevorzugten Ausführungsform wird die Verbindung der Formel (I) oder (II) in genau einer oder genau zwei lochtransportierenden Schichten eingesetzt, und in den weiteren vorhandenen lochtransportierenden Schichten werden andere Verbindungen eingesetzt, bevorzugt aromatische Aminverbindungen. Weitere Verbindungen, die neben den Verbindungen der Formel (I) oder (II) bevorzugt in lochtransportierenden Schichten der erfindungsgemäßen OLEDs eingesetzt werden, sind insbesondere Indenofluorenamin-Derivate, Aminderivate, Hexaazatriphenylenderivate, Aminderivate mit kondensierten Aromaten, Monobenzoindenofluorenamine, Dibenzoindenofluorenamine, Spirobifluoren-Amine, Fluoren-Amine, Spiro-Dibenzopyran-Amine, Dihydroacridin-Derivate, Spirodibenzofurane und Spirodibenzothiophene, Phenanthren-Diarylamine, Spiro-Tribenzotropolone, Spirobifluorene mit meta-Phenyldiamingruppen, Spiro-Bisacridine, Xanthen-Diarylamine, und 9,10-Dihydroanthracen-Spiroverbindungen mit Diarylaminogruppen.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Besonders bevorzugt sind die in der folgenden Tabelle gezeigten Verbindungen:

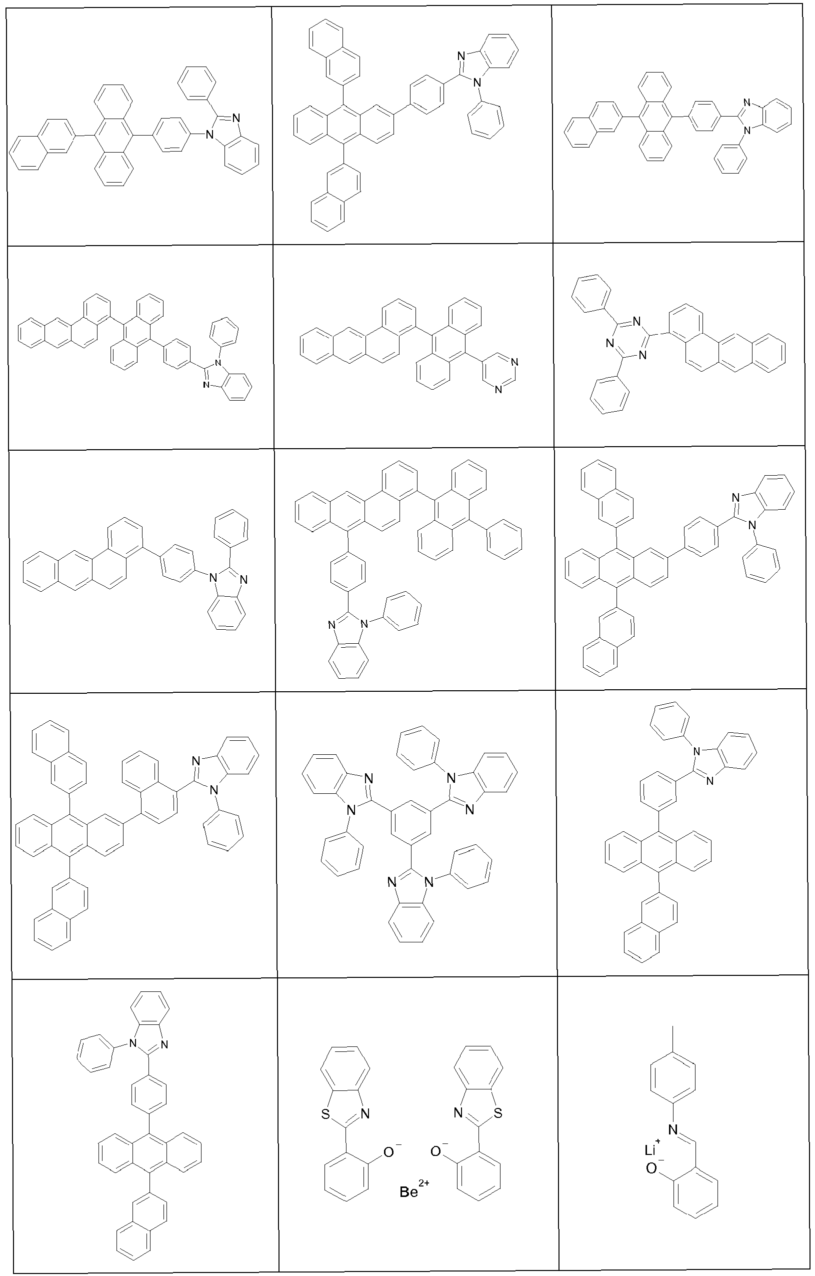

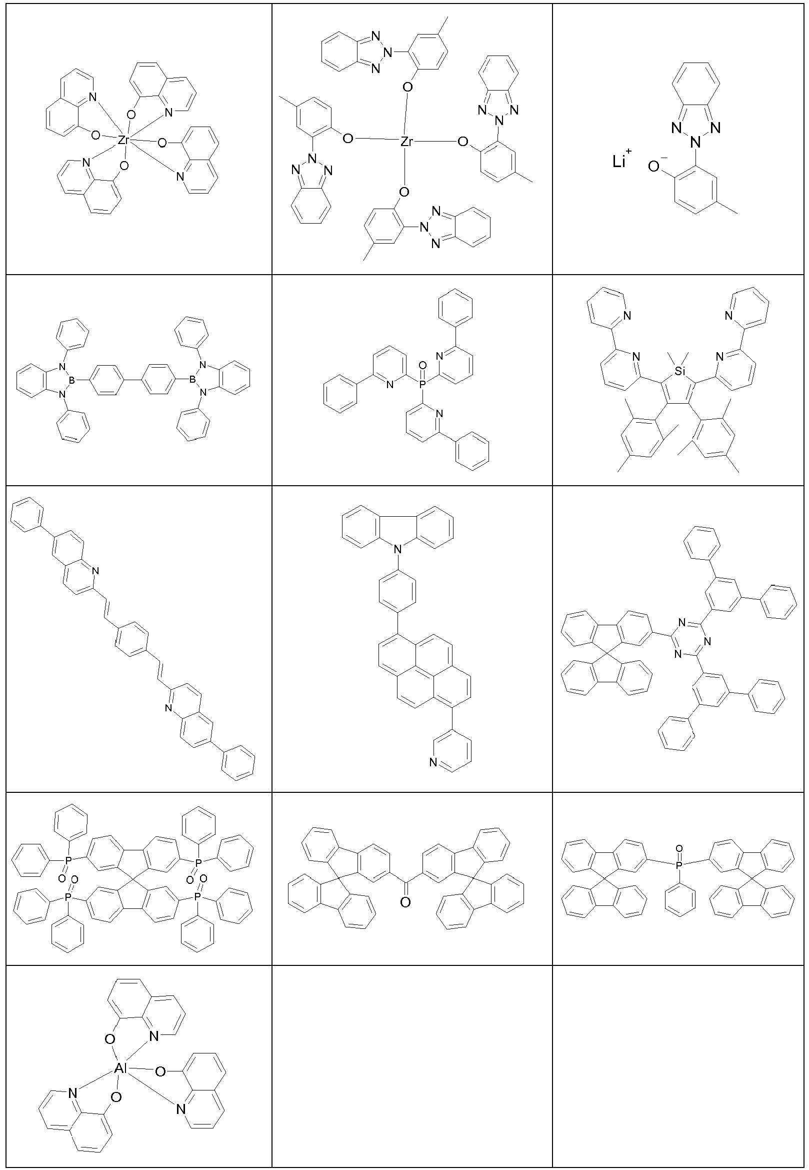

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) oder (II) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) oder (II) in Displays, als Lichtquellen in Beleuchtungsanwendungen sowie als Lichtquellen in medizinischen und/oder kosmetischen Anwendungen eingesetzt werden.

### Beispiele

### A) Synthesebeispiele

### Beispiel 1-1:

### Synthese der erfindungsgemäßen Verbindung 1-1 und Varianten

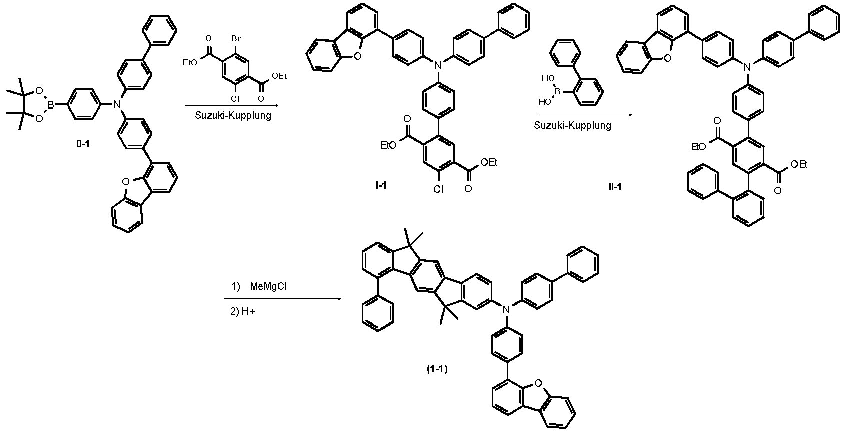

### Zwischenstufe I-1

22 g des Boronesterderivates 0-1 (34,8 mmol) und 11.7 g Brom-Chlordicarbonsäureester-Derivate (34,8 mmol) werden in 200 mL Toluol, 100mL Ethanol und 50 ml Wasser suspendiert. 7,4g Natriumcarbonat werden zugegeben. Die Reaktionslösung wird entgast und mit N₂ gesättigt. Danach wird sie mit 0,6 g (0,51 mmol) Pd(Ph₃P)₄ versetzt. Die Reaktionsmischung wird 16 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird in Heptan ausgerührt. Die Ausbeute beträgt 23 g (87% d. Th).

### Zwischenstufe II-1

6,3 g 2-Phenyl-phenylboronsäure (31,6 mmol) und 23 g des Chlor-Derivats I-1 (30 mmol) werden in 260 mL Toluol und 100 mL Wasser suspendiert. 8,3 g Kaliumcarbonat werden dazugegeben. Die Lösung wird entgast und mit N₂ gesättigt. Danach wird sie mit 276 mg (0,3 mmol) Pd₂(dba)₃ und 250 mg SPhos (0,3 mmol) versetzt. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus EtOH umkristallisiert. Die Ausbeute beträgt 20,7 g (80% d. Th).

Analog dazu werden folgende Verbindungen hergestellt:

| | Edukt 1 | Boronsäure Derivat 1 | Boronsäure Derivat 2 | Produkt |
|---|---|---|---|---|
| **II-2** | 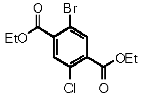 | 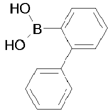 | 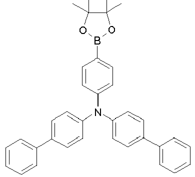 | 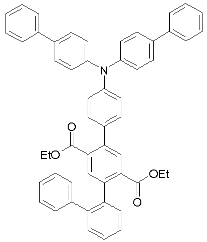 |
| **II-3** | 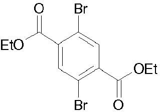 | 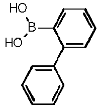 | 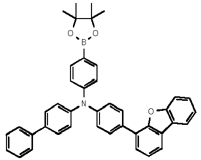 | 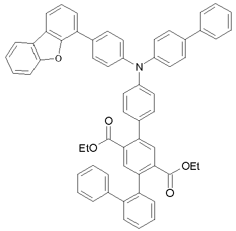 |
| **II-4** | 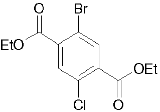 | 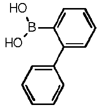 | 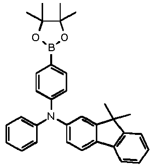 | 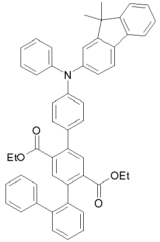 |
| **II-5** | 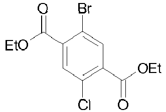 | 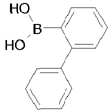 | 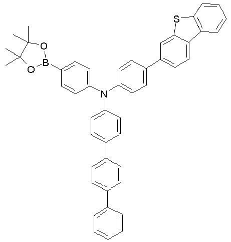 | 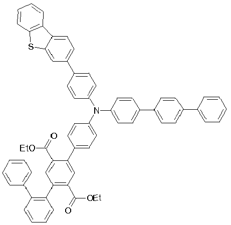 |
| **II-6** | 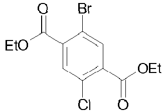 | 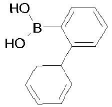 | 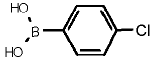 | 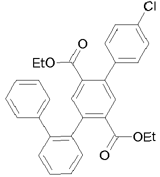 |
| **II-7** | 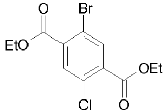 | 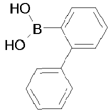 | 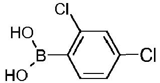 | 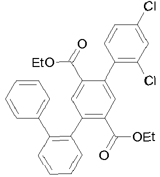 |
| **II-8** | 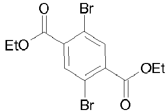 | 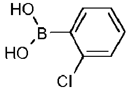 | 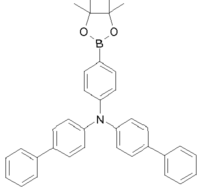 | 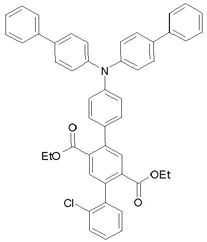 |
| **II-9** | 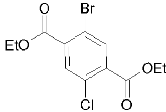 | 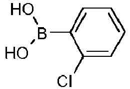 | 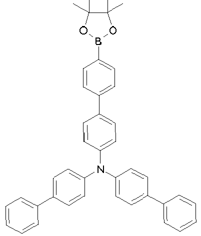 | 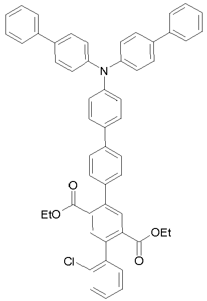 |
| **II-10** | 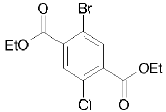 | 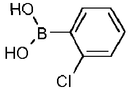 | 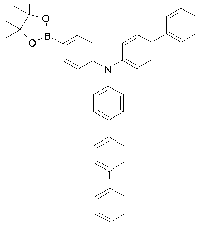 | 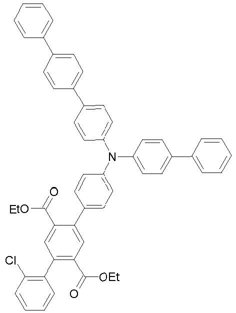 |
| **II-11** | 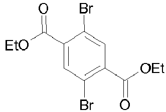 | 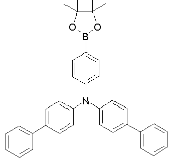 | 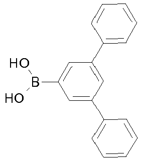 | 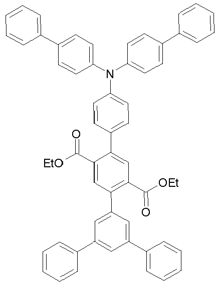 |
| **II-12** | 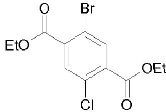 | 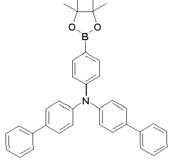 | 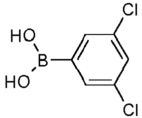 | 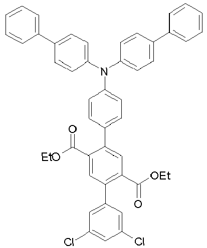 |
| **II-13** | 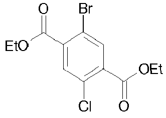 | 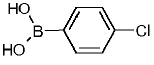 | 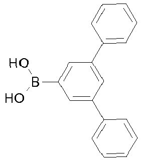 | 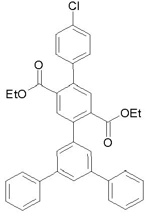 |
| **II-14** | 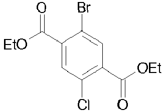 | 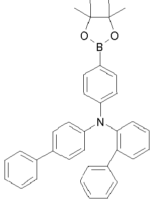 | 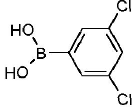 | 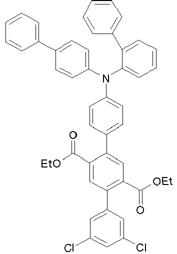 |
| **II-15** | 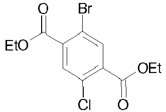 | 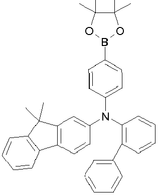 | 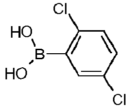 | 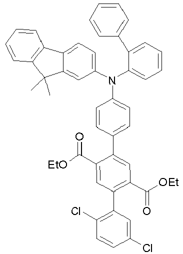 |
| **II-16** | 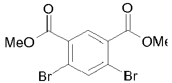 | 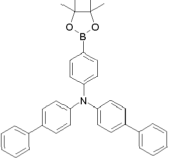 | 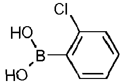 | 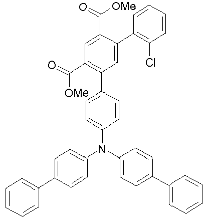 |
| **II-17** | 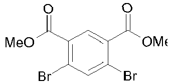 | 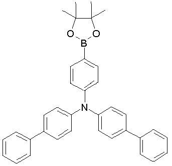 | 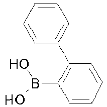 | 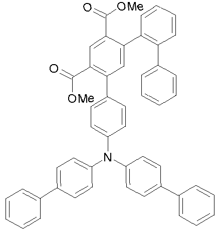 |
| **II-18** | 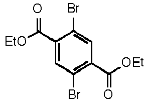 | 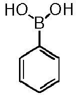 | 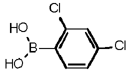 | 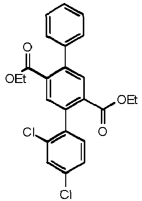 |
| **II-19** | 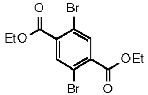 | 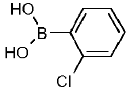 | 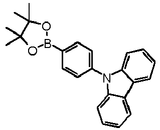 | 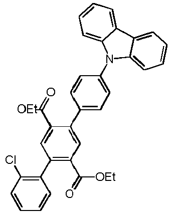 |

### Verbindung 1-1

15,0 g (17,4 mmol) Zwischenstufe II-1 werden in einem ausgeheizten Kolben in 150 mL getrocknetem THF gelöst. Die Lösung wird mit N₂ gesättigt. Die klare Lösung wird auf -5°C abgekühlt und dann werden 35 mL (105 mmol) einer 3M-Methylmagnesiumchlorid-Lösung zugegeben. Die Reaktionsmischung wird langsam auf Raumtemperatur erwärmt und dann mit Ammoniumchlorid gequencht. Das Gemisch wird im Anschluss zwischen Essigester und Wasser verteilt, die organische Phase dreimal mit Wasser gewaschen, über Na₂SO₄ getrocknet und einrotiert. Die einrotierte Lösung wird in Toluol gelöst und mit 8 g Amberlyst 15 versetzt. Der Ansatz wird auf 110°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Heptan nachgewaschen. Der Rückstand wird im Vakuum bei 40°C getrocknet. Nach Filtration des Rohproduktes über Kieselgel mit Heptan:Essigsester, 1:1 erhält man 13 g (90% der Theorie) des Produkts. Abschließend wird das Material im Hochvakuum sublimiert. Reinheit beträgt 99.9%.

Analog dazu werden folgende Verbindungen hergestellt:

| | Edukt 1 | Edukt 2 | Produkt |
|---|---|---|---|
| **1-2** | 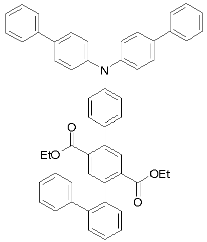 | MeMgBr | 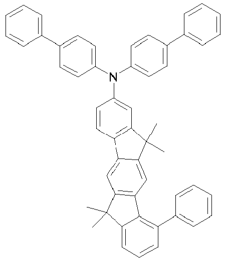 |
| **1-3** | 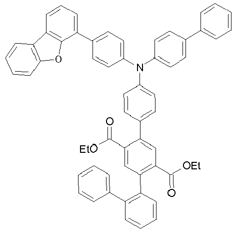 | iPrMgBr | 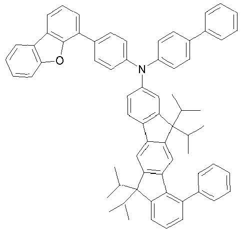 |
| **1-4** | 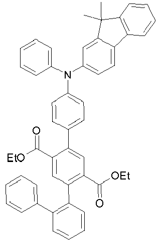 | MeMgBr | 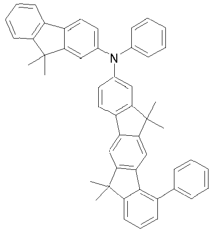 |
| **1-5** | 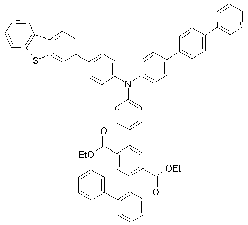 | MeMgBr | 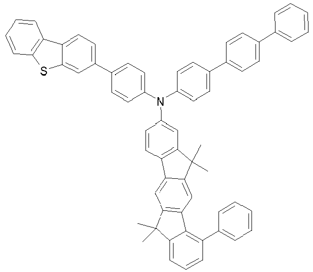 |
| **1-6** | 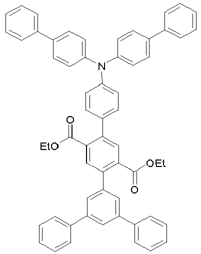 | MeMgCl | 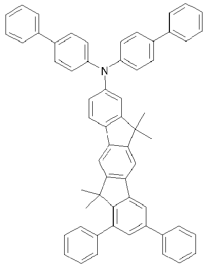 |
| **1-7** | 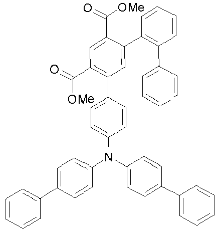 | MeMgBr | 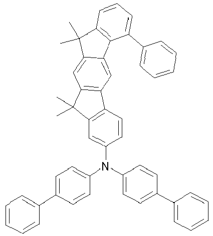 |
| **III-1** | 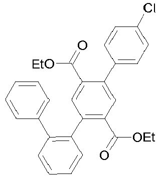 | MeLi | 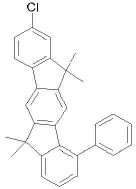 |
| **III-2** | 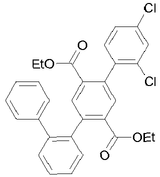 | MeMgBr | 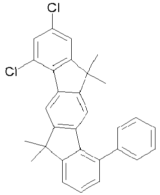 |
| **III-3** | 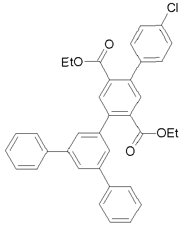 | MeMgBr | 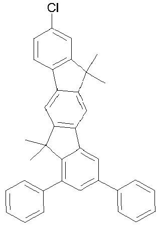 |
| **III-4** | 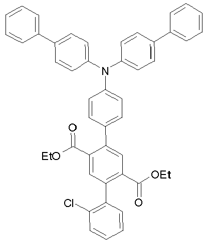 | MeMgBr | 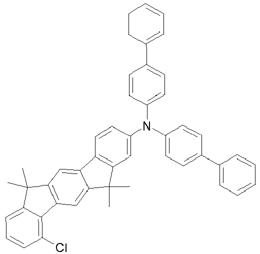 |
| **III-5** | 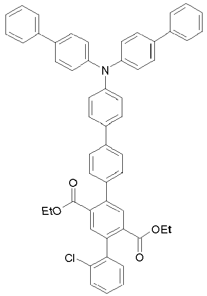 | MeMgCl | 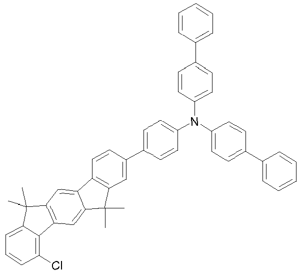 |
| **III-6** | 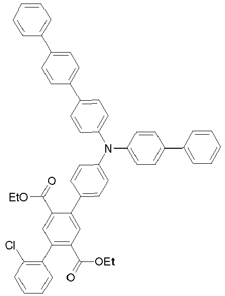 | MeLi | 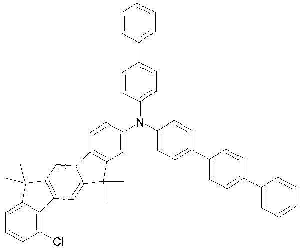 |
| **III-7** | 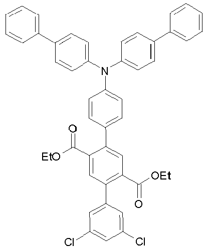 | MeMgBr | 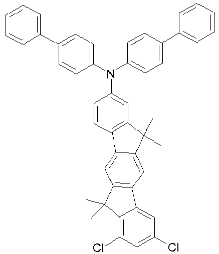 |
| **III-8** | 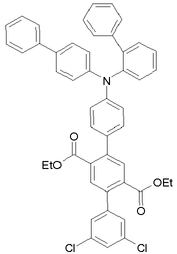 | MeMgCl | 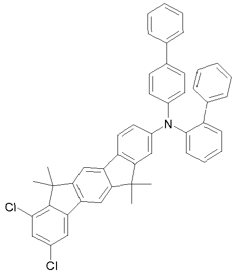 |
| **III-9** | 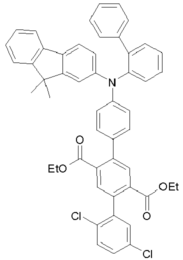 | MeMgBr | 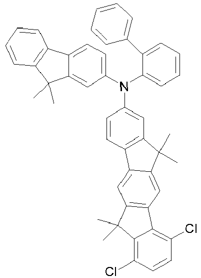 |
| **III-10** | 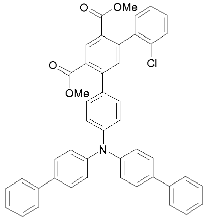 | MeMgBr | 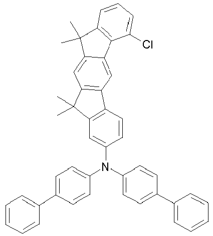 |
| **III-11** | 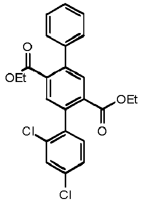 | MeMgCl | 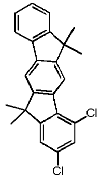 |
| **III-12** | 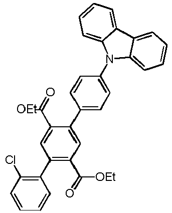 | MeMgCl | 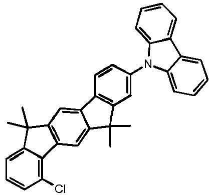 |

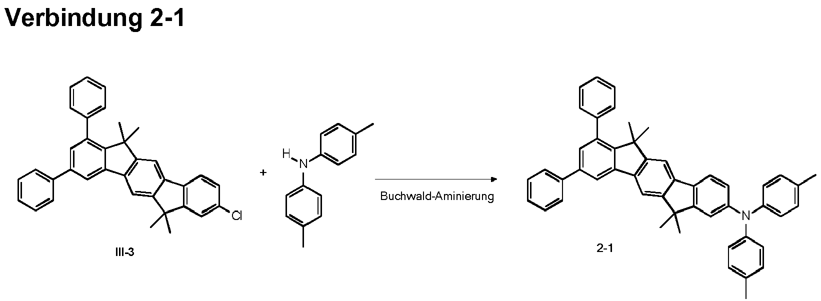

5,95 g Bis-p-Tolyl-amin (30,2 mmol) und 15 g der Zwischenstufe III-1 (30,2 mmol) werden in 300 mL Toluol gelöst. Die Lösung wird entgast und mit N₂ gesättigt. Danach wird sie mit 0,28 g (0,302 mmol) Pd₂(dba)₃ und 0,6 mL einer 1M Lösung von (tBu)₃P versetzt und anschließend werden 4,3 g Natrium-tert-butylat (45,3 mol) zugegeben. Die Reaktionsmischung wird 6 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert.

Nach Filtration des Rohproduktes über Kieselgel mit Toluol wird der verbleibende Rückstand aus Heptan/Toluol umkristallisiert. Die Ausbeute beträgt 14,3 g (72% d. Th). Abschließend wird das Material im Hochvakuum sublimiert. Reinheit beträgt 99.9%.

Analog dazu werden folgende Verbindungen hergestellt:

| | Edukt 1 | Edukt 2 | Produkt |
|---|---|---|---|
| **2-2** | 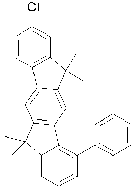 | 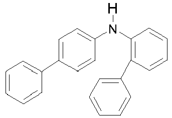 | 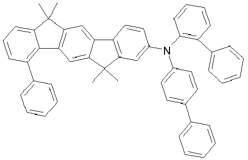 |
| **2-3** | 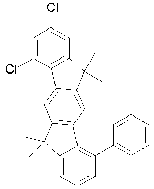 | 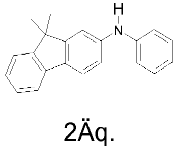 | 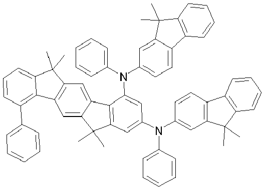 |
| **2-4** | 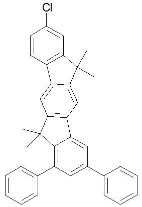 | 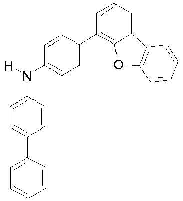 | 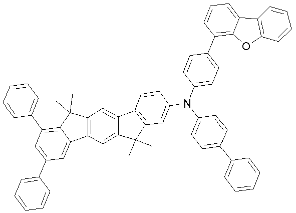 |
| **2-5** | 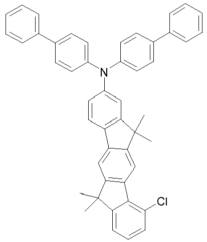 | 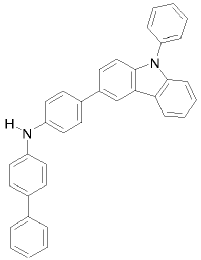 | 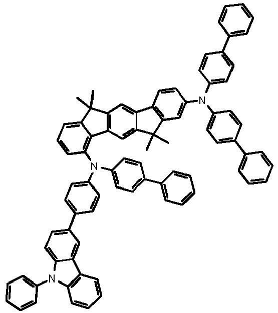 |
| **2-6** | 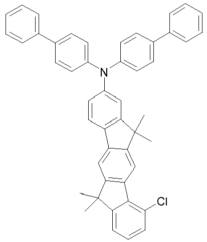 | 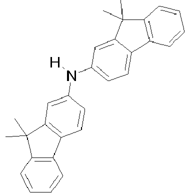 | 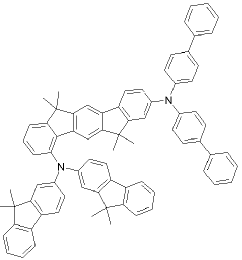 |
| **2-7** | 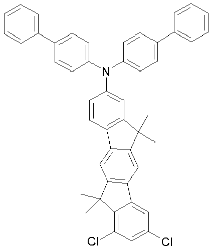 | 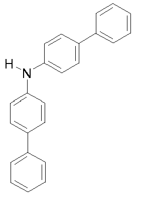 | 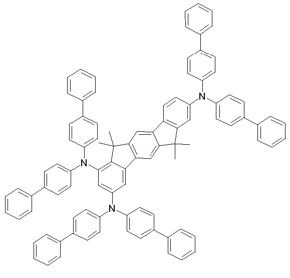 |
| **2-8** | 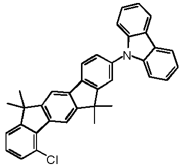 | 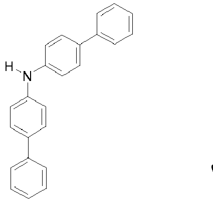 | 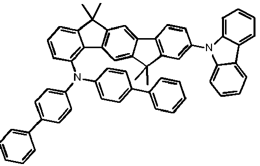 |
| **2-9** | 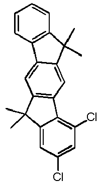 | 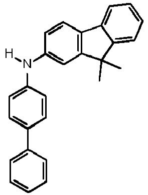 | 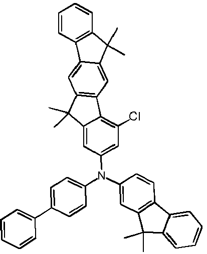 |
| **2-10** | 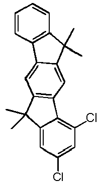 | 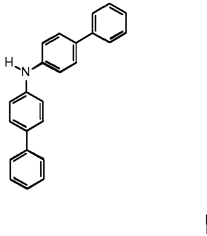 | 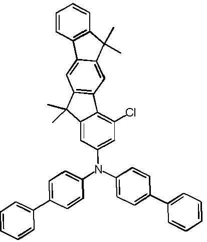 |

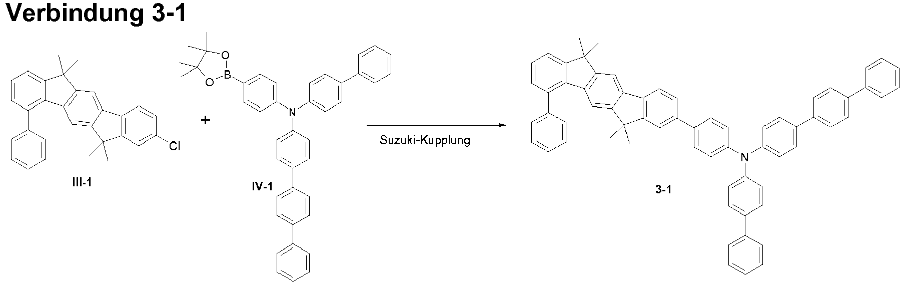

17,1 g (28,51 mmol) des Pinacolboronester-Derivats IV-1 und 12 g (28,51 mmol) Zwischenstufe III-1 werden in 350 mL Toluol und 4,1 g Natrium-tert-Butylat (42,8 mmol) suspendiert. Zu dieser Suspension werden 0,26 g (0,285 mmol) Pd₂(dba)₂ gegeben, und die Reaktionsmischung wird 12h unter Rückfluss erhitzt. Nach Erkalten wird die organische Phase abgetrennt, über Kieselgel filtriert, dreimal mit 80 mL Wasser gewaschen und anschließend zur Trockene eingeengt. Nach Filtration des Rohproduktes über Kieselgel mit Toluol wird der verbleibende Rückstand aus Heptan/Toluol umkristallisiert. Die Ausbeute beträgt 18 g (75% d. Th). Abschließend wird das Material im Hochvakuum sublimiert. Reinheit beträgt 99.9%.

Analog dazu werden folgende Verbindungen hergestellt:

| | Edukt 1 | Edukt 2 | Produkt |
|---|---|---|---|
| **3-2** | 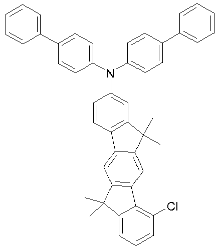 | 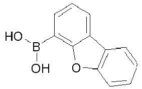 | 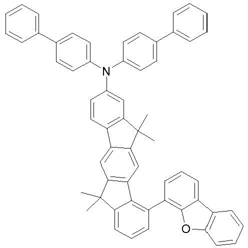 |
| **3-3** | 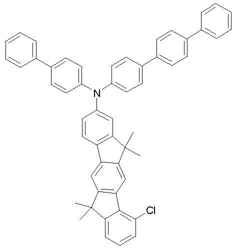 | 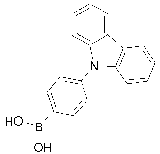 | 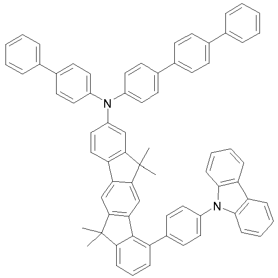 |
| **3-4** | 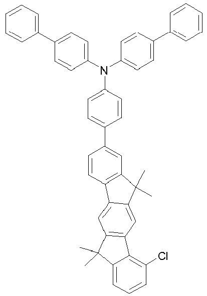 | 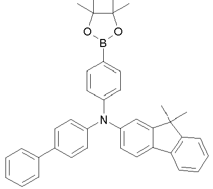 | 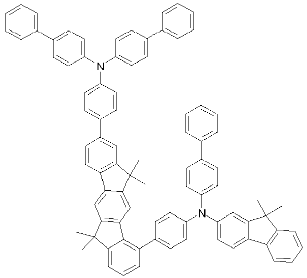 |
| **3-5** | 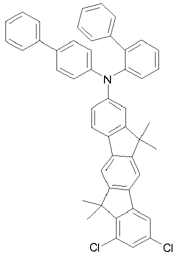 | 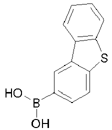 | 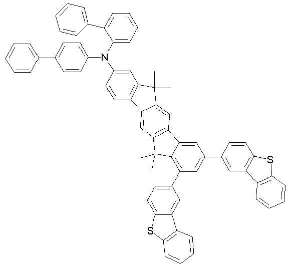 |
| **3-6** | 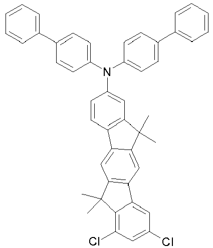 | 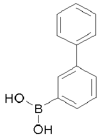 | 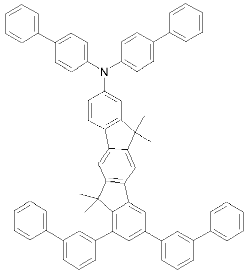 |
| **3-7** | 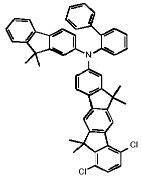 | 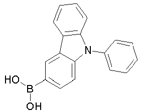 | 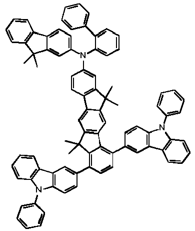 |
| **3-8** | 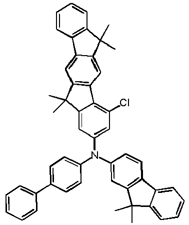 | 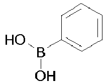 | 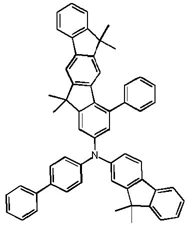 |
| **3-9** | 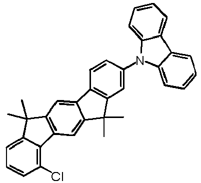 | 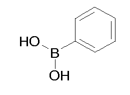 | 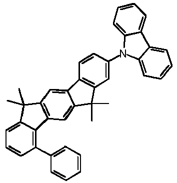 |
| **3-10** | 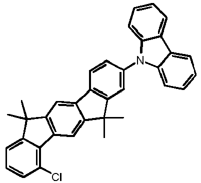 | 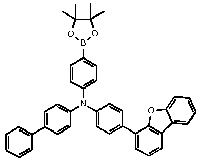 | 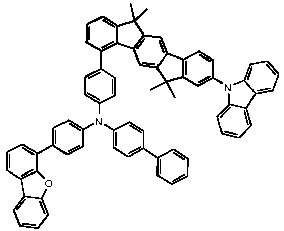 |
| **3-11** | 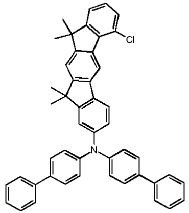 | 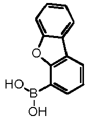 | 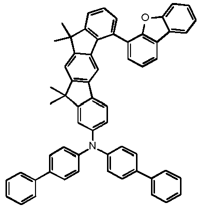 |
| **3-12** | 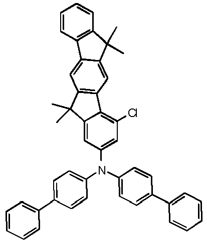 | 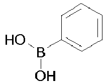 | 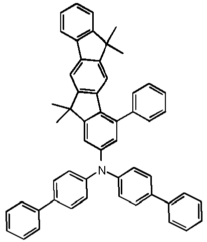 |
| **3-13** | 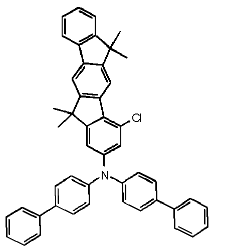 | 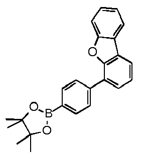 | 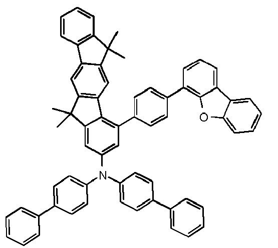 |

### B) Device-Beispiele

### 1) Allgemeines Herstellungsverfahren für die OLEDs und Charakterisierung der OLEDs

Glasplättchen, die mit strukturiertem ITO (Indium Zinn Oxid) der Dicke 50 nm beschichtet sind, bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Der genaue Aufbau der OLEDs ist den folgenden Tabellen zu entnehmen. Die zur Herstellung der OLEDs benötigten Materialien sind in einer folgenden Tabelle gezeigt.

Alle Materialien werden in einer Vakuumkammer thermisch aufgedampft. Dabei besteht die Emissionsschicht aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1 :EG1 :TEG1 (55%:35%:10%) bedeutet hierbei, dass das Material IC1 in einem Volumenanteil von 55%, EG1 in einem Anteil von 35% und TEG1 in einem Anteil von 10% in der Schicht vorliegt. Analog besteht auch die Elektronentransportschicht und die Lochinjektionsschicht aus einer Mischung von zwei Materialien. Die Strukturen der Materialien, die in den OLEDs verwendet werden, sind in Tabelle 1 gezeigt.

Die OLEDs werden standardmäßig charakterisiert. Hierfür werden die Elektrolumineszenzspektren, die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Angabe EQE @ 10mA/cm² bezeichnet die externe Quanteneffizienz, die bei 10mA/cm² erreicht wird. Die Angabe U @ 10 mA/cm² bezeichnet die Betriebsspannung bei 10 mA/cm². Als Lebensdauer LT80 @ 60mA/cm2 wird die Zeit definiert, nach der die Leuchtdichte bei Betrieb mit einer Stromdichte von 60 mA/cm² auf 80% ihres Anfangswertes abgesunken ist, ohne Verwendung eines Beschleunigungsfaktors.

### 2) Verwendung der erfindungsgemäßen Verbindungen in der HIL und HTL von blau fluoreszierenden Devices

Es werden OLEDs mit dem folgenden Aufbau hergestellt:

| ***Ex.*** | ***HIL*** | ***HTL*** | ***EBL*** | ***EML*** | ***ETL*** | ***EIL*** |
|---|---|---|---|---|---|---|
| | Dicke / nm | Dicke / nm | Dicke / nm | Dicke / nm | Dicke / nm | Dicke / nm |
| E1 | HTM-2: p-dotiert (5%) 20 nm | HTM-2 180 nm | EBM 10 nm | H:SEB(5%) 20 nm | ETM:LiQ(50%) 30 nm | LiQ 1 nm |

Dabei werden sehr gute Ergebnisse für die Leistungsdaten der OLED erhalten: Die EQE @ 10 mA/cm² beträgt ca. 7.5 %, die Betriebsspannung U @ 10 mA/cm² beträgt ca. 4 V, und die Lebensdauer LT80 @ 60 mA/cm² beträgt mehr als 300 h.

### 3) Verwendung der erfindungsgemäßen Verbindungen in der EBL von grün phosphoreszierenden Devices

Es werden OLEDs mit dem folgenden Aufbau hergestellt:

| ***Ex.*** | ***HIL*** | ***HTL*** | ***EBL*** | ***EML*** | ***ETL*** | ***EIL*** |
|---|---|---|---|---|---|---|
| | Dicke / nm | Dicke / nm | Dicke / nm | Dicke / nm | Dicke / nm | Dicke / nm |
| E2 | HTM: p-dotiert (5%) 20 nm | HTM 220 nm | HTM-1 10 nm | TMM-1: TMM-2 (28%):TEG(12%) 30 nm | ETM:LiQ(50%) 30 nm | LiQ 1 nm |
| E3 | HTM: p-dotiert (5%) 20 nm | HTM 220 nm | HTM-2 10 nm | TMM-1: TMM-2 (28%):TEG(12%) 30 nm | ETM:LiQ(50%) 30 nm | LiQ 1 nm |
| E4 | HTM: p-dotiert (5%) 20 nm | HTM 220 nm | HTM-3 10 nm | TMM-1: TMM-2 (28%):TEG(12%) 30 nm | ETM:LiQ(50%) 30 nm | LiQ 1 nm |
| E5 | HTM: p-dotiert (5%) 20 nm | HTM 220 nm | HTM-4 10 nm | TMM-1: TMM-2 (28%):TEG(12%) 30 nm | ETM:LiQ(50%) 30 nm | LiQ 1 nm |
| E6 | HTM: p-dotiert (5%) 20 nm | HTM 220 nm | HTM-5 10 nm | TMM-1: TMM-2 (28%):TEG(12%) 30 nm | ETM:LiQ(50%) 30 nm | LiQ 1 nm |

Dabei werden in allen Fällen sehr gute Ergebnisse für die Leistungsdaten der OLEDs erhalten: Die EQE @ 10 mA/cm² beträgt ca. 14 % in allen Fällen, die Betriebsspannung U @ 10 mA/cm² beträgt ca. 4 V in allen Fällen, und die Lebensdauer LT80 @ 60 mA/cm² beträgt mehr als 300 h in allen Fällen.

### 4) Vergleich der OLED-Leistungsdaten zwischen erfindungsgemäßer Verbindung HTM-1 und Vergleichsverbindung RefHTM

Es wird eine erfindungsgemäße OLED E6 hergestellt, die denselben Aufbau wie die unter 2) beschriebene OLED E1 aufweist, mit dem einzigen Unterschied, dass die Verbindung HTM-1 anstelle der Verbindung HTM-2 in der HIL und der HTL der OLED vorliegt.

Zum Vergleich wird eine OLED V1 hergestellt, die denselben Aufbau wie die erfindungsgemäße OLED E6 aufweist, mit dem einzigen Unterschied, dass die Verbindung RefHTM anstelle der Verbindung HTM-1 in der HIL und der HTL der OLED vorliegt.

Bei der erfindungsgemäßen OLED E6 wird ein deutlich verbesserter Wert für die EQE und eine leicht verringerte Betriebsspannung gefunden, verglichen mit der Vergleichs-OLED V1. Die Lebensdauer LT80 @ 60 mA/cm² beträgt mehr als 300 h in beiden Fällen. Die erhaltenen Werte sind in der folgenden Tabelle gezeigt:

| | U @ 10 mA/cm² | EQE @ 10 mA/cm² |
|---|---|---|
| | [V] | [%] |
| E1 | 3.8 | 7.7 |
| V1 | 4.0 | 7.4 |

| | | |
|---|---|---|
| **Tabelle 1: Verwendete Verbindungen** | | |
| 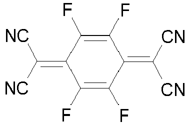 | 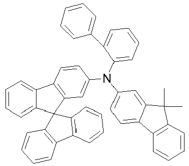 | 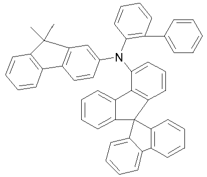 |
| p-Dotand F4TCNQ | HTM | EBM |
| 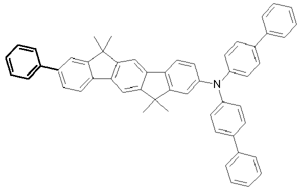 | 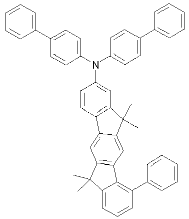 | 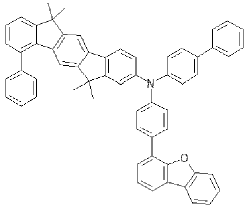 |
| RefHTM | HTM-1 | HTM-2 |
| 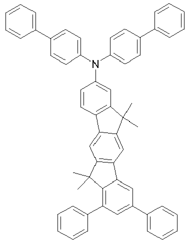 | 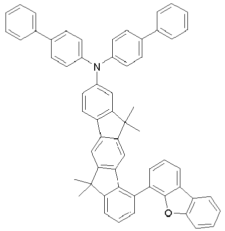 | 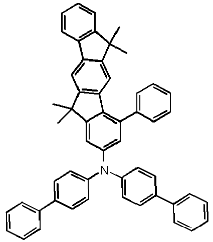 |
| HTM-3 | HTM-4 | HTM-5 |
| 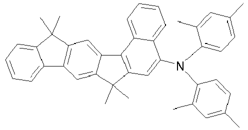 | 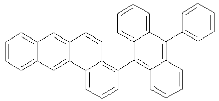 | 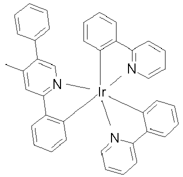 |
| SEB | H | TEG |
| 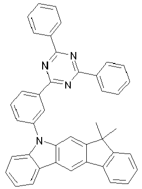 | 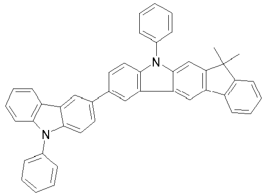 | 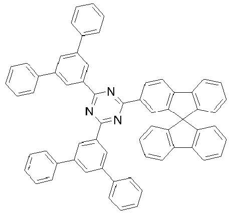 |
| TMM-1 | TMM-2 | ETM |
| 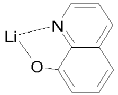 | | |
| LiQ | | |

## Patentansprüche

1. Verbindung der folgenden Formel (I) oder (II) 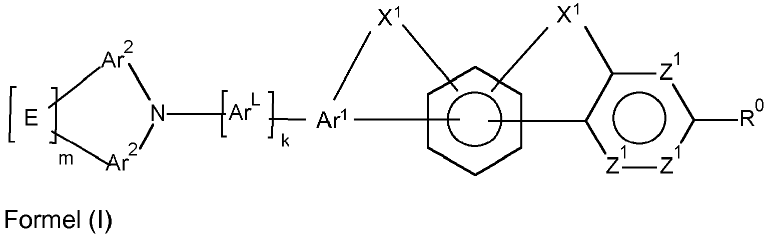 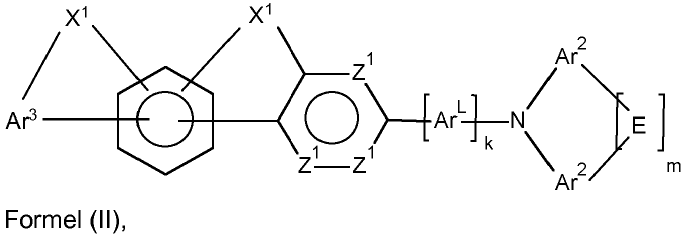 wobei für die auftretenden Variablen gilt:
Z¹ ist bei jedem Auftreten gleich oder verschieden gewählt aus CR¹ und CR³;
Ar¹ ist eine Arylgruppe mit 6 bis 20 aromatischen Ringatomen, die mit einem oder mehreren Resten R³ substituiert sein kann, oder eine Heteroarylgruppe mit 5 bis 20 aromatischen Ringatomen, die mit einem oder mehreren Resten R³ substituiert sein kann;
Ar² ist bei jedem Auftreten gleich oder verschieden gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
Ar³ ist eine Arylgruppe mit 6 bis 20 aromatischen Ringatomen, die mit einem oder mehreren Resten R² substituiert sein kann, oder eine Heteroarylgruppe mit 5 bis 20 aromatischen Ringatomen, die mit einem oder mehreren Resten R² substituiert sein kann;
X¹ ist bei jedem Auftreten gleich oder verschieden eine divalente Gruppe gewählt aus -C(R⁴)₂-, -C(R⁴)₂-C(R⁴)₂-, -CR⁴=CR⁴-, -Si(R⁴)₂-, NR⁴, O und S;
Ar^{L} ist gewählt aus aromatischen Ringsystemen mit 6 bis 40 aromatischen Ringatomen, die mit einem oder mehreren Resten R⁵ substituiert sein können, und heteroaromatischen Ringsystemen mit 5 bis 40 aromatischen Ringatomen, die mit einem oder mehreren Resten R⁵ substituiert sein können;
E ist eine Einfachbindung oder eine divalente Gruppe gewählt aus C(R⁵)₂, Si(R⁵)₂, N(R⁵), O, und S;
R⁰ ist gewählt aus H, D, aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können;
R¹ ist bei jedem Auftreten gleich oder verschieden gewählt aus aromatischen Ringsystemen mit 6 bis 40 aromatischen Ringatomen, die mit einem oder mehreren Resten R⁶ substituiert sein können, und heteroaromatischen Ringsystemen mit 5 bis 40 aromatischen Ringatomen, die mit einem oder mehreren Resten R⁶ substituiert sein können;
R² ist bei jedem Auftreten gleich oder verschieden gewählt aus H, D, F, CN, Si(R⁶)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁰ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch-R⁶C=CR⁶-, -C≡C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R³, R⁴, R⁵ bei jedem Auftreten gleich oder verschieden gewählt aus H, D, F, C(=O)R⁶, CN, Si(R⁶)₃, N(R⁶)₂, P(=O)(R⁶)₂, OR⁶, S(=O)R⁶, S(=O)₂R⁶, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ bzw. R⁴ bzw. R⁵ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁶ substituiert sein können; und wobei eine oder mehrere CH₂-Gruppen in den genannten Alkyl-, Alkoxy-, Alkenyl- und Alkinylgruppen durch -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO oder SO₂ ersetzt sein können;
R⁶ ist bei jedem Auftreten gleich oder verschieden gewählt aus H, D, F, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁷ substituiert sein können; und wobei eine oder mehrere CH₂-Gruppen in den genannten Alkyl-, Alkoxy-, Alkenyl- und Alkinylgruppen durch -R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R⁷ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁷ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
k ist gleich 0, 1, 2 oder 3, wobei im Fall k=0 die Gruppe Ar^{L} wegfällt und das Stickstoffatom der Gruppe der Formel (N) die Anbindungsposition darstellt;
m ist gleich 0 oder 1, wobei im Fall m=0 die Gruppe E wegfällt und die Gruppen Ar² nicht miteinander verbunden sind;
wobei in Formel (I) und (II) alle unsubstituiert gezeichneten Positionen jeweils mit einem Rest R³ substituiert sein können; und
wobei in Formel (I) und in Formel (II) jeweils mindestens eine Gruppe Z¹ vorliegt, die CR¹ ist.

2. Verbindung nach Anspruch 1, **dadurch gekennzeichnet, dass** in einer der Formeln (I) und (II) jeweils eine oder zwei Gruppen Z¹ gleich CR¹ sind, und die anderen Gruppen Z¹ gleich CR³ sind.

3. Verbindung nach Anspruch 1 oder 2, **dadurch gekennzeichnet, dass**

4. Verbindung nach einem oder mehreren der Ansprüche 1 bis 3, **dadurch gekennzeichnet, dass** X¹ gleich C(R⁴)₂ ist.

5. Verbindung nach einem oder mehreren der Ansprüche 1 bis 4, **dadurch gekennzeichnet, dass** k gleich 0 ist.

6. Verbindung nach einem oder mehreren der Ansprüche 1 bis 4, **dadurch gekennzeichnet, dass** k nicht gleich 0 ist und -(Ar^{L})ₖ- einer der folgenden Formeln entspricht
| | | |
|---|---|---|
| 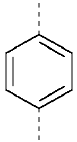 | 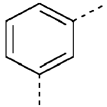 | 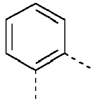 |
| Ar^{L}-1 | Ar^{L}-2 | Ar^{L}-3 |
| 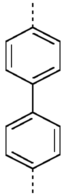 | 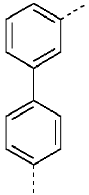 | 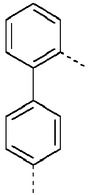 |
| Ar^{L}-4 | Ar^{L}-5 | Ar^{L}-6 |
| 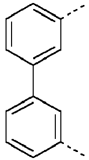 | 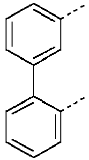 | 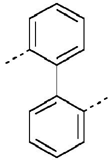 |
| Ar^{L}-7 | Ar^{L}-8 | Ar^{L}-9 |
| 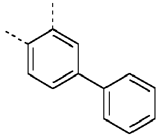 | 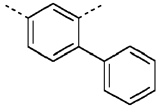 | 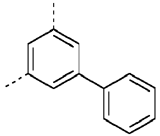 |
| Ar^{L}-10 | Ar^{L}-11 | Ar^{L}-12 |
| 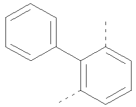 | 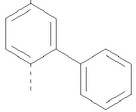 | 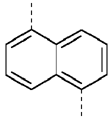 |
| Ar^{L}-13 | Ar^{L}-14 | Ar^{L}-15 |
| 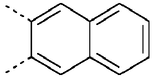 | 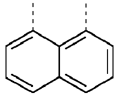 | 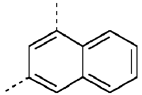 |
| Ar^{L}-16 | Ar^{L}-17 | Ar^{L}-18 |
| 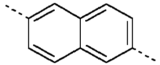 | 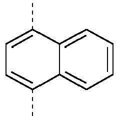 | 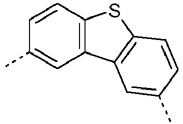 |
| Ar^{L}-19 | Ar^{L}-20 | Ar^{L}-21 |
| 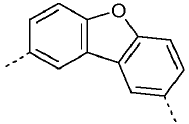 | 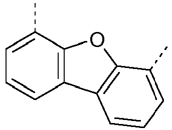 | 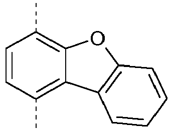 |
| Ar^{L}-22 | Ar^{L}-23 | Ar^{L}-24 |
| 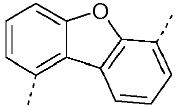 | 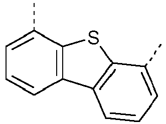 | 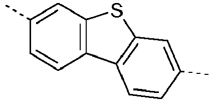 |
| Ar^{L}-25 | Ar^{L}-26 | Ar^{L}-27 |
| 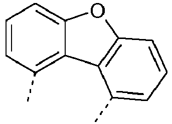 | 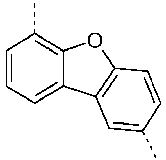 | 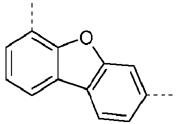 |
| Ar^{L}-28 | Ar^{L}-29 | Ar^{L}-30 |
| 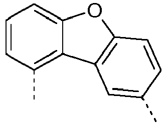 | 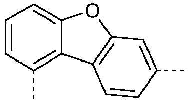 | 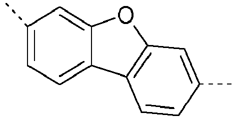 |
| Ar^{L}-31 | Ar^{L}-32 | Ar^{L}-33 |
| 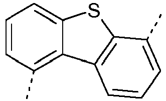 | 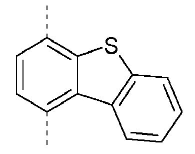 | 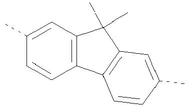 |
| Ar^{L}-34 | Ar^{L}-35 | Ar^{L}-36 |
| 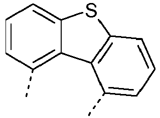 | 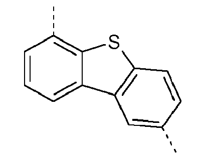 | 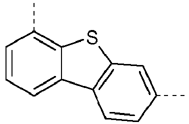 |
| Ar^{L}-37 | Ar^{L}-38 | Ar^{L}-39 |
| 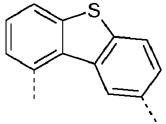 | 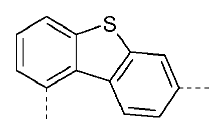 | 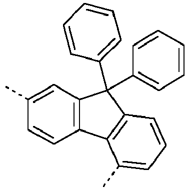 |
| Ar^{L}-40 | Ar^{L}-41 | Ar^{L}-42 |
| 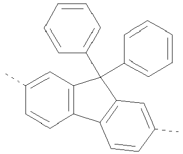 | 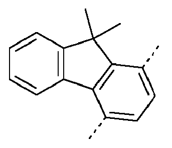 | 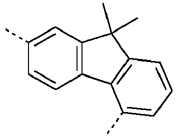 |
| Ar^{L}-43 | Ar^{L}-44 | Ar^{L}-45 |
| 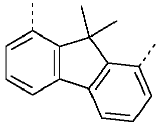 | 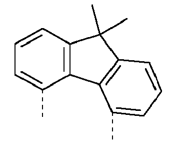 | 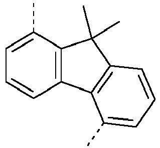 |
| Ar^{L}-46 | Ar^{L}-47 | Ar^{L}-48 |
| 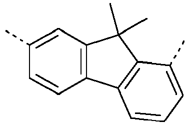 | 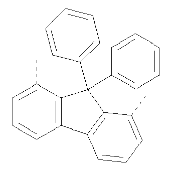 | 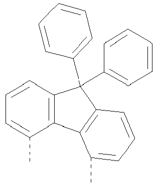 |
| Ar^{L}-49 | Ar^{L}-50 | Ar^{L}-51 |
| 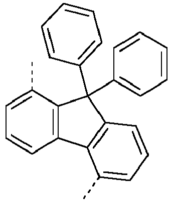 | 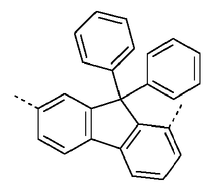 | 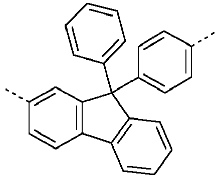 |
| Ar^{L}-52 | Ar^{L}-53 | Ar^{L}-54 |
| 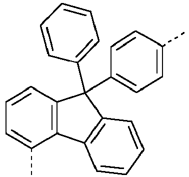 | 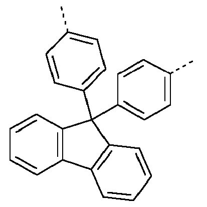 | 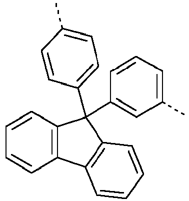 |
| Ar^{L}-55 | Ar^{L}-56 | Ar^{L}-57 |
| 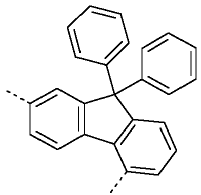 | 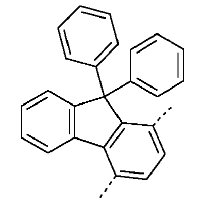 | 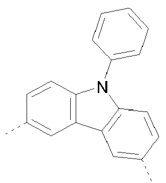 |
| Ar^{L}-58 | Ar^{L}-59 | Ar^{L}-60 |
| 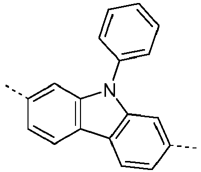 | 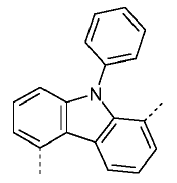 | 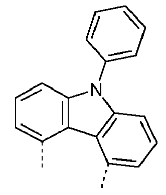 |
| Ar^{L}-61 | Ar^{L}-62 | Ar^{L}-63 |
| 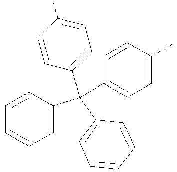 | 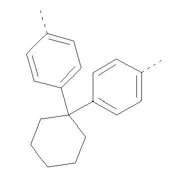 | 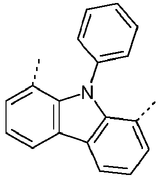 |
| Ar^{L}-64 | Ar^{L}-65 | Ar^{L}-66 |
| 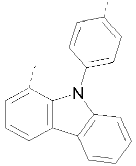 | 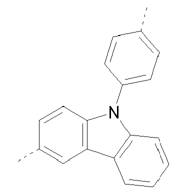 | 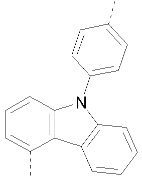 |
| Ar^{L}-67 | Ar^{L}-68 | Ar^{L}-69 |
| 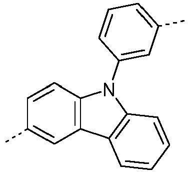 | 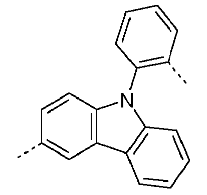 | 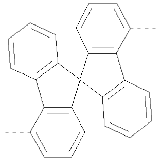 |
| Ar^{L}-70 | Ar^{L}-71 | Ar^{L}-72 |
| 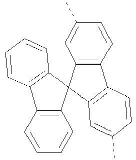 | 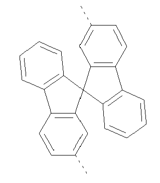 | 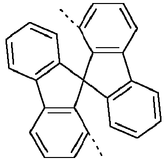 |
| Ar^{L}-73 | Ar^{L}-74 | Ar^{L}-75 |
wobei die gestrichelten Linien jeweils die Bindungen an den Rest der Formel (I) bzw. (II) darstellen.

7. Verbindung nach einem oder mehreren der Ansprüche 1 bis 6, **dadurch gekennzeichnet, dass** Ar² bei jedem Auftreten gleich oder verschieden gewählt ist aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Indenocarbazolyl, Dibenzofuranyl, Dibenzothiophenyl, Carbazolyl, Benzofuranyl, Benzothiophenyl, benzokondensiertes Dibenzofuranyl, benzokondensiertes Dibenzothiophenyl, Naphthyl-substituiertes Phenyl, Fluorenyl-substituiertes Phenyl, Spirobifluorenyl-substituiertes Phenyl, Dibenzofuranyl-substituiertes Phenyl, Dibenzothiophenyl-substituiertes Phenyl, Carbazolyl-substituiertes Phenyl, Pyridyl-substituiertes Phenyl, Pyrimidyl-substituiertes Phenyl, und Triazinyl-substituiertes Phenyl, wobei die genannten Gruppen jeweils mit einem oder mehreren Resten R⁵ substituiert sein können.

8. Verbindung nach einem oder mehreren der Ansprüche 1 bis 7, **dadurch gekennzeichnet, dass** die Gruppe 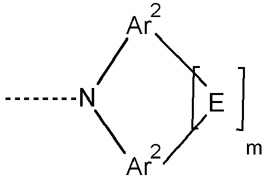 in Formel (I) und (II) jeweils gewählt ist aus den folgenden Formeln
| | |
|---|---|
| 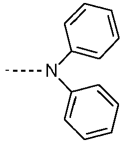 | 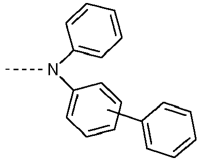 |
| A-1 | A-2 |
| 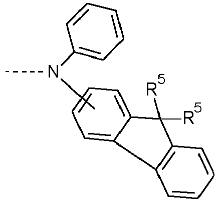 | 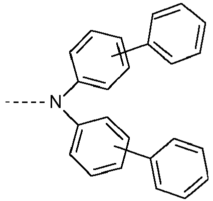 |
| A-3 | A-4 |
| 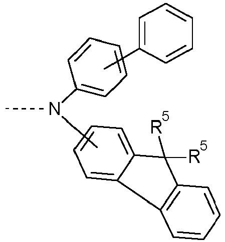 | 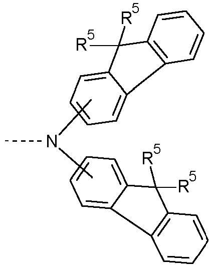 |
| A-5 | A-6 |
| 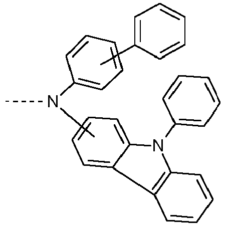 | 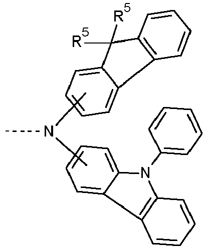 |
| A-7 | A-8 |
| 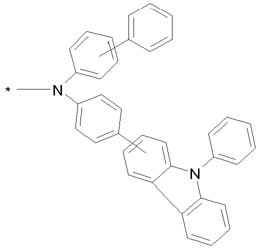 | 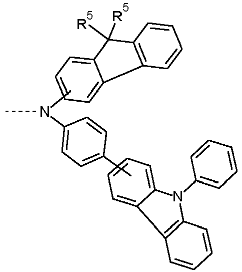 |
| A-9 | A-10 |
| 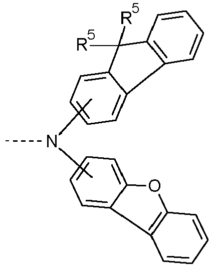 | 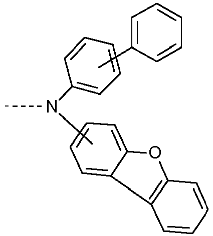 |
| A-11 | A-12 |
| 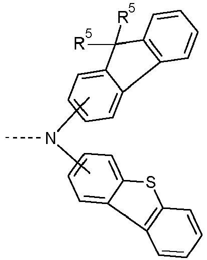 | 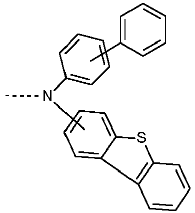 |
| A-13 | A-14 |
| 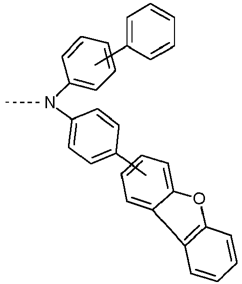 | 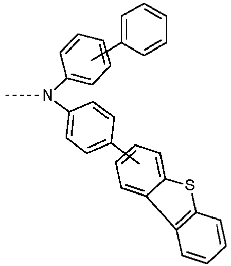 |
| A-15 | A-16 |
| 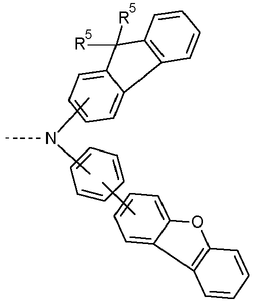 | 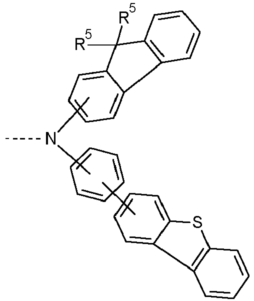 |
| A-17 | A-18 |
| 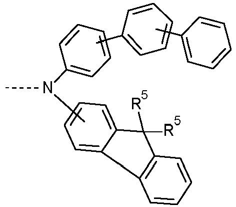 | 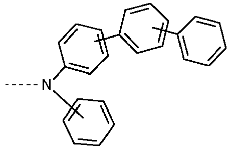 |
| A-19 | A-20 |
| 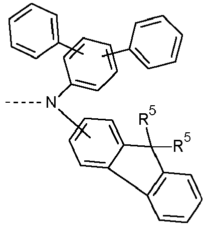 | 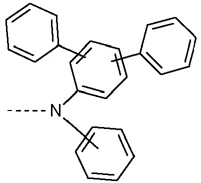 |
| A-21 | A-22 |
| 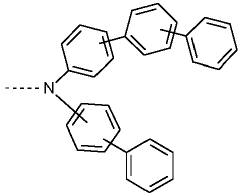 | 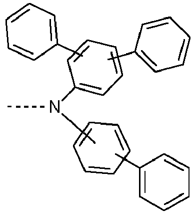 |
| A-23 | A-24 |
| 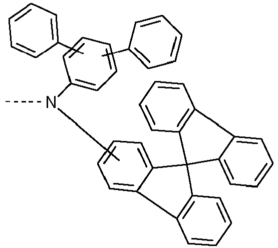 | 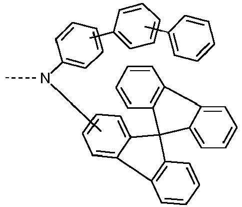 |
| A-25 | A-26 |
| 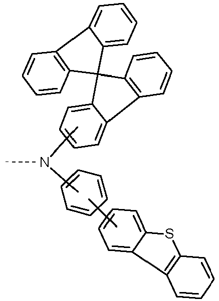 | 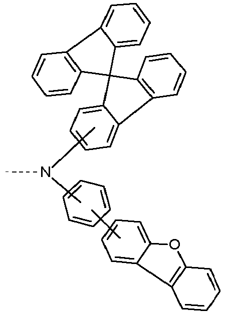 |
| A-27 | A-28 |
| 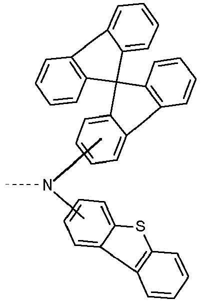 | 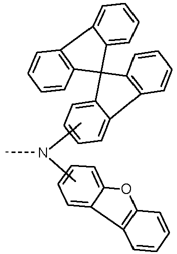 |
| A-29 | A-30 |
| 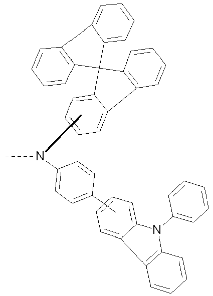 | 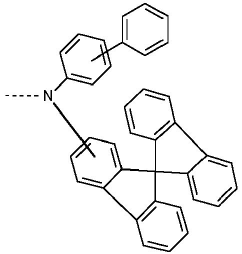 |
| A-31 | A-32 |
| 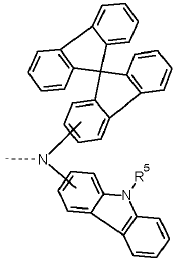 | 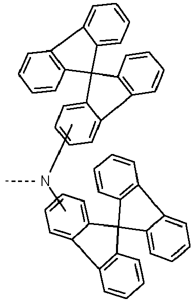 |
| A-33 | A-34 |
| 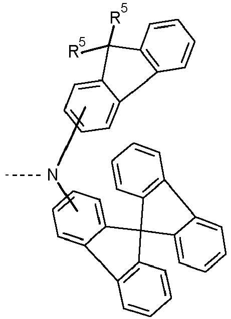 | 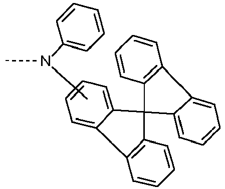 |
| A-35 | A-36 |
| 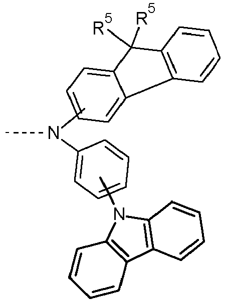 | 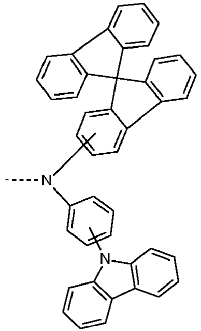 |
| A-37 | A-38 |
| 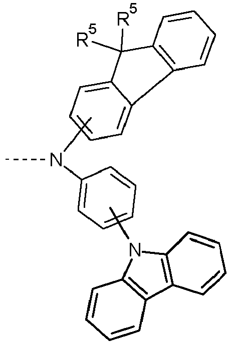 | |
| A-39 | |
wobei die Gruppen an ihren freien Positionen jeweils mit einem Rest R⁵ substituiert sein können, und bevorzugt in den freien Positionen unsubstituiert sind, und wobei die gestrichelten Bindungen die Bindungen an den Rest der Formel (I) bzw. (II) darstellen.

9. Verbindung nach einem oder mehreren der Ansprüche 1 bis 8, **dadurch gekennzeichnet, dass** R⁰ gleich H ist.

10. Verbindung nach einem oder mehreren der Ansprüche 1 bis 9, **dadurch gekennzeichnet, dass** R¹ bei jedem Auftreten gleich oder verschieden gewählt ist aus monovalenten Gruppen abgeleitet von Benzol, Biphenyl, Terphenyl, Quaterphenyl, Naphthalin, Fluoren, insbesondere 9,9'-Dimethylfluoren und 9,9'-Diphenylfluoren, Benzofluoren, Spirobifluoren, Indenofluoren, Indenocarbazol, Dibenzofuran, Dibenzothiophen, Benzocarbazol, Carbazol, Benzofuran, Benzothiophen, Indol, Chinolin, Pyridin, Pyrimidin, Pyrazin, Pyridazin, und Triazin, wobei die monovalenten Gruppen jeweils mit einem oder mehreren Resten R⁶ substituiert sein können.

11. Verbindung nach einem oder mehreren der Ansprüche 1 bis 10, **dadurch gekennzeichnet, dass** R² und R³ gleich H sind.

12. Verbindung nach einem oder mehreren der Ansprüche 1 bis 11, **dadurch gekennzeichnet, dass** Formel (I) einer der folgenden Formeln entspricht:
| |
|---|
| 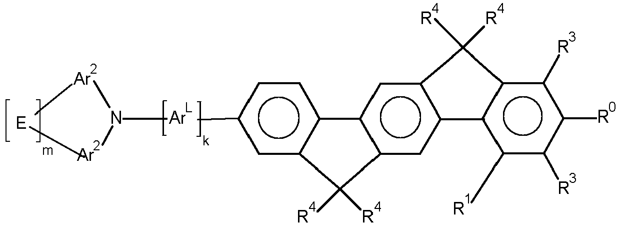 |
| Formel (I-A-1-a) |
| 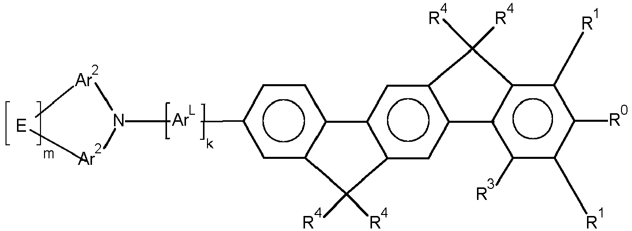 |
| Formel (I-A-2-a) |
| 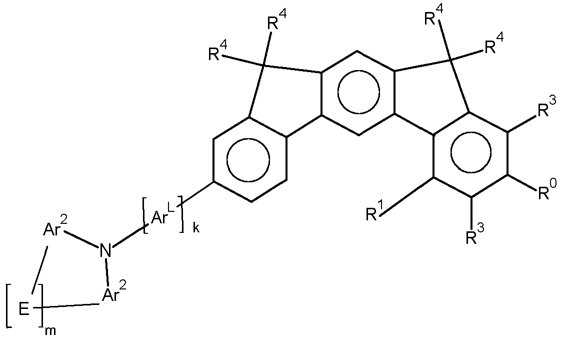 |
| Formel (I-B-1-a) |

13. Verfahren zur Herstellung einer Verbindung gemäß Formel (I) oder (II) nach einem oder mehreren der Ansprüche 1 bis 12, **dadurch gekennzeichnet, dass** eine Benzolverbindung, die zwei Carbonsäureestergruppen und mindestens eine reaktive Gruppe trägt, mit einer Benzolverbindung umgesetzt wird, die eine Boronsäuregruppe und mindestens eine Gruppe gewählt aus reaktiven Gruppen X und aromatischen oder heteroaromatischen Gruppen Ar enthält, wobei die Boronsäuregruppe und die mindestens eine Gruppe gewählt aus Gruppen X und Ar in ortho- oder in meta-Stellung am Benzolring zueinander stehen.

14. Oligomer, Polymer oder Dendrimer, enthaltend eine oder mehrere Verbindungen gemäß Formel (I) oder (II) nach einem oder mehreren der Ansprüche 1 bis 12, wobei die Bindung(en) zum Polymer, Oligomer oder Dendrimer an beliebigen, in Formel (I) oder (II) mit R¹, R², R³, R⁴ oder R⁵ substituierten Positionen lokalisiert sein können.

15. Formulierung, enthaltend mindestens eine Verbindung nach einem oder mehreren der Ansprüche 1 bis 12 oder mindestens ein Polymer, Oligomer oder Dendrimer nach Anspruch 14, sowie mindestens ein Lösungsmittel.

16. Elektronische Vorrichtung, enthaltend mindestens eine Verbindung nach einem oder mehreren der Ansprüche 1 bis 12, oder mindestens ein Polymer, Oligomer oder Dendrimer nach Anspruch 14.

17. Elektronische Vorrichtung nach Anspruch 16, **dadurch gekennzeichnet, dass** sie eine organische Elektrolumineszenzvorrichtung ist und Anode, Kathode und mindestens eine emittierende Schicht enthält, und dass die Verbindung in einer lochtransportierenden Schicht der Vorrichtung enthalten ist.

18. Organische Elektrolumineszenzvorrichtung nach Anspruch 17, **dadurch gekennzeichnet, dass** die Verbindung in einer Elektronenblockierschicht der Vorrichtung enthalten ist.

19. Verwendung einer Verbindung nach einem oder mehreren der Ansprüche 1 bis 12 in einer elektronischen Vorrichtung.

## Claims

1. Compound of the following formula (I) or (II) 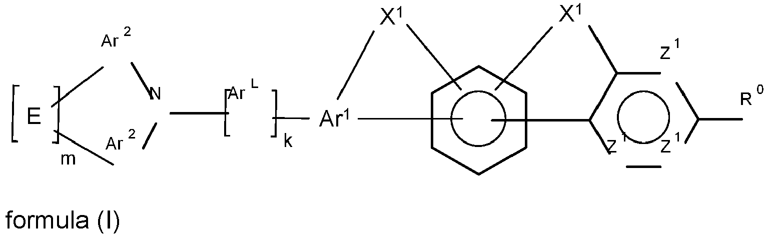 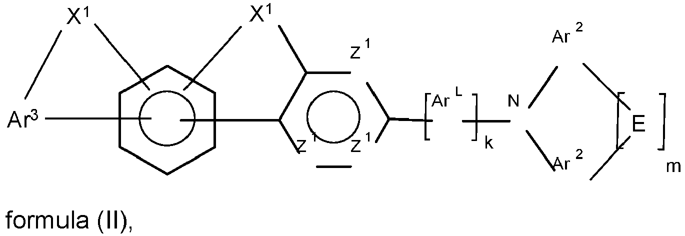 where the following applies to the variables occurring:
Z¹ is selected on each occurrence, identically or differently, from CR¹ and CR³;
Ar¹ is an aryl group having 6 to 20 aromatic ring atoms, which may be substituted by one or more radicals R³, or a heteroaryl group having 5 to 20 aromatic ring atoms, which may be substituted by one or more radicals R³;
Ar² is selected on each occurrence, identically or differently, from aromatic ring systems having 6 to 40 aromatic ring atoms, which may be substituted by one or more radicals R⁵, and heteroaromatic ring systems having 5 to 40 aromatic ring atoms, which may be substituted by one or more radicals R⁵;
Ar³ is an aryl group having 6 to 20 aromatic ring atoms, which may be substituted by one or more radicals R², or a heteroaryl group having 5 to 20 aromatic ring atoms, which may be substituted by one or more radicals R²;
X¹ is on each occurrence, identically or differently, a divalent group selected from -C(R⁴)₂-, -C(R⁴)₂-C(R⁴)₂-, -CR⁴=CR⁴-, -Si(R⁴)₂-, NR⁴, O and S;
Ar^{L} is selected from aromatic ring systems having 6 to 40 aromatic ring atoms, which may be substituted by one or more radicals R⁵, and heteroaromatic ring systems having 5 to 40 aromatic ring atoms, which may be substituted by one or more radicals R⁵;
E is a single bond or a divalent group selected from C(R⁵)₂, Si(R⁵)₂, N(R⁵), O and S;
R⁰ is selected from H, D, aromatic ring systems having 6 to 40 aromatic ring atoms, which may be substituted by one or more radicals R⁶, and heteroaromatic ring systems having 5 to 40 aromatic ring atoms, which may be substituted by one or more radicals R⁶;
R¹ is selected on each occurrence, identically or differently, from aromatic ring systems having 6 to 40 aromatic ring atoms, which may be substituted by one or more radicals R⁶, and heteroaromatic ring systems having 5 to 40 aromatic ring atoms, which may be substituted by one or more radicals R⁶;
R² is selected on each occurrence, identically or differently, from H, D, F, CN, Si(R⁶)₃, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁰ may be linked to one another and may form a ring; where the said alkyl, alkoxy, alkenyl and alkynyl groups and the said aromatic ring systems and heteroaromatic ring systems may in each case be substituted by one or more radicals R⁶; and where one or more CH₂ groups in the said alkyl, alkoxy, alkenyl and alkynyl groups may be replaced by -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO or SO₂;
R³, R⁴, R⁵ are selected on each occurrence, identically or differently, from H, D, F, C(=O)R⁶, CN, Si(R⁶)₃, N(R⁶)₂, P(=O)(R⁶)₂, OR⁶, S(=O)R⁶, S(=O)₂R⁶, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ or R⁴ or R⁵ may be linked to one another and may form a ring; where the said alkyl, alkoxy, alkenyl and alkynyl groups and the said aromatic ring systems and heteroaromatic ring systems may in each case be substituted by one or more radicals R⁶; and where one or more CH₂ groups in the said alkyl, alkoxy, alkenyl and alkynyl groups may be replaced by -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO or SO₂;
R⁶ is selected on each occurrence, identically or differently, from H, D, F, C(=O)R⁷, CN, Si(R⁷)₃, N(R⁷)₂, P(=O)(R⁷)₂, OR⁷, S(=O)R⁷, S(=O)₂R⁷, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁶ may be linked to one another and may form a ring; where the said alkyl, alkoxy, alkenyl and alkynyl groups and the said aromatic ring systems and heteroaromatic ring systems may in each case be substituted by one or more radicals R⁷; and where one or more CH₂ groups in the said alkyl, alkoxy, alkenyl and alkynyl groups may be replaced by -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO or SO₂;
R⁷ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁷ may be linked to one another and may form a ring; and where the said alkyl, alkoxy, alkenyl and alkynyl groups, aromatic ring systems and heteroaromatic ring systems may be substituted by F or CN;
k is equal to 0, 1, 2 or 3, where, in the case where k=0, the group Ar^{L} is omitted and the nitrogen atom of the group of the formula (N) represents the bonding position;
m is equal to 0 or 1, where, in the case where m=0, the group E is omitted and the groups Ar² are not connected to one another;
where in formulae (I) and (II) all positions drawn as unsubstituted may in each case be substituted by a radical R³; and
where in formula (I) and in formula (II) in each case at least one group Z¹ that is CR¹ is present.

2. Compound according to Claim 1, **characterised in that** in one of the formulae (I) and (II), in each case one or two groups Z¹ are equal to CR¹, and the other groups Z¹ are equal to CR³.

3. Compound according to Claim 1 or 2, **characterised in that**

4. Compound according to one or more of Claims 1 to 3, **characterised in that** X¹ is equal to C(R⁴)₂.

5. Compound according to one or more of Claims 1 to 4, **characterised in that** k is equal to 0.

6. Compound according to one or more of Claims 1 to 4, **characterised in that** k is not equal to 0 and -(Ar^{L})ₖ- corresponds to one of the following formulae
| | | |
|---|---|---|
| 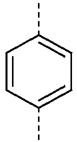 | 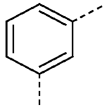 | 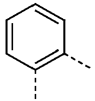 |
| Ar^{L}-1 | Ar^{L}-2 | Ar^{L}-3 |
| 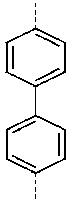 | 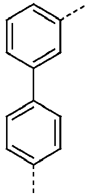 | 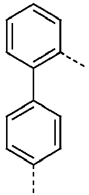 |
| Ar^{L}-4 | Ar^{L}-5 | Ar^{L}-6 |
| 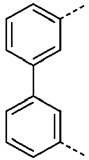 | 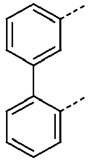 | 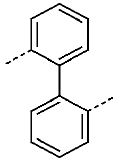 |
| Ar^{L}-7 | Ar^{L}-8 | Ar^{L}-9 |
| 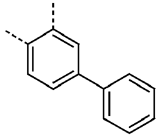 | 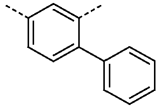 | 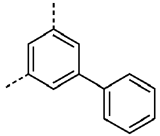 |
| Ar^{L}-10 | Ar^{L}-11 | Ar^{L}-12 |
| 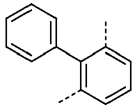 | 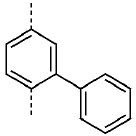 | 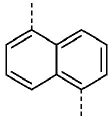 |
| Ar^{L}-13 | Ar^{L}-14 | Ar^{L}-15 |
| 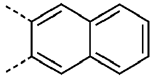 | 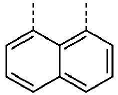 | 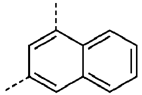 |
| Ar^{L}-16 | Ar^{L}-17 | Ar^{L}-18 |
| 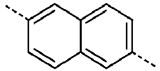 | 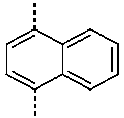 | 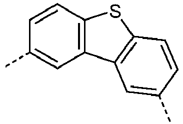 |
| Ar^{L}-19 | Ar^{L}-20 | Ar^{L}-21 |
| 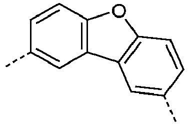 | 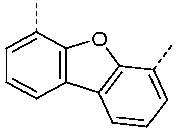 | 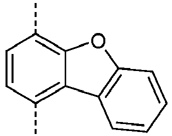 |
| Ar^{L}-22 | Ar^{L}-23 | Ar^{L}-24 |
| 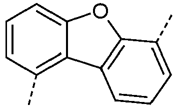 | 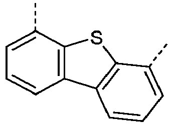 | 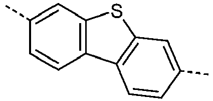 |
| Ar^{L}-25 | Ar^{L}-26 | Ar^{L}-27 |
| 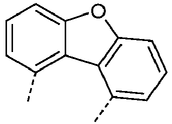 | 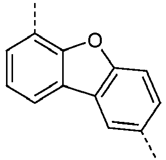 | 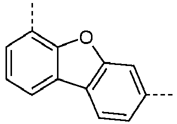 |
| Ar^{L}-28 | Ar^{L}-29 | Ar^{L}-30 |
| 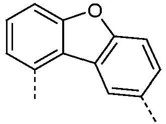 | 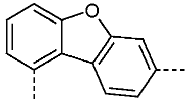 | 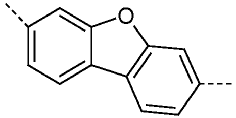 |
| Ar^{L}-31 | Ar^{L}-32 | Ar^{L}-33 |
| 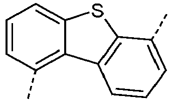 | 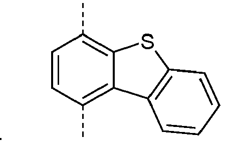 | 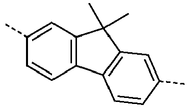 |
| Ar^{L}-34 | Ar^{L}-35 | Ar^{L}-36 |
| 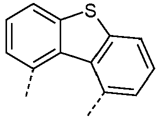 | 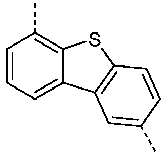 | 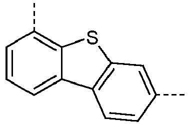 |
| Ar^{L}-37 | Ar^{L}-38 | Ar^{L}-39 |
| 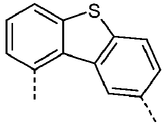 | 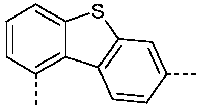 | 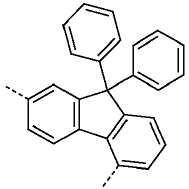 |
| Ar^{L}-40 | Ar^{L}-41 | Ar^{L}-42 |
| 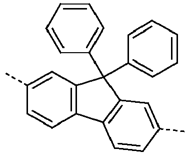 | 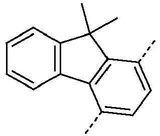 | 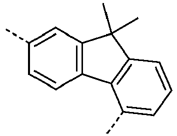 |
| Ar^{L}-43 | Ar^{L}-44 | Ar^{L}-45 |
| 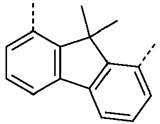 | 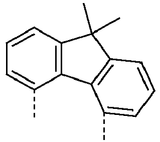 | 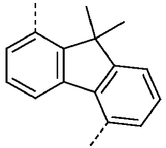 |
| Ar^{L}-46 | Ar^{L}-47 | Ar^{L}-48 |
| 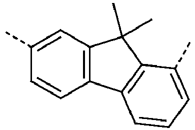 | 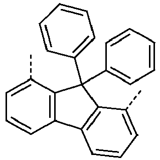 | 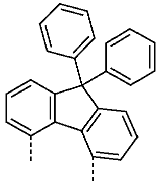 |
| Ar^{L}-49 | Ar^{L}-50 | Ar^{L}-51 |
| 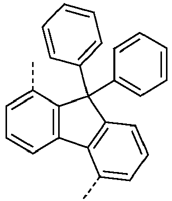 | 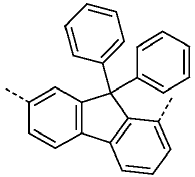 | 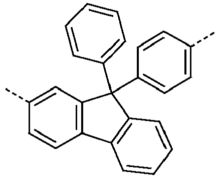 |
| Ar^{L}-52 | Ar^{L}-53 | Ar^{L}-54 |
| 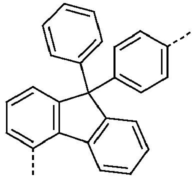 | 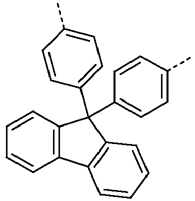 | 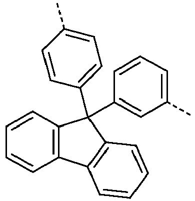 |
| Ar^{L}-55 | Ar^{L}-56 | Ar^{L}-57 |
| 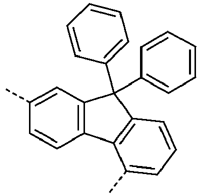 | 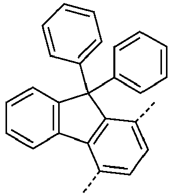 | 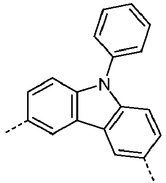 |
| Ar^{L}-58 | Ar^{L}-59 | Ar^{L}-60 |
| 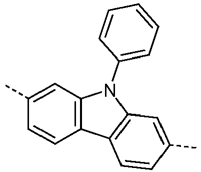 | 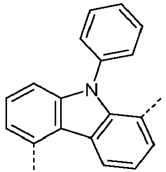 | 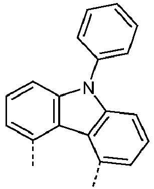 |
| Ar^{L}-61 | Ar^{L}-62 | Ar^{L}-63 |
| 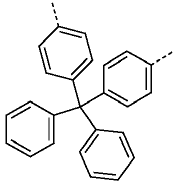 | 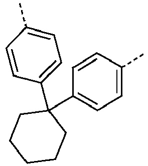 | 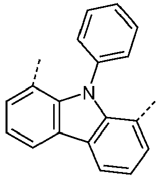 |
| Ar^{L}-64 | Ar^{L}-65 | Ar^{L}-66 |
| 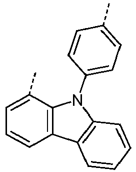 | 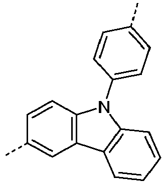 | 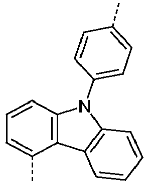 |
| Ar^{L}-67 | Ar^{L}-68 | Ar^{L}-69 |
| 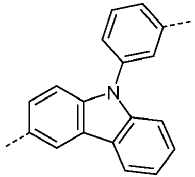 | 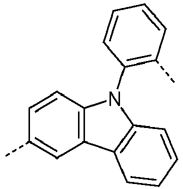 | 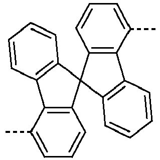 |
| Ar^{L}-70 | Ar^{L}-71 | Ar^{L}-72 |
| 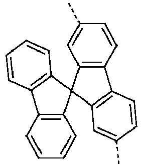 | 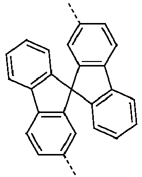 | 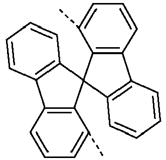 |
| Ar^{L}-73 | Ar^{L}-74 | Ar^{L}-75 |
where the dashed lines in each case represent the bonds to the remainder of the formula (I) or (II).

7. Compound according to one or more of Claims 1 to 6, **characterised in that** Ar² is selected on each occurrence, identically or differently, from phenyl, biphenyl, terphenyl, quaterphenyl, naphthyl, fluorenyl, in particular 9,9'-dimethylfluorenyl and 9,9'-diphenylfluorenyl, benzofluorenyl, spirobifluorenyl, indenofluorenyl, indenocarbazolyl, dibenzofuranyl, dibenzothiophenyl, carbazolyl, benzofuranyl, benzothiophenyl, benzo-fused dibenzofuranyl, benzo-fused dibenzothiophenyl, naphthyl-substituted phenyl, fluorenyl-substituted phenyl, spirobifluorenyl-substituted phenyl, dibenzofuranyl-substituted phenyl, dibenzothiophenyl-substituted phenyl, carbazolyl-substituted phenyl, pyridyl-substituted phenyl, pyrimidyl-substituted phenyl and triazinyl-substituted phenyl, where the said groups may in each case be substituted by one or more radicals R⁵.

8. Compound according to one or more of Claims 1 to 7, **characterised in that** the group 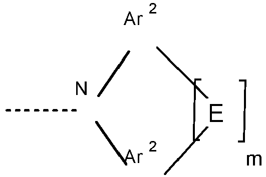 in formulae (I) and (II) is in each case selected from the following formulae
| | |
|---|---|
| 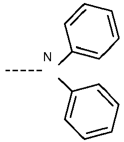 | 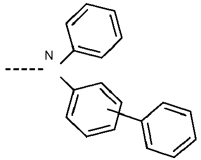 |
| A-1 | A-2 |
| 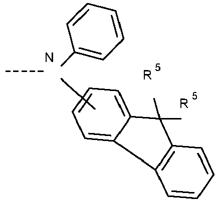 | 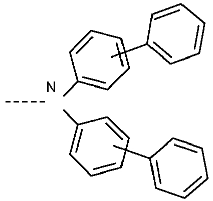 |
| A-3 | A-4 |
| 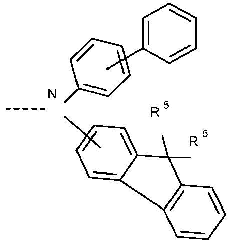 | 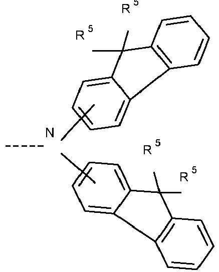 |
| A-5 | A-6 |
| | |
|---|---|
| 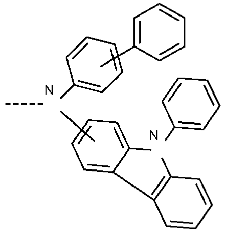 | 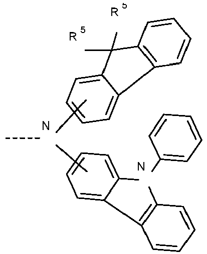 |
| A-7 | A-8 |
| 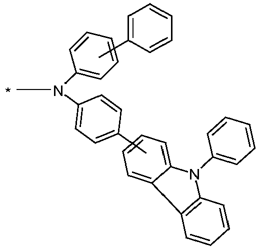 | 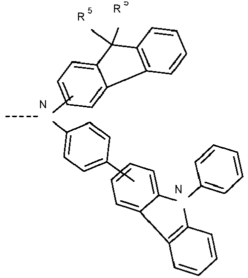 |
| A-9 | A-10 |
| 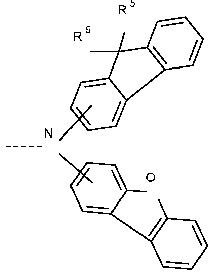 | 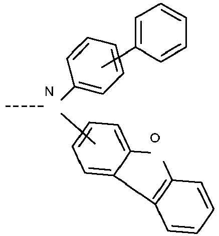 |
| A-11 | A-12 |
| 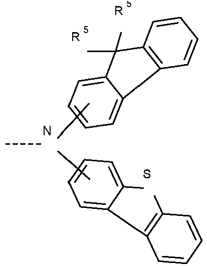 | 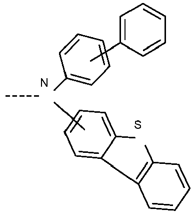 |
| A-13 | A-14 |
| | |
|---|---|
| 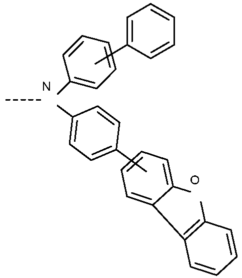 | 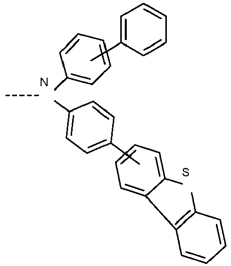 |
| A-15 | A-16 |
| 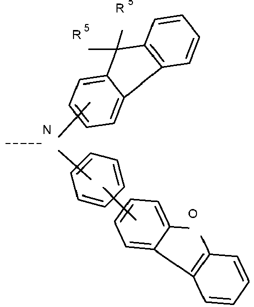 | 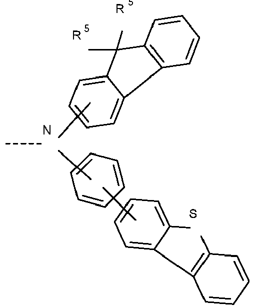 |
| A-17 | A-18 |
| 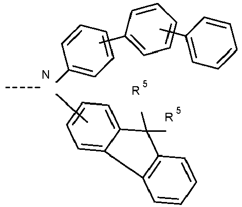 | 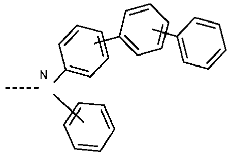 |
| A-19 | A-20 |
| 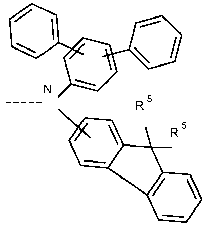 | 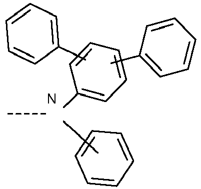 |
| A-21 | A-22 |
| | |
|---|---|
| 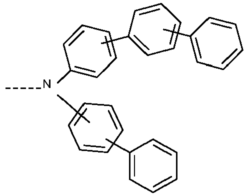 | 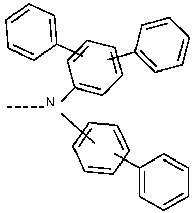 |
| A-23 | A-24 |
| 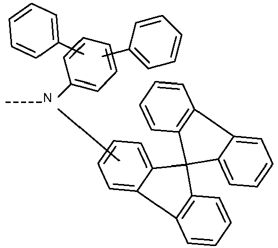 | 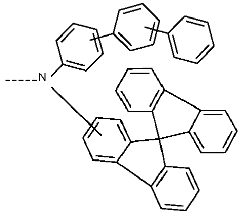 |
| A-25 | A-26 |
| 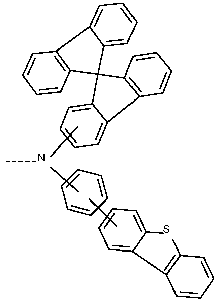 | 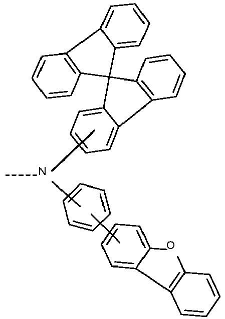 |
| A-27 | A-28 |
| 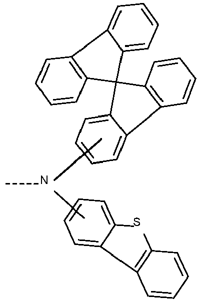 | 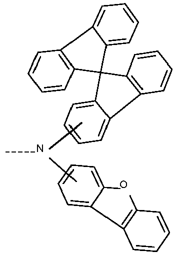 |
| A-29 | A-30 |
| | |
|---|---|
| 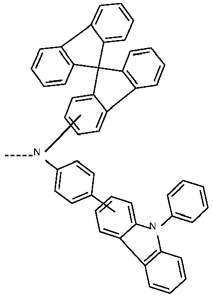 | 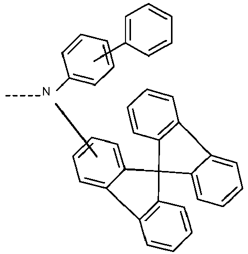 |
| A-31 | A-32 |
| 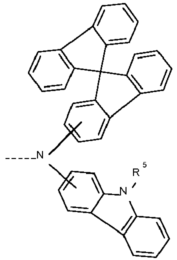 | 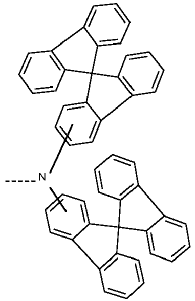 |
| A-33 | A-34 |
| 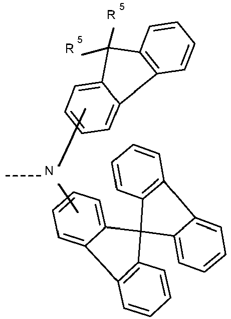 | 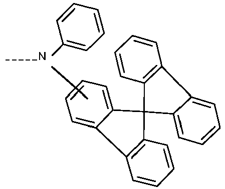 |
| A-35 | A-36 |
| | |
|---|---|
| 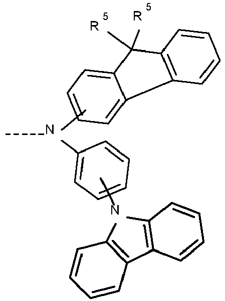 | 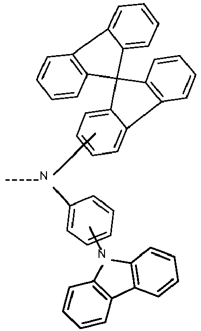 |
| A-37 | A-38 |
| 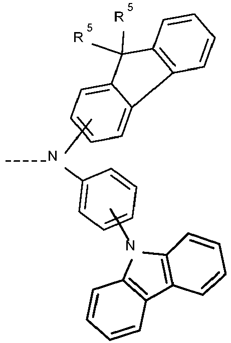 | |
| A-39 | |
where the groups may in each case be substituted at their free positions by a radical R⁵, and are preferably unsubstituted in the free positions, and
where the dashed bonds represent the bonds to the remainder of the formula (I) or (II).

9. Compound according to one or more of Claims 1 to 8, **characterised in that** R⁰ is equal to H.

10. Compound according to one or more of Claims 1 to 9, **characterised in that** R¹ is selected on each occurrence, identically or differently, from monovalent groups derived from benzene, biphenyl, terphenyl, quarter-phenyl, naphthalene, fluorene, in particular 9,9'-dimethylfluorene and 9,9'-diphenylfluorene, benzofluorene, spirobifluorene, indenofluorene, indeno-carbazole, dibenzofuran, dibenzothiophene, benzocarbazole, carbazole, benzofuran, benzothiophene, indole, quinoline, pyridine, pyrimidine, pyrazine, pyridazine and triazine, where the monovalent groups may in each case be substituted by one or more radicals R⁶.

11. Compound according to one or more of Claims 1 to 10, **characterised in that** R² and R³ are equal to H.

12. Compound according to one or more of Claims 1 to 11, **characterised in that** formula (I) corresponds to one of the following formulae:

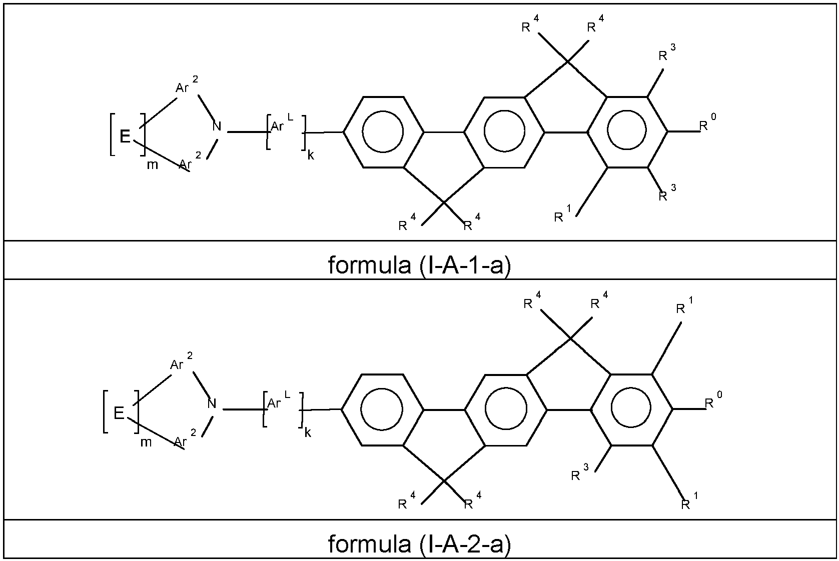

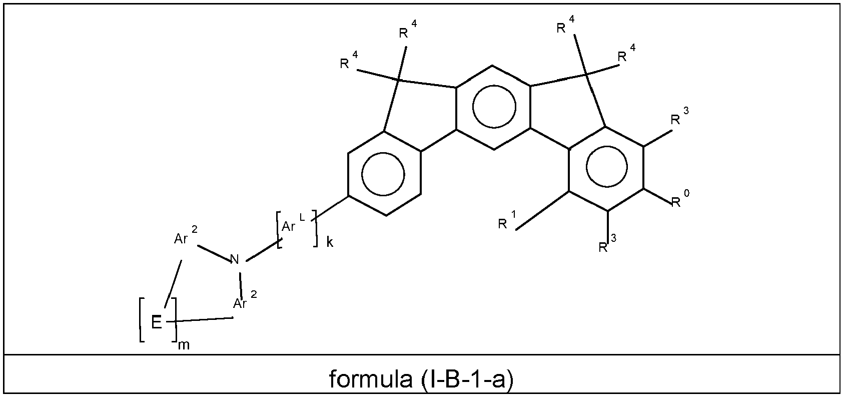

13. Process for the preparation of a compound of the formula (I) or (II) according to one or more of Claims 1 to 12, **characterised in that** a benzene compound carrying two carboxylate groups and at least one reactive group is reacted with a benzene compound containing a boronic acid group and at least one group selected from reactive groups X and aromatic or heteroaromatic groups Ar, where the boronic acid group and the at least one group selected from groups X and Ar are in the ortho- or meta-position to one another on the benzene ring.

14. Oligomer, polymer or dendrimer containing one or more compounds of the formula (I) or (II) according to one or more of Claims 1 to 12, where the bond(s) to the polymer, oligomer or dendrimer may be localised at any desired positions in formula (I) or (II) that are substituted by R¹, R², R³, R⁴ or R⁵.

15. Formulation comprising at least one compound according to one or more of Claims 1 to 12 or at least one polymer, oligomer or dendrimer according to Claim 14, and at least one solvent.

16. Electronic device containing at least one compound according to one or more of Claims 1 to 12, or at least one polymer, oligomer or dendrimer according to Claim 14.

17. Electronic device according to Claim 16, **characterised in that** it is an organic electroluminescent device and contains anode, cathode and at least one emitting layer, and **in that** the compound is present in a hole-transporting layer of the device.

18. Organic electroluminescent device according to Claim 17, **characterised in that** the compound is present in an electron-blocking layer of the device.

19. Use of a compound according to one or more of Claims 1 to 12 in an electronic device.

## Revendications

1. Composé de formule (I) ou (II) suivante 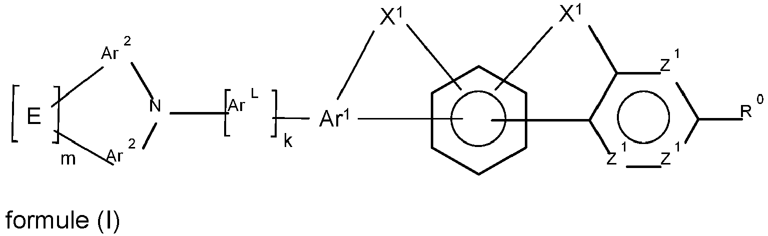 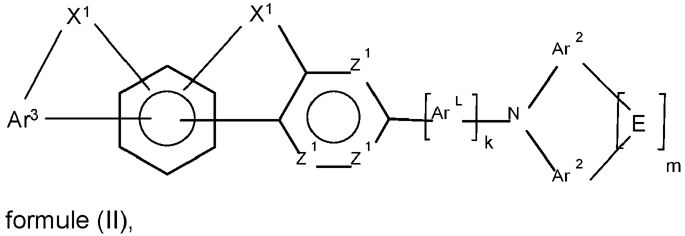 dans lequel ce qui suit s'applique aux variables présentes :
Z¹ est choisi à chaque occurrence, de manière identique ou différente, parmi CR¹ et CR³ ;
Ar¹ est un groupement aryle ayant de 6 à 20 atomes de cycle aromatique, pouvant être substitué par un ou plusieurs radicaux R³, ou un groupement hétéroaryle ayant de 5 à 20 atomes de cycle aromatique, pouvant être substitué par un ou plusieurs radicaux R³ ;
Ar² est choisi à chaque occurrence, de manière identique ou différente, parmi des noyaux aromatiques ayant de 6 à 40 atomes de cycle aromatique, pouvant être substitués par un ou plusieurs radicaux R⁵, et des noyaux hétéroaromatiques ayant de 5 à 40 atomes de cycle aromatique, pouvant être substitués par un ou plusieurs radicaux R⁵ ;
Ar³ est un groupement aryle ayant de 6 à 20 atomes de cycle aromatique, pouvant être substitué par un ou plusieurs radicaux R², ou un groupement hétéroaryle ayant de 5 à 20 atomes de cycle aromatique, pouvant être substitué par un ou plusieurs radicaux R² ;
X¹ est à chaque occurrence, de manière identique ou différente, un groupement divalent choisi parmi -C(R⁴)₂-, -C(R⁴)₂-C(R⁴)₂-, -CR⁴=CR⁴-, -Si(R⁴)₂-, NR⁴, O et S ;
Ar^{L} est choisi parmi des noyaux aromatiques ayant de 6 à 40 atomes de cycle aromatique, pouvant être substitués par un ou plusieurs radicaux R⁵, et des noyaux hétéroaromatiques ayant de 5 à 40 atomes de cycle aromatique, pouvant être substitués par un ou plusieurs radicaux R⁵ ;
E est une liaison simple ou un groupement divalent choisi parmi C(R⁵)₂, Si(R⁵)₂, N(R⁵), O et S ;
R⁰ est choisi parmi H, D, des noyaux aromatiques ayant de 6 à 40 atomes de cycle aromatique, pouvant être substitués par un ou plusieurs radicaux R⁶, et des noyaux hétéroaromatiques ayant de 5 à 40 atomes de cycle aromatique, pouvant être substitués par un ou plusieurs radicaux R⁶ ;
R¹ est choisi à chaque occurrence, de manière identique ou différente, parmi des noyaux aromatiques ayant de 6 à 40 atomes de cycle aromatique, pouvant être substitués par un ou plusieurs radicaux R⁶, et des noyaux hétéroaromatiques ayant de 5 à 40 atomes de cycle aromatique, pouvant être substitués par un ou plusieurs radicaux R⁶ ;
R² est choisi à chaque occurrence, de manière identique ou différente, parmi H, D, F, CN, Si(R⁶)₃, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁰ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁶ ; et où un ou plusieurs groupements CH₂ dans lesdits groupements alkyle, alcoxy, alcényle et alcynyle peuvent être remplacés par -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO ou SO₂ ;
R³, R⁴, R⁵ sont choisis à chaque occurrence, de manière identique ou différente, parmi H, D, F, C(=O)R⁶, CN, Si(R⁶)₃, N(R⁶)₂, P(=O)(R⁶)₂, OR⁶, S(=O)R⁶, S(=O)₂R⁶, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R³ ou R⁴ ou R⁵ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁶ ; et où un ou plusieurs groupements CH₂ dans lesdits groupements alkyle, alcoxy, alcényle et alcynyle peuvent être remplacés par -R⁶C=CR⁶-, -C=C-, Si(R⁶)₂, C=O, C=NR⁶, -C(=O)O-, -C(=O)NR⁶-, NR⁶, P(=O)(R⁶), -O-, -S-, SO ou SO₂ ;
R⁶ est choisi à chaque occurrence, de manière identique ou différente, parmi H, D, F, C(=O)R⁷, CN, Si(R⁷)₃, N(R⁷)₂, P(=O)(R⁷)₂, OR⁷, S(=O)R⁷, S(=O)₂R⁷, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁶ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁷ ; et où un ou plusieurs groupements CH₂ dans lesdits groupements alkyle, alcoxy, alcényle et alcynyle peuvent être remplacés par -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO ou SO₂ ;
R⁷ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁷ peuvent être liés les uns aux autres et peuvent former un cycle ; et où lesdits groupements alkyle, alcoxy, alcényle et alcynyle, lesdits noyaux aromatiques et lesdits noyaux hétéroaromatiques peuvent être substitués par F ou CN ;
k est égal à 0, 1, 2 ou 3, où, dans le cas où k=0, le groupement Ar^{L} est omis et l'atome d'azote du groupement de formule (N) représente la position de liaison ;
m est égal à 0 ou 1, où, dans le cas où m=0, le groupement E est omis et les groupements Ar² ne sont pas reliés les uns aux autres ;
où dans les formules (I) et (II), toutes les positions dessinées comme étant non substituées peuvent dans chaque cas être substituées par un radical R³ ; et
où dans la formule (I) et dans la formule (II), dans chaque cas au moins un groupement Z¹ qui est CR¹ est présent.

2. Composé selon la revendication 1, **caractérisé en ce que**, dans l'une parmi les formules (I) et (II), dans chaque cas un ou deux groupements Z¹ sont égaux à CR¹, et les autres groupements Z¹ sont égaux à CR³.

3. Composé selon la revendication 1 ou 2, **caractérisé en ce que**

4. Composé selon l'une ou plusieurs parmi les revendications 1 à 3, **caractérisé en ce que** X¹ est égal à C(R⁴)₂.

5. Composé selon l'une ou plusieurs parmi les revendications 1 à 4, **caractérisé en ce que** k est égal à 0.

6. Composé selon l'une ou plusieurs parmi les revendications 1 à 4, **caractérisé en ce que** k n'est pas égal à 0 et -(Ar^{L})ₖ- correspond à l'une des formules suivantes
| | | |
|---|---|---|
| 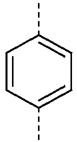 | 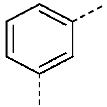 | 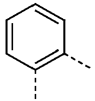 |
| Ar^{L}-1 | Ar^{L}-2 | Ar^{L}-3 |
| 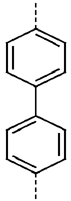 | 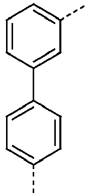 | 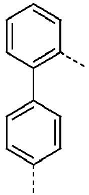 |
| Ar^{L}-4 | Ar^{L}-5 | Ar^{L}-6 |
| 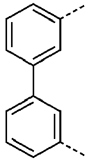 | 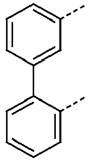 | 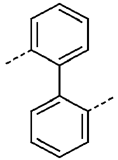 |
| Ar^{L}-7 | Ar^{L}-8 | Ar^{L}-9 |
| 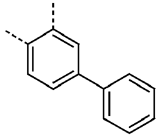 | 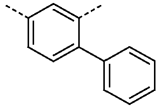 | 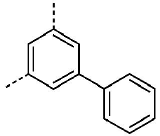 |
| Ar^{L}-10 | Ar^{L}-11 | Ar^{L}-12 |
| 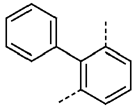 | 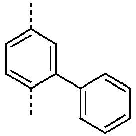 | 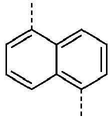 |
| Ar^{L}-13 | Ar^{L}-14 | Ar^{L}-15 |
| 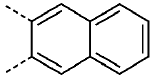 | 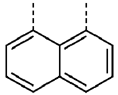 | 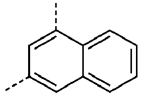 |
| Ar^{L}-16 | Ar^{L}-17 | Ar^{L}-18 |
| 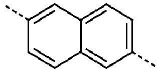 | 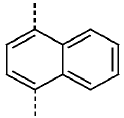 | 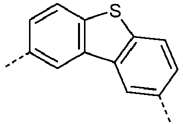 |
| Ar^{L}-19 | Ar^{L}-20 | Ar^{L}-21 |
| 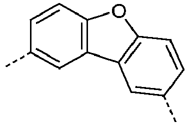 | 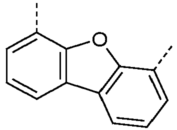 | 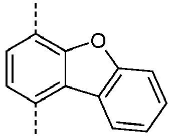 |
| Ar^{L}-22 | Ar^{L}-23 | Ar^{L}-24 |
| 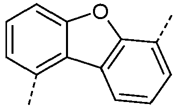 | 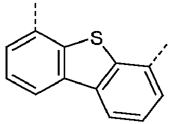 | 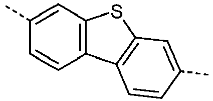 |
| Ar^{L}-25 | Ar^{L}-26 | Ar^{L}-27 |
| 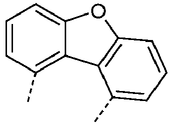 | 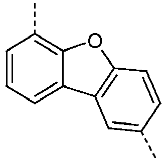 | 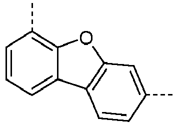 |
| Ar^{L}-28 | Ar^{L}-29 | Ar^{L}-30 |
| 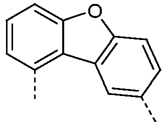 | 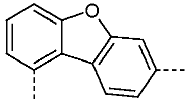 | 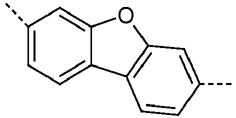 |
| Ar^{L}-31 | Ar^{L}-32 | Ar^{L}-33 |
| 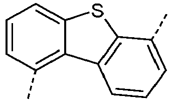 | 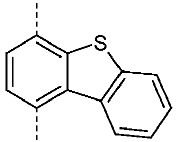 | 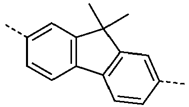 |
| Ar^{L}-34 | Ar^{L}-35 | Ar^{L}-36 |
| 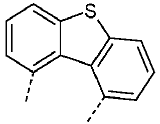 | 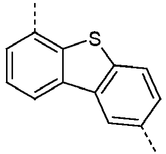 | 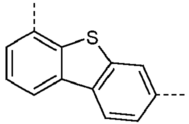 |
| Ar^{L}-37 | Ar^{L}-38 | Ar^{L}-39 |
| 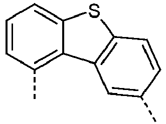 | 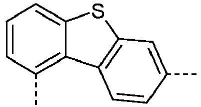 | 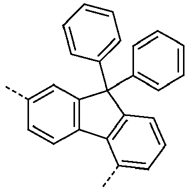 |
| Ar^{L}-40 | Ar^{L}-41 | Ar^{L}-42 |
| 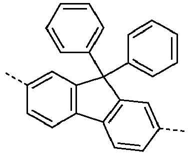 | 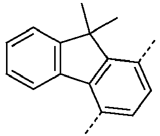 | 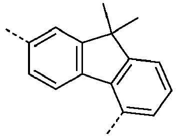 |
| Ar^{L}-43 | Ar^{L}-44 | Ar^{L}-45 |
| 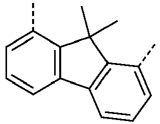 | 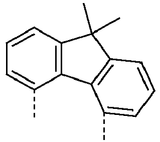 | 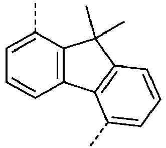 |
| Ar^{L}-46 | Ar^{L}-47 | Ar^{L}-48 |
| 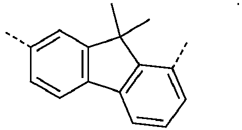 | 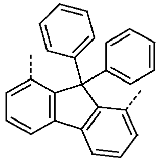 | 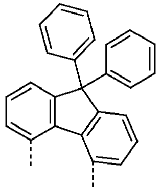 |
| Ar^{L}-49 | Ar^{L}-50 | Ar^{L}-51 |
| 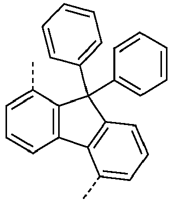 | 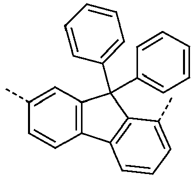 | 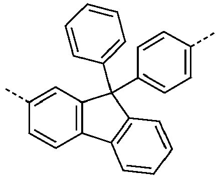 |
| Ar^{L}-52 | Ar^{L}-53 | Ar^{L}-54 |
| 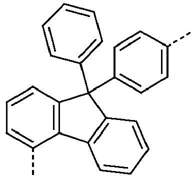 | 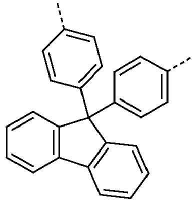 | 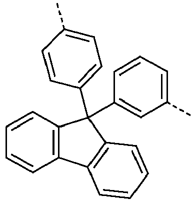 |
| Ar^{L}-55 | Ar^{L}-56 | Ar^{L}-57 |
| 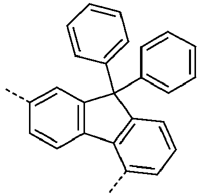 | 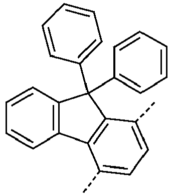 | 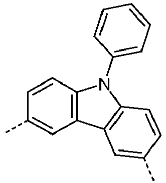 |
| Ar^{L}-58 | Ar^{L}-59 | Ar^{L}-60 |
| 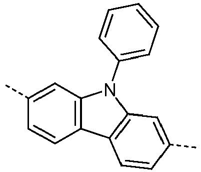 | 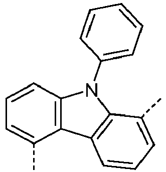 | 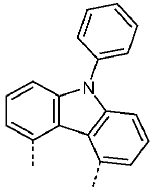 |
| Ar^{L}-61 | Ar^{L}-62 | Ar^{L}-63 |
| 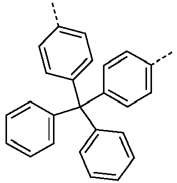 | 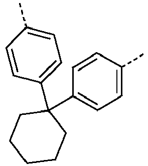 | 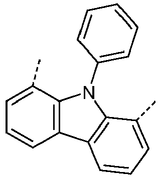 |
| Ar^{L}-64 | Ar^{L}-65 | Ar^{L}-66 |
| 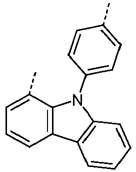 | 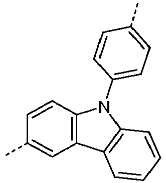 | 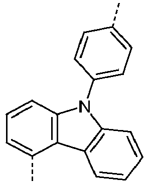 |
| Ar^{L}-67 | Ar^{L}-68 | Ar^{L}-69 |
| 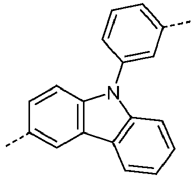 | 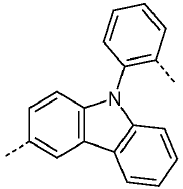 | 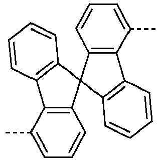 |
| Ar^{L}-70 | Ar^{L}-71 | Ar^{L}-72 |
| 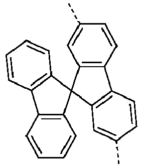 | 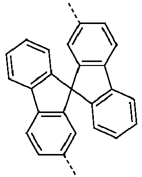 | 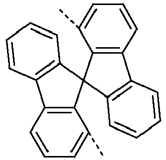 |
| Ar^{L}-73 | Ar^{L}-74 | Ar^{L}-75 |
où les lignes en pointillé dans chaque cas représentent les liaisons au reste de la formule (I) ou (II).

7. Composé selon l'une ou plusieurs parmi les revendications 1 à 6, **caractérisé en ce que** Ar² est choisi à chaque occurrence, de manière identique ou différente, parmi phényle, biphényle, terphényle, quaterphényle, naphtyle, fluorényle, en particulier 9,9'-diméthylfluorényle et 9,9'-diphénylfluorényle, benzofluorényle, spirobifluorényle, indénofluorényle, indénocarbazolyle, dibenzofuranyle, dibenzothiophényle, carbazolyle, benzofuranyle, benzothiophényle, dibenzofuranyle benzo-condensé, dibenzothiophényle benzo-condensé, phényle à substitution naphtyle, phényle à substitution fluorényle, phényle à substitution spirobifluorényle, phényle à substitution dibenzofuranyle, phényle à substitution dibenzothiophényle, phényle à substitution carbazolyle, phényle à substitution pyridyle, phényle à substitution pyrimidyle et phényle à substitution triazinyle, où lesdits groupements peuvent dans chaque cas être substitués par un ou plusieurs radicaux R⁵.

8. Composé selon l'une ou plusieurs parmi les revendications 1 à 7, **caractérisé en ce que** le groupement 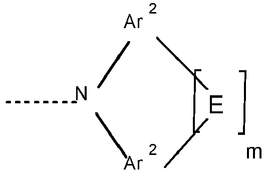 dans les formules (I) et (II) est dans chaque cas choisi parmi les formules suivantes
| | |
|---|---|
| 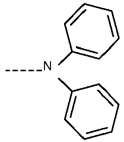 | 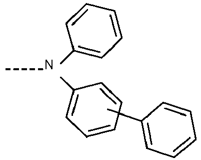 |
| A-1 | A-2 |
| 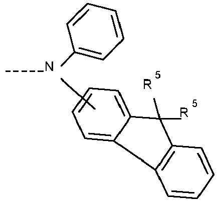 | 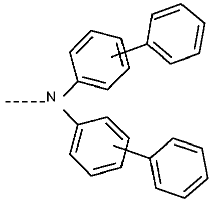 |
| A-3 | A-4 |
| 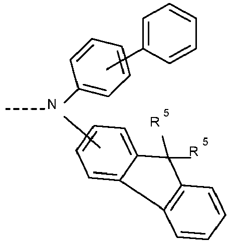 | 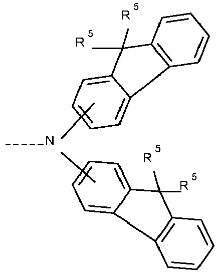 |
| A-5 | A-6 |
| 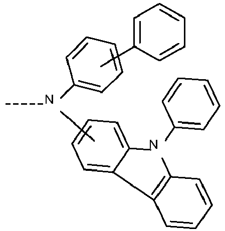 | 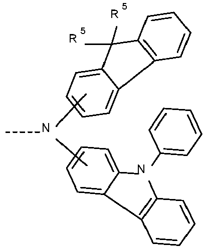 |
| A-7 | A-8 |
| 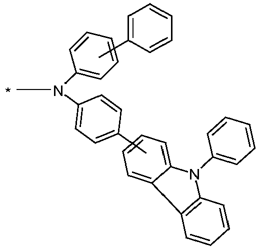 | 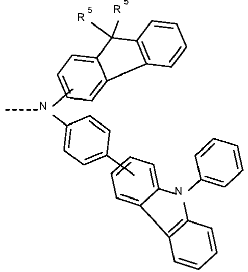 |
| A-9 | A-10 |
| 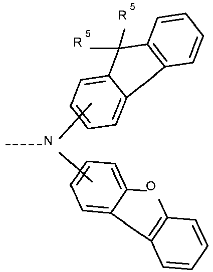 | 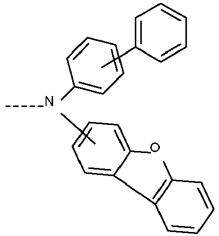 |
| A-11 | A-12 |
| 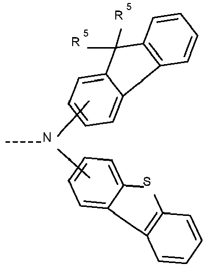 | 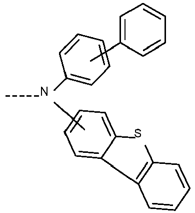 |
| A-13 | A-14 |
| 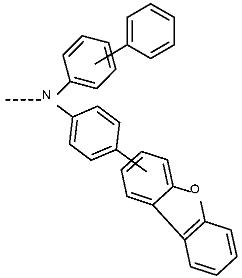 | 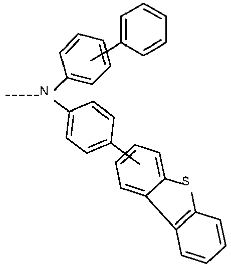 |
| A-15 | A-16 |
| 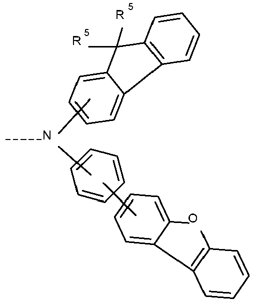 | 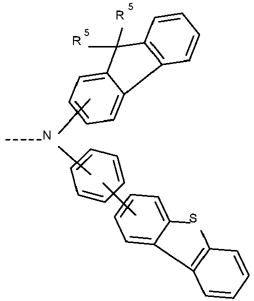 |
| A-17 | A-18 |
| 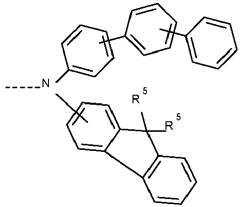 | 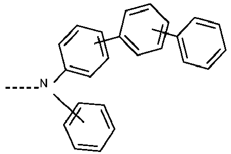 |
| A-19 | A-20 |
| 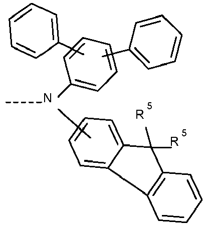 | 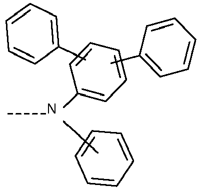 |
| A-21 | A-22 |
| 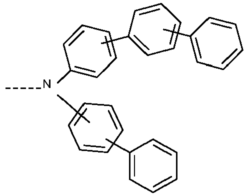 | 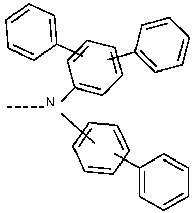 |
| A-23 | A-24 |
| 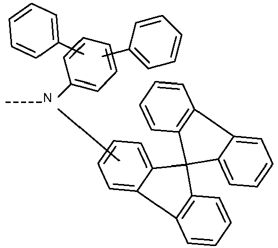 | 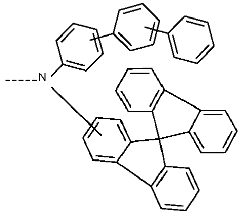 |
| A-25 | A-26 |
| 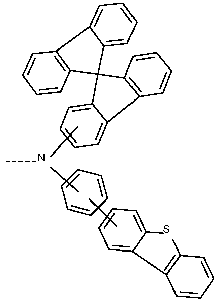 | 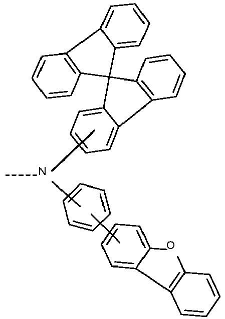 |
| A-27 | A-28 |
| 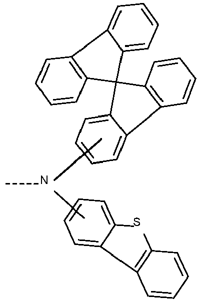 | 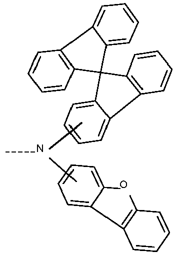 |
| A-29 | A-30 |
| 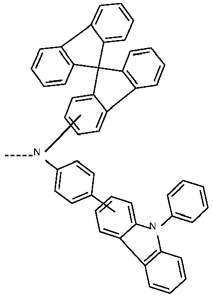 | 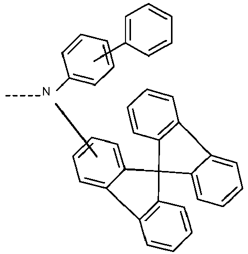 |
| A-31 | A-32 |
| 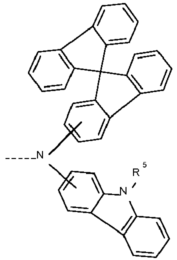 | 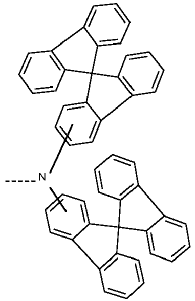 |
| A-33 | A-34 |
| 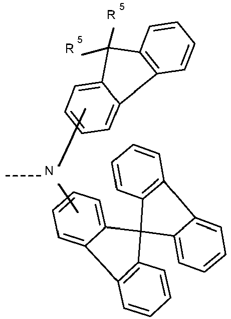 | 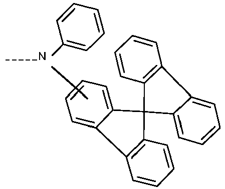 |
| A-35 | A-36 |
| 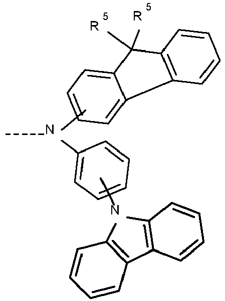 | 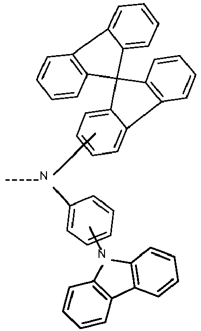 |
| A-37 | A-38 |
| 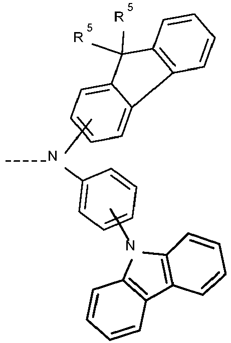 | |
| A-39 | |
où les groupements peuvent dans chaque cas être substitués au niveau de leurs positions libres par un radical R⁵, et sont préférablement non substitués dans les positions libres, et où les liaisons en pointillé représentent les liaisons au reste de la formule (I) ou (II).

9. Composé selon l'une ou plusieurs parmi les revendications 1 à 8, **caractérisé en ce que** R⁰ est égal à H.

10. Composé selon l'une ou plusieurs parmi les revendications 1 à 9, **caractérisé en ce que** R¹ est choisi à chaque occurrence, de manière identique ou différente, parmi les groupements monovalents dérivés de benzène, biphényle, terphényle, quaterphényle, naphtalène, fluorène, en particulier 9,9'-diméthylfluorène et 9,9'-diphénylfluorène, benzofluorène, spirobifluorène, indénofluorène, indénocarbazole, dibenzofurane, dibenzothiophène, benzocarbazole, carbazole, benzofurane, benzothiophène, indole, quinoléine, pyridine, pyrimidine, pyrazine, pyridazine et triazine, où les groupements monovalents peuvent dans chaque cas être substitués par un ou plusieurs radicaux R⁶.

11. Composé selon l'une ou plusieurs parmi les revendications 1 à 10, **caractérisé en ce que** R² et R³ sont égaux à H.

12. Composé selon l'une ou plusieurs parmi les revendications 1 à 11, **caractérisé en ce que** la formule (I) correspond à l'une des formules suivantes :

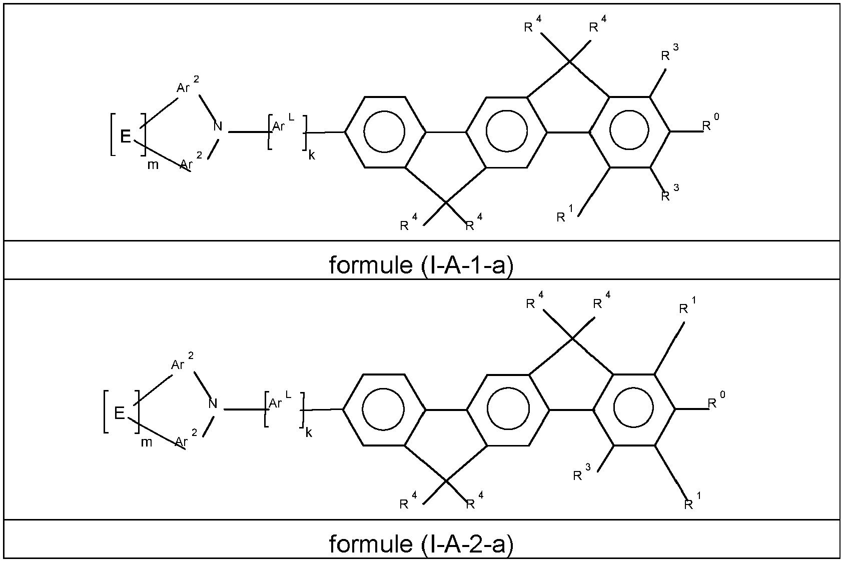

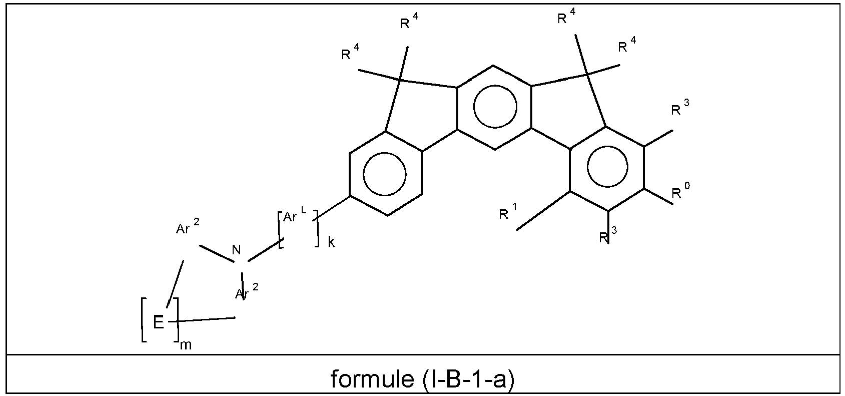

13. Procédé de préparation d'un composé de formule (I) ou (II) selon l'une ou plusieurs parmi les revendications 1 à 12, **caractérisé en ce qu'**un composé de benzène portant deux groupements carboxylate et au moins un groupement réactif est réagi avec un composé de benzène contenant un groupement d'acide boronique et au moins un groupement choisi parmi les groupements réactifs X et les groupements aromatiques ou hétéroaromatiques Ar, où le groupement d'acide boronique et le au moins un groupement choisi parmi les groupements X et Ar se trouvent en position ortho ou méta l'un par rapport à l'autre dans le cycle de benzène.

14. Oligomère, polymère ou dendrimère contenant un ou plusieurs composés de formule (I) ou (II) selon l'une ou plusieurs parmi les revendications 1 à 12, dans lequel la ou les liaisons au polymère, oligomère ou dendrimère peuvent être localisées au niveau de positions désirées quelconques dans la formule (I) ou (II) qui sont substituées par R¹, R², R³, R⁴ ou R⁵.

15. Formulation comprenant au moins un composé selon l'une ou plusieurs parmi les revendications 1 à 12 ou au moins un polymère, oligomère ou dendrimère selon la revendication 14, et au moins un solvant.

16. Dispositif électronique contenant au moins un composé selon l'une ou plusieurs parmi les revendications 1 à 12, ou au moins un polymère, oligomère ou dendrimère selon la revendication 14.

17. Dispositif électronique selon la revendication 16, **caractérisé en ce qu'**il s'agit d'un dispositif électroluminescent organique et qu'il contient une anode, une cathode et au moins une couche émettrice, et **en ce que** le composé est présent dans une couche de transport de trous du dispositif.

18. Dispositif électroluminescent organique selon la revendication 17, **caractérisé en ce que** le composé est présent dans une couche de blocage d'électrons du dispositif.

19. Utilisation d'un composé selon l'une ou plusieurs parmi les revendications 1 à 12, dans un dispositif électronique.
